# EUROPEAN PATENT APPLICATION

(11) **EP 3 682 947 A1**
(43) Date of publication of application: **22.07.2020**
(21) Application number: 20159424.9
(22) Date of filing: 02.05.2011
(51) Int. Cl.: A61P 29/00, C07D 417/06, A61K 31/427

(54) **COMPOUNDS AND METHODS FOR REGULATING INTEGRIN CD11B/CD18**

(30) Priority: 08.07.2010 US 36236310 P
(62) Divisional of application: 17177006.8
(71) Applicant: GB006, Inc., San Diego, CA 92121 (US)
(72) Inventor: GUPTA, Vineet, Pinecrest, FL 33156 (US)
(74) Representative: Murgitroyd & Company

(57) **Abstract**

A pharmaceutical formulation of a β2 integrin agonist including an effective amount of a β2 integrin agonist and a pharmaceutically acceptable carrier. A method of activating β2 integrins by interacting the β2 integrins with a β2 integrin agonist. A method of treating a patient by administering an effective amount of a β2integrin agonist, and activating β2 integrins. A method of treating inflammation by administering a β2 integrin agonist to a patient with inflammation, activating β2 integrins, and reducing inflammation. Methods of treating and/or preventing renal ischemia-reperfusion (I/R) injury, reducing injury in a patient due to insertion of a stent, performing an assay for the identification of small molecule modulators of β2 integrins, detecting a disease in a patient, and improving the general wellness of a patient.

## Description

### BACKGROUND OF THE INVENTION

### 1. TECHNICAL FIELD

The present invention relates to activation of integrin CD11b/CD18 with various agents. The present invention further relates to treating inflammatory diseases.

### 2. BACKGROUND ART

Integrins are non-covalently linked α/βheterodimeric receptors that mediate cell adhesion, migration and signaling. Together with their ligands, integrins play central roles in many processes including development, hemostasis, inflammation and immunity, and in pathologic conditions such as cancer invasion and cardiovascular disease. Leukocyte migration and recruitment is essential for their normal immune response to injury and infection and in various inflammatory and autoimmune disorders [1]. Leukocyte functions are modulated by β2 integrins, including highly expressed integrin CD11b/CD18 (also known as Mac-1, CR3 and αMβ2) [2]. CD11b/CD18 recognizes the complement fragment iC3b, Fibrinogen, and ICAM-1 as ligands, among various others. CD11b/CD18 has been implicated in many inflammatory and autoimmune diseases, such as ischemia-reperfusion injury (including acute renal failure and atherosclerosis), tissue damage, stroke, neointimal thickening in response to vascular injury and the resolution of inflammatory processes [3-7].

Leukocytic β2 integrins modulate tumor infiltration. In response to injury or infection leukocytes are recruited into the tissues where they participate in immune clearance [2]. Tumors also secrete inflammatory cytokines to recruit CD11b+ myeloid cells to facilitate neovascularization [8]. The β2 integrins, a sub-family of α/β heterodimeric integrin receptors that have a common β-subunit (β2, CD18) but distinct α-subunits (CD11a, CD11b, CD11c and CD11d [9]), are leukocyte specific receptors [10]. Integrin CD11b/CD18, one of the two major β2 integrins in leukocytes, mediates binding of leukocytes to their various ligands (>30 in number) and mediates leukocyte migration and recruitment into inflamed tissue [1, 11]. During cancer treatments, irradiated tumors recruit large numbers of specific leukocytes, bone marrow-derived CD11b+ myeloid cells expressing matrix metalloproteinase-9 (MMP-9) that restore tumor vasculature and allow tumor re-growth and recurrence [12]. Recent studies have shown that treatment with CD11b antagonists (anti-CD11b antibody) reduces CD11b+ myeloid cell infiltration and an enhancement of tumor response to radiation in mice [12].

Inflammatory leukocytes potentiate anti-GBM nephritis. Experimental anti-GBM nephritis in mice is a model of rapidly progressive glomerulonephritis, is characterized by proteinuria, leukocyte infiltration and glomerular crescent formation [13, 14]. Leukocytes play a critical role in the pathogenesis of anti-GBM nephritis, and their number correlates with the percentage of crescentic glomeruli. CD11b-/- animals show no proteinurea and strong protection of renal function [15], showing that agents targeting this integrin have a potential to treat this disease.

In addition to increasing cell adhesion and modulating migration, CD11b/CD18 activation mediates a number of intracellular signaling events, including production of reactive oxygen species and modulation of a number of pro- and anti-inflammatory genes in myeloid cells [16-21]. Integrin activation and ligand binding leads to its clustering on the cell surface and initiates outside-in signaling, including the activation of PI3-K/Akt and MAPK/ERK1/2 pathways [17, 22], thereby mimicking the anchorage-dependent pro-survival signals in most cells. Ligation and clustering of CD11b/CD18 also synergistically potentiates intracellular signaling by other receptors (such as Toll-like receptors (TLRs) and cytokine receptors interleukin-1 receptor (IL-1R) and TNFR) and both induce NF-κB dependent expression of pro-inflammatory cytokines (e.g.; IL-1β, IL-6, TNF-α) as well as release of other factors (e.g.; Tissue Factor). CD11b/CD18 deficiency enhances TLR4-triggered production of pro-inflammatory cytokines, suggesting that CD11b/CD18 could have a protective role and may negatively regulate pro-inflammatory pathways in leukocytes (1-3).

Thus, there is a considerable potential for agents that modulate the function of CD11b/CD18 as therapeutic agents for the treatment of various inflammatory conditions. CD11b/CD18 is normally expressed in a constitutively inactive conformation in circulating leukocytes and in many other cells, but is rapidly activated to mediate cell adhesion, migration and accumulation of cells at the sites of inflammation [23]. CD11b/CD18 is also expressed on other cell types and tissues, including microglia, hepatocytes and a sub-type of T- and B-cells. Indeed, blocking CD11b/CD18 and its ligands with antibodies and ligand mimics (anti-adhesion therapy) [24-26] and genetic ablation of CD11b or CD18 decreases the severity of inflammatory response *in vivo* in many experimental models [27, 28]. However, such blocking agents have had little success in treating inflammatory/autoimmune diseases in humans [28, 29], perhaps because complete blockage of CD11b/CD18 with antibodies is difficult due to availability of a large mobilizable intracellular pool of CD1 1b/CD18 [30, 31] or because suppressing leukocyte recruitment with blocking agents requires occupancy of >90% of active integrin receptors [32]. Anti-integrin β2 antibodies have also shown unexpected side effects [33]. Additionally, whether transient activation of a fraction of native integrin receptors *in vivo*, as is expected from treatment with an activating agent, will have any significant biological effect in physiologically relevant settings remains an open question.

Therefore, there is a need for novel agents, such as antibodies, proteins, peptides, chemical compounds and small molecules, that selectively regulate the ligand binding and function of β2 integrins, including integrins CD11a/CD18, CD11b/CD18 and CD11c/CD18. Additionally, there is a need for agents that activate integrins (agonists) by targeting or binding to an allosteric regulatory site, such as the hydrophobic site-for-isoleucine (SILEN) pocket in CD11b/CD18, but not the ligand binding site on the integrin. Thus, there is a need for integrin activating agents that do not block ligand binding functions of integrins. Moreover, agents and methods to enhance or promote integrin-mediated cell-adhesion and cellular functions are highly desired. However, progress towards identifying such agonists has been slow, especially agonists that selectively target and activate β2 integrins, including CD11b/CD18, with only a few reported discoveries [34, 35].

The present invention describes a novel approach that involves integrin CD11b/CD18 activation, rather than its blockade, as a strategy for modulating CD11b/CD18 and also the function of cells (such as leukocytes, microglia, hepatocytes and lymphocytes), including their migration, recruitment and other biological functions. Such biological functions include generation of effector molecules, such as cytokines. It was strategized that various agents, such as small molecules, which are easily delivered *in vivo* and can be readily optimized for use in different mammals, would be the best approach for activating integrins. Here, it is shown that, without limitation, inflammatory disease can be reduced by CD11b/CD18 activation with novel small molecules. This shows that integrin activation is a novel, useful, pharmacologically targetable methodology to treat, without limitation, a variety of inflammatory and autoimmune diseases and conditions. This invention describes a novel strategy, as an alternative to the anti-adhesion strategy that is currently practiced in literature, for regulating the biological function of integrins and integrin-expressing cells. Many different types of agents can activate integrins, such as biologics, antibodies, antibody fragments, proteins, lipids, oligonucleotides and chemical compounds.

An important requirement of useful agonists and compositions that regulate β2 integrins, including CD11b/CD18, is that they do not negatively impact the cell, tissue and animal viability. It is an object of the present invention to describe such agonists, compositions and methods. In addition, it is an objective of the present invention to show that transient activation of a fraction of native receptors *in vivo*, as is expected from treatment with an agonist and method of this invention, has a biological effect in physiologically relevant model systems. In addition, the present invention provides other related advantages.

### SUMMARY OF THE INVENTION

The present invention provides for a pharmaceutical formulation of a β2 integrin agonist including an effective amount of a β2 integrin agonist and a pharmaceutically acceptable carrier.

The present invention provides for a method of activating β2 integrins by interacting the β2 integrins with a β2 integrin agonist.

The present invention also provides for a method of treating a patient by administering an effective amount of a β2 integrin agonist; and activating β2 integrins.

The present invention provides for a method of treating inflammation by administering a β2 integrin agonist to a patient with inflammation, activating β2 integrins, and reducing inflammation.

The present invention further provides for a method of treating and/or preventing renal ischemia-reperfusion (I/R) injury by administering a β2 integrin agonist to a patient, and activating β2 integrins.

The present invention provides for a method of reducing restenosis in a patient by administering a β2 integrin agonist prior to the insertion of a device, such as a stent, and activating β2 integrins.

The present invention provides for a method of reducing restenosis in a patient by administering a device, such as a stent, that is coated with a β2 integrin agonist and activating β2 integrins.

The present invention also provides for a method of performing an assay for the identification of small molecule modulators of β2 integrins by identifying sites in the β2 integrins that modulate activity of the β2 integrin, determining an exact three-dimensional structure of a binding pocket, and identifying small molecules that can interact with the binding pocket.

The present invention provides for a method of detecting a disease in a patient by administering a β2 integrin agonist, detecting binding of the β2 integrin agonist to a β2 integrin, and confirming the presence of the disease.

The present invention also provides for a method of improving the general wellness of a patient by administering an effective amount of a β2 integrin agonist, and activating β2 integrins.

### DESCRIPTION OF THE DRAWINGS

Other advantages of the present invention are readily appreciated as the same becomes better understood by reference to the following detailed description when considered in connection with the accompanying drawings wherein:

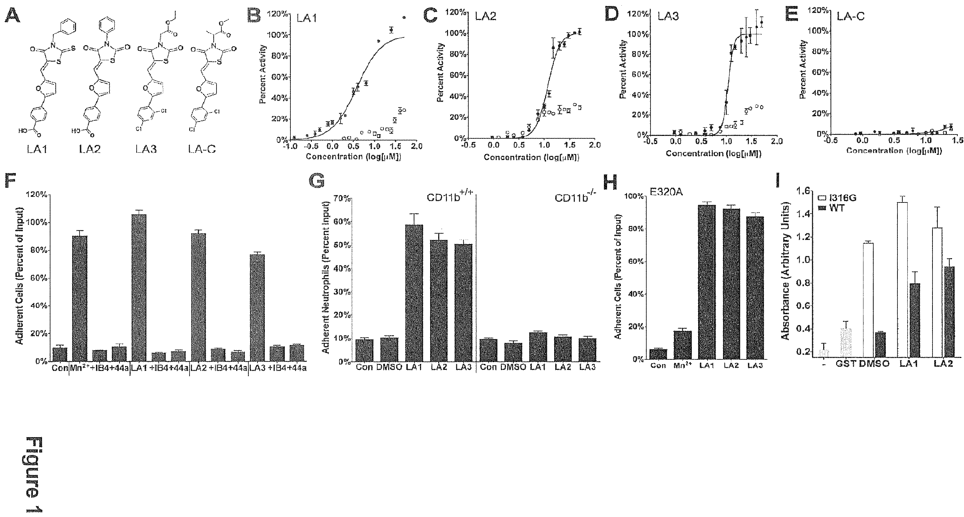

FIGURES 1A-1I show that leukadherins increase CD11b/CD18 dependent cell adhesion, 1A shows the chemical structures of LA1, LA2, LA3 and LA-C; 1B-1E show dose-response curves showing percentage of input K562 CD11b/CD18 (filled circles) and K562 (open circles) cells adhering to immobilized Fg in the presence of increasing amounts of LA1, LA2, LA3 and LA-C; 1F shows histograms showing LA1-3 induced adhesion of K562 CD1 1b/CD18 to Fg in the absence or presence of blocking antibodies IB4 and 44a, also shown is the reference level of adhesion in the presence of physiologic Ca2₊ and Mg2₊ ions (Con) and with known agonist Mn2₊. Data shown are mean₊SEM; 1G shows histograms showing adhesion of WT (CD11b_{+/+}) and CD11b_{-/-} neutrophils to immobilized Fg in the absence (DMSO) or the presence of LA1-3 as compared to basal levels of adhesion (Con), data shown are mean₊SEM; 1H shows histograms showing LA1-3 induced binding of K562 E320A cells to immobilized Fg in the absence or presence of blocking antibodies (IB4, 44a), also shown is the reference level of K562 E320A adhesion with Ca2₊ and Mg2₊ ions (Con) and Mn2₊, data shown are mean₊SEM; 1I shows histograms showing binding of recombinant GST-αA-domain constructs to the immobilized Fg in the absence (DMSO) or presence of LA1 and LA2, also shown is the background signal obtained in the absence of any protein (-) or with the GST construct alone (GST), data shown are mean+SEM;

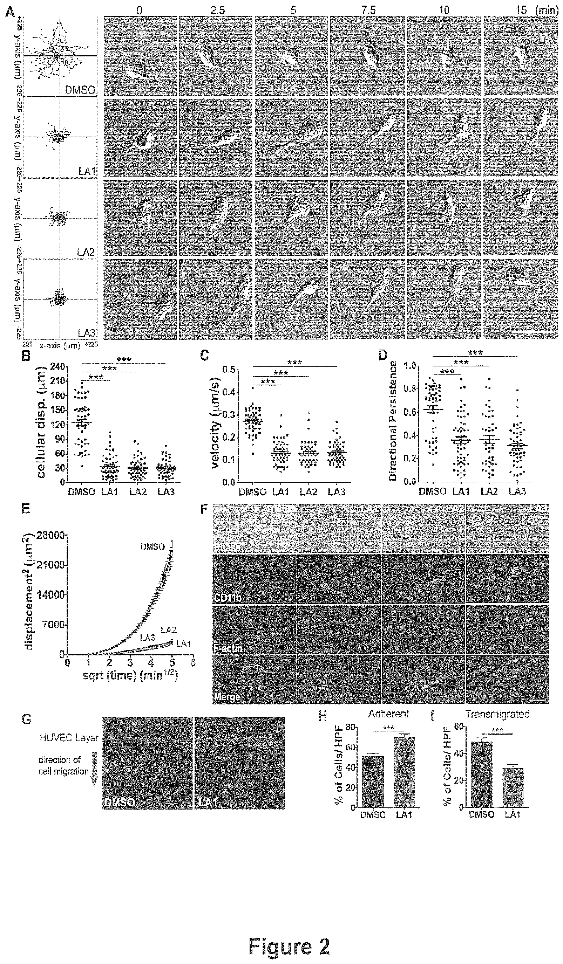

FIGURES 2A-2I show that leukadherins affect cell migration. Figure 2A. Track plots showing analysis of migrating WT neutrophils in Zigmond chambers in response to an *f*MLP gradient and in the absence (DMSO) or presence of compounds LA1, LA2 and LA3 (>50 cells/condition from >3 independent experiments/condition). Representative cell images at various time-points from time-lapse video microscopy are also shown. Scale bar represents 25 microm; 2B-2E show quantitative analyses of the mean displacement (2B), mean velocity (2C), directional persistence (2D) and mean displacement square plots (2E) show the effects of leukadherins on cell motility. Lines indicate mean ± SEM, *** p<0.0001; 2F show fluorescence images of CD11b localization in chemotaxing WT neutrophils in response to MLP and in the absence (DMSO) or presence of LA1, LA2 and LA3. Representative confocal and phase contrast images of migrating neutrophils stained for CD11b (green) and F-actin (red) are shown. Scale bar represents 5 microm; 2G-2I show transendothelial migration of THP-1 cells across HUVEC layer. 2G. Representative confocal images (10X) showing THP-1 cells (green) transmigrating across TNFa-stimulated HUVEC layer (red) in response to chemokine MCP-1 gradient in the absence (DMSO) or presence of LA1. 2H. Histogram showing quantification of THP-1 adhered to the HUVECs. 2I. Histograms showing quantification of transmigrated THP-1 cells. Data shown are mean ± SEM. *** p<0.0001;

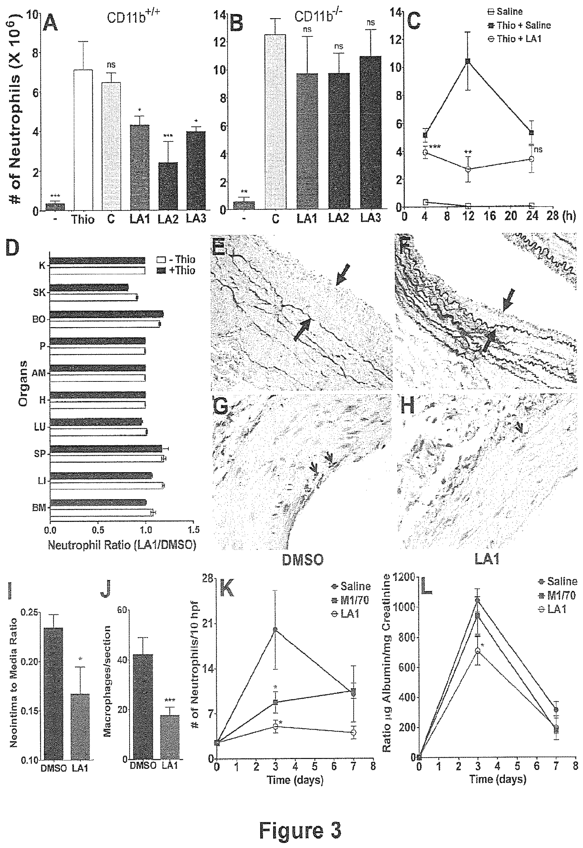

FIGURES 3A-3L show that leukadherins decrease inflammatory recruitment of leukocytes *in vivo* and preserve organ function *in vivo.* 3A-3B are bar graphs showing the total number of neutrophils in the peritoneal fluid of WT (A) or CD11b^{-/-} (B) mice 4 hours after intraperitoneal injection of thioglycollate from various treatment groups (thioglycollate alone or thioglycollate injection subsequent to administration of vehicle (C), LA1, LA2 or LA3). Saline injection was used as a control (n = 4-9/group), data shown are mean ± SEM. *p < 0.05, **p < 0.001, ***p < 0.0001, ns=not significant (one-way ANOVA). 3C. Graph showing the number of neutrophils in the peritoneal fluid of WT animals 4 hours, 12 hours and 24 hours after thioglycolate-injection in the absence (filled black square) or presence of LA1 (open red circles). Peritoneal neutrophils in the absence of thioglycolate-injection are also shown (open black squares). **p < 0.001, ***p < 0.0001, ns=not significant 3D. Bar graph showing ratio of neutrophils detected in various organs 4 hours after DMSO or LA1 treatment of WT mice (n=3/group) without (-Thio) or with thioglycolate-induced inflammation (+Thio). BM indicates bone marrow; LI, liver; SP, spleen; LU, lung; H, heart: AM, abdominal muscle; P, pancrease; BO, bowel; and K, kidney. Data shown are mean ± SD. 3E-F. Representative photomicrographs of arteries 21 days after balloon injury from rats treated with vehicle (DMSO) or LA1. Arrows point to the neoinitmal thickening. 3G-H. Photomicrographs of representative arteries 3 days after balloon injury from rats treated with DMSO or LA1. Arrows point to CD68⁺ macrophages. I. Bar graph showing the neointima to media ratio determined by morphometric analysis of the injured arteries from DMSO or LA1-treated rats (n=7-9/group). Data shown are mean ± SEM. *p<0.05. J. Bar graph showing quantification of macrophage infiltrates in injured arteries (3 days post injury) from rats treated with DMSO or LA1 (n=12/group). Data shown are mean + SEM. ***p < 0.0001. 3K-L. Graphs showing agonist LA1 ameliorates kidney injury better than antagonist M1/70. 3K. Graph displaying number of glomerular neutrophils in untreated (saline), antagonist- (M1/70) and LA1-treated mice at various time-points (n=3-4/group, except 0d time-point, where n=2). Data shown are mean ± SEM. *p < 0.05. 3K. Graph plotting measured proteinuria in untreated (saline), antagonist- (M1/70) and LA1-treated mice at various time-points (n=3-8/group, except 0d time-point, where n=2). Data shown are mean ± SEM. *p < 0.05.

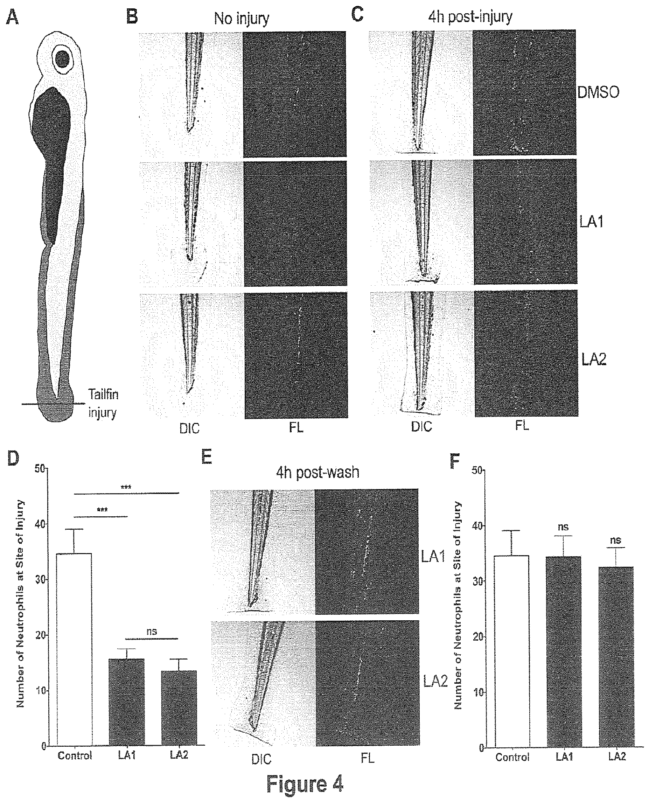

FIGURES 4A-4F show that blockade of inflammatory neutrophil recruitment can be reversed by removal of leukadherins, 4A shows a zebrafish tailfin injury model; 4B-4C are photomicrograph (left) and fluorescence images (right) of the 3dpf larvae tail without (4B) and with injury (4C), representative images from zebrafish treated with vehicle (DMSO), LA1 and LA2 show neutrophil (green) accumulation in the tail; 4D is a bar graph showing quantitation of the number of neutrophils near the site of tailfin injury in zebrafish larvae treated with vehicle (Control), LA1 and LA2 (n = 12-16 zebrafish larvae per group), data shown are mean+SEM. ***p<0.0001 (one-way ANOVA); 4E shows representative photomicrograph (left) and fluorescence images (right) of the larvae tail showing neutrophil (green) accumulation in the tail 4 hours after removal of compounds LA1 and LA2; 4F is a bar graph showing quantitation of the number of neutrophils near the site of tailfin injury 4 hours after removal of LA1 and LA2 (n = 8-12 larvae per group). Data shown are mean+SEM. ns=not significant (one-way ANOVA);

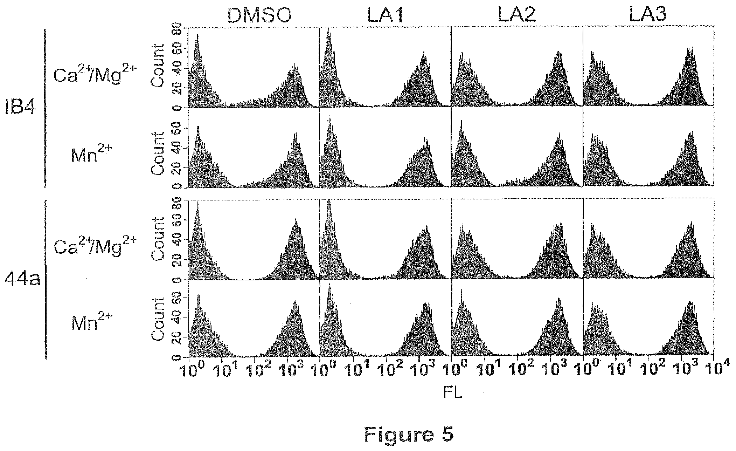

FIGURE 5 shows that leukadherins do not affect surface CD11b/CD18 expression, FACS analysis showing level of CD11b/CD18 expression on the surface of live K562 CD11b/CD18 using mAbs IB4 and 44a (black) and isotype IgG2a control mAb (gray), data shown are representative of at least three independent experiments;

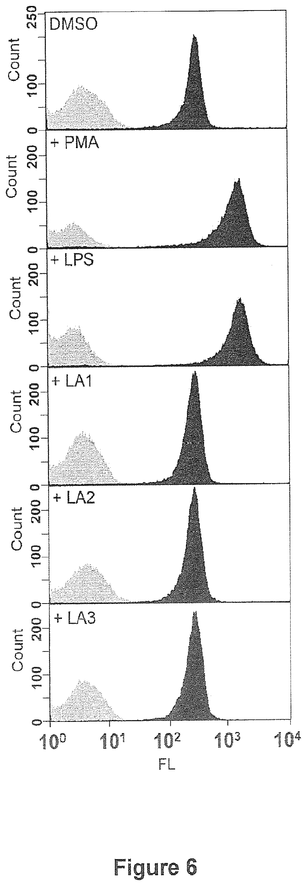

FIGURE 6 shows that leukadherins do not mobilize CD11b/CD18 from internal pools and do not affect surface CD11b/CD18 expression on human neutrophils. FACS analysis showing level of CD11b/CD18 expression on the surface of live human neutrophils using mAb IB4 (black) and isotype IgG2a control mAb (gray). Neutrophils were incubated with antibodies in the presence of vehicle (DMSO), PMA, LPS or leukadherins LA1-3 and analyzed as described in the methods section. Data shown are representative of two to three independent experiments. It shows that, while neutrophil activation with LPS and PMA leads to expected increase in the surface expression of CD11b/CD18, leukadherin treatment does not lead to any increase in the CD11b/CD18 surface-expression;

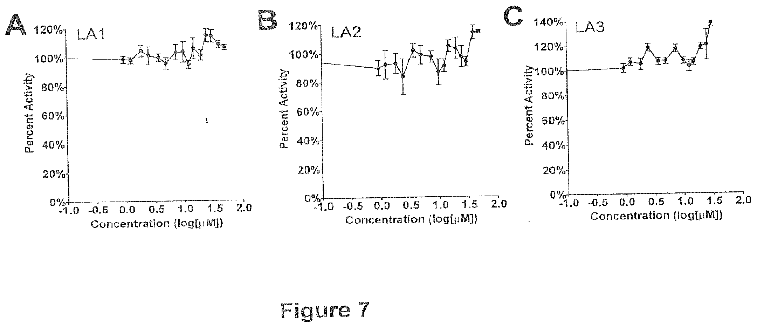

FIGURES 7A-7C show that leukadherins are true agonists and do not inhibit cell adhesion in the presence of agonist Mn²⁺-ions. A-C. Dose-response curves showing percentage of input K562 CD11b/CD18 cells adhering to immobilized Fg in the presence of agonist Mn²⁺-ions (1mM) and in the presence of increasing amounts of LA1 (**A**), LA2 (**B**) and LA3 (**C**). Data shown are mean ± SEM from three independent wells and is representative of at least two independent experiments;

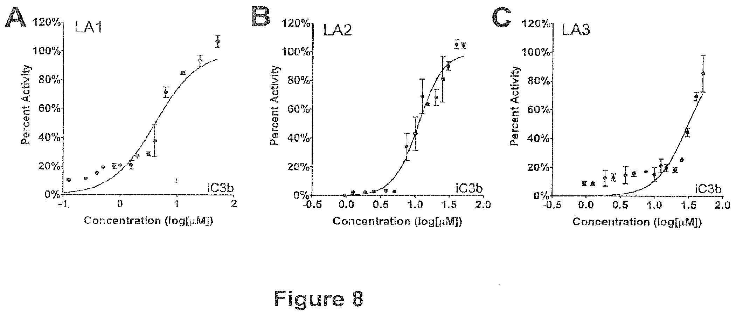

FIGURES 8A-8C show that leukadherin dependent CD11b/CD18 activation is independent of ligand type. A-C. Leukadherins increase binding of CD11b/CD18 to iC3b in a dose-dependent fashion. Dose-response curves showing percentage of input K562 CD11b/CD18 cells adhering to immobilized iC3b in the presence of increasing amounts of LA1 (A), LA2 (B) and LA3 (C). Data shown are mean ± SEM from six independent wells and is representative of at least three independent experiments;

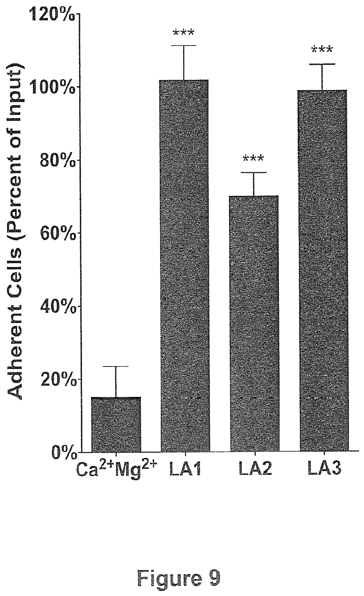

FIGURE 9 shows that leukadherin dependent CD11b/CD18 activation is independent of ligand type. Leukadherins increase binding of CD11b/CD18 to ICAM-1. Histograms showing the relative binding of K562 CD11b/CD18 cells (expressed as a percentage of input cells) adhering to immobilized ICAM-1 in the presence of buffer alone (containing 1mM Ca²⁺ and Mg²⁺ ions each) or leukadherins LA1, LA2 or LA3. Data shown are mean ± SEM from six to nine independent wells and is representative of at least three independent experiments. *** p < 0.0001;

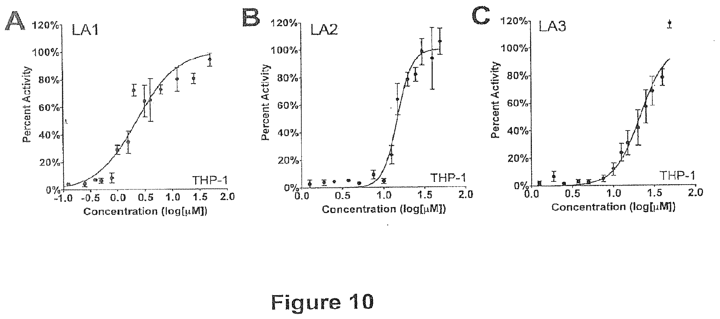

FIGURES 10A-10C show that leukadherin dependent CD11b/CD18 activation is independent of cell type. A-C. Leukadherins increase binding of THP-1 cells to Fg in a dose-dependent fashion. Dose-response curves showing percentage of input THP-1 cells adhering to immobilized Fg in the presence of increasing amounts of LA1 (A), LA2 (B) and LA3 (C). Data shown are mean ± SEM from six independent wells and is representative of at least three independent experiments;

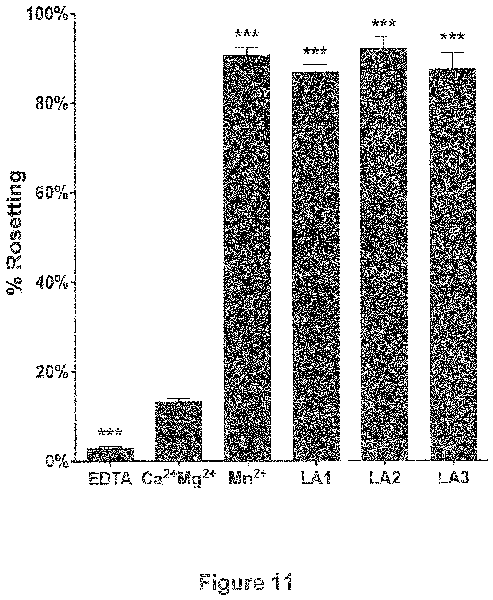

FIGURE 11 shows that leukadherins increase binding of iC3b-opsonized RBCs by K562 cells. Histograms showing the relative binding of iC3b-opsonized sheep red blood cells (RBCs) (EiC3bs) to CD11b/CD18 expressing K562 cells in the presence of EDTA (10 mM), control (1mM Ca²⁺ and Mg²⁺ ions each), activating Mn²⁺ ions (1mM) or leukadherins LA1-3 and expressed as percentage of total cells showing rosettes. Each histogram represents mean ± SEM of triplicate determinations from a representative experiment (one of three performed). *** p < 0.0001. As CD11b/CD18 is also a known phagocytosis receptor, these results show that LA1-3 also up-regulates the phagosytosis function of CD11b/CD18;

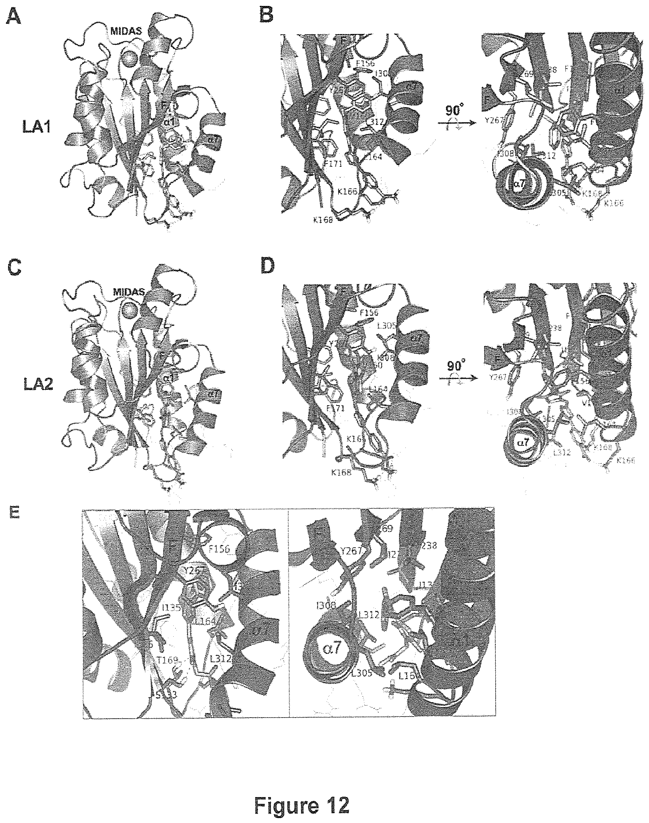

FIGURES 12A-12E are cartoon diagrams showing computational models for the binding of LA1-3 in an activation-sensitive region of the CD11b A-domain, 12A and 12C show a model of the αA-domain in its open conformation (copper ribbon) showing the docking of LA1 (green stick model) and LA2 (blue stick model) in the activation-sensitive F-α7 region, a metal ion at the MIDAS site is shown as a gray sphere; 12E shows a model of the αA-domain in its open conformation (blue ribbon) showing the docking of LA3 (yellow stick model) in the activation-sensitive F-α7 region. In agreement with studies with LA3 like compounds (12E), the leukadherins LA1 and LA2 were found to be oriented such that their most hydrophobic moieties interact with the hydrophobic pocket between helices α7 and α1 and the F-strand, hydrophobic residues forming the binding pocket (highlighted) include α7 Leu305, Ile308 and Leu312, α1 Phe156, V160, Leu164, F-strand Tyr267, Ile269, as well as other hydrophobic pocket residues including Ile236, Val238, Ile135, Phe137, Phe171, the hydophilic carboxylic acid moiety of the leukadherin compounds is oriented away from the hydrophobic pocket potentially forming ionic interactions with Lys166 and/or Lys168; 12B shows zoomed-in views of the activation-sensitive F-α7 region of the αA-domain (copper ribbon) from the docked structure (12A), the two views are rotated by 90° with respect to each other, interacting residues from the activation-sensitive hydrophobic region are shown as copper sticks and are labeled, dashed lines highlight potential hydrogen bond interactions between LA1 and the αA-domain; 12D shows zoomed-in views of the activation-sensitive F-α7 region of the αA-domain (copper ribbon) from the docked structure (12B), the two views are rotated by 90° with respect to each other, interacting residues from the activation-sensitive hydrophobic region are shown as copper sticks and are labeled, dashed lines highlight potential hydrogen bond interactions between LA2 and the αA-domain;

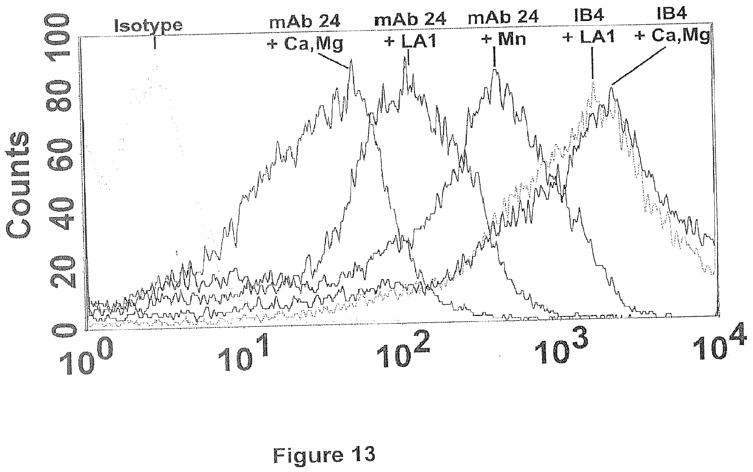

FIGURE 13 shows that leukadherins activate full-length integrin CD11b/CD18 on live K562 cells. FACS analysis showing the reactivity of activation-sensitive antibody mAb 24 with cell surface expressed CD11b/CD18 in the absence (Ca, Mg, dark gray histogram) or presence of agonist LA1 (red histogram) or Mn²⁺ ions (blue histogram). Level of CD11b/CD18 surface expression was analyzed using mAb IB4 in the absence (Ca, Mg) and presence of LA1 (black histogram), which shows no difference in total CD11b/CD18 surface expression. Binding by isotype control mAb (light gray) is also shown. Data shown are representative of at least three independent experiments. The data shows an clear increase in mAb24 reactivity with CD11b/CD18 in the presence of LA1 to levels previously observed with constitutively active CD11b/CD18;

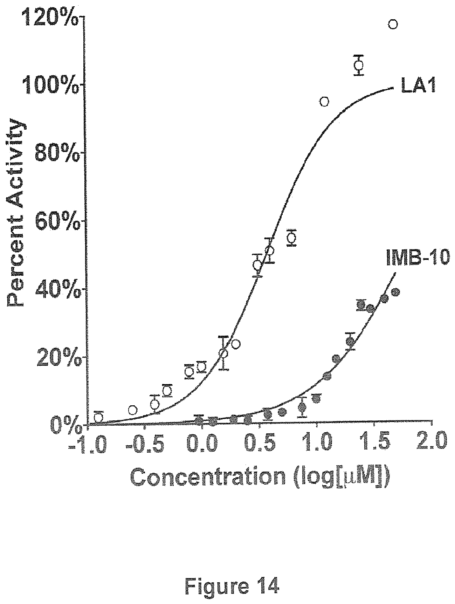

FIGURE 14 shows that leukadherins show higher affinity for CD11b/CD18 as compared to IMB-10. Dose-response curves showing percentage of input K562 CD11b/CD18 cells adhering to immobilized Fg in the presence of increasing amounts of LA1 and IMB-10 (4) with an EC₅₀ value of 4 mM for LA1 and >50 mM for IMB-10. Data shown are mean ± SEM from six independent wells and is representative of at least three independent experiments;

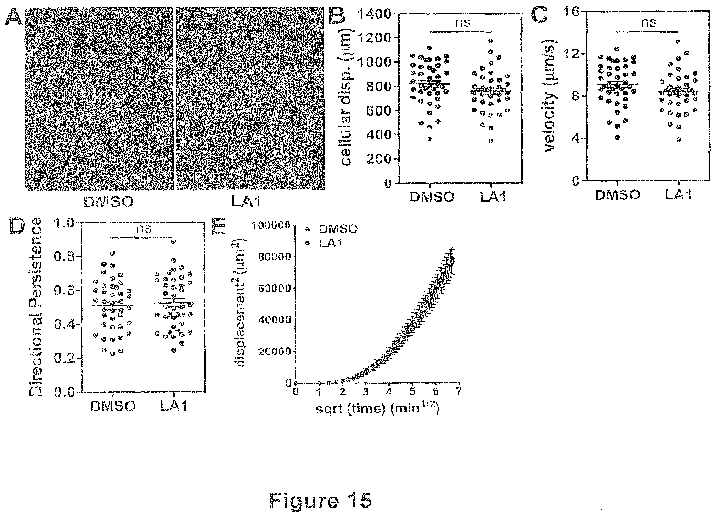

FIGURES 15A-15E show that leukadherins do not affect neutrophil migration in 3D gels *in vitro.* A. Still images from different time-lapse series imaging WT B6 neutrophils migrating towards *f*MLP gradient in 3D collagen gels in the absence (DMSO) or presence of leukadherin LA1. Migration tracks for 40 individual cells over a period of 45 minutes from each movie are also presented. B-E. Quantitative analyses of at least 40 neutrophils from each condition are also presented and do not show a significant difference in total cellular displacement over the 45 min recording period (B), migration velocity (C) and meandring index (D). Also, a plot of displacement squared versus square root of time (E) shows directed cell-migration under both conditions;

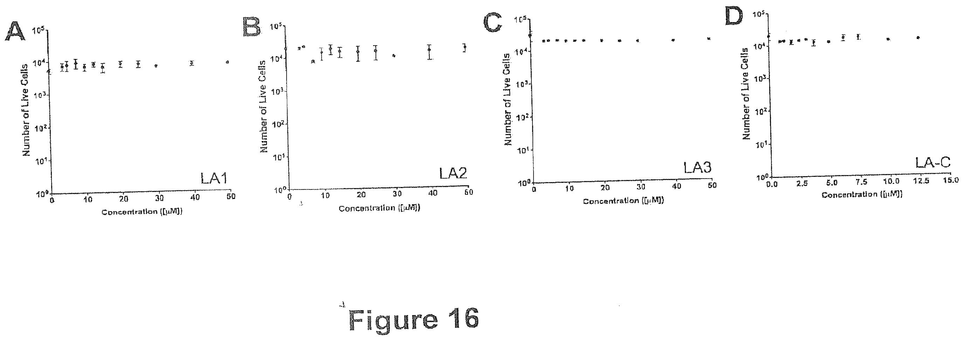

FIGURES 16A-16D show that leukadherins show no cytotoxicity *in vitro*, K562 CD11b/CD18 cells were incubated at 37 °C in the presence of increasing amounts of LA1 (16A), LA2 (16B), LA3 (16C) and LA-C (16D) and the number of live cells were determined after 24 hours, data shown are mean+SEM from an assay done in triplicate and is representative of at least two independent experiments;

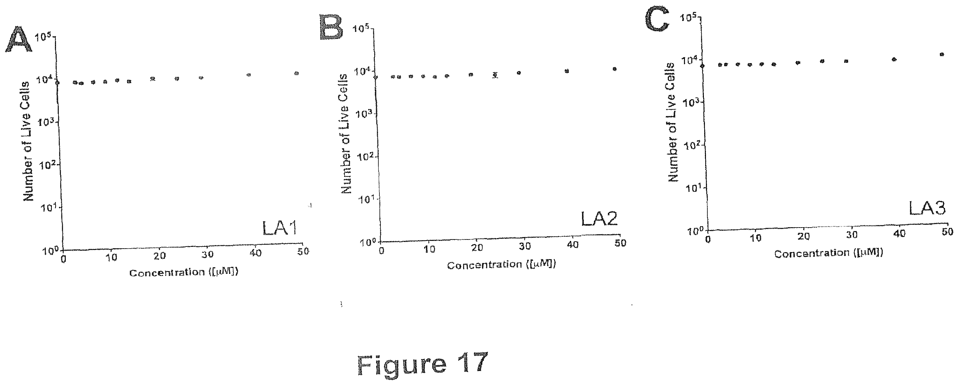

FIGURES 17A-17C show that leukadherins show no neutrophil cytotoxicity *in vitro.* WT B6 neutrophils were incubated at 37°C in the presence of increasing amounts of LA1 (A), LA2 (B) and LA3 (C) and the number of live cells were determined with the MTS reagent after a 4h total incubation. Data shown are mean ± SEM from an assay done in triplicate and is representative of at least two independent experiments. Results clearly show that these compounds are not toxic to primary neutrophils at concentrations as high as 50 microM.

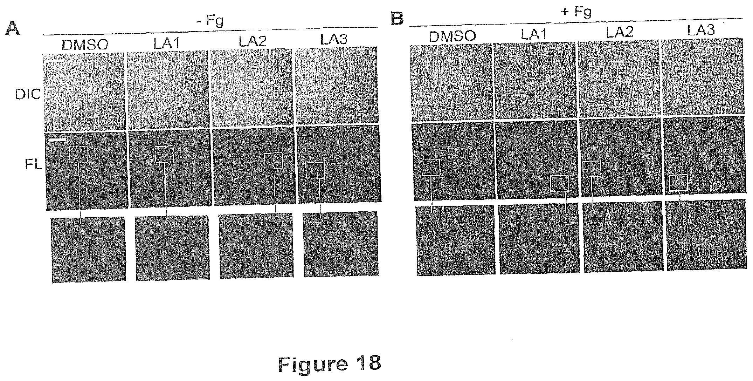

FIGURES 18A-18B show fluorescence images of CD11b clustering on K562 CD11b/CD18 cell surface to show that leukadherins do not induce integrin clustering and outside-in signaling. Integrin activation and ligand binding leads to clustering of integrins on the cell surface and initiates outside-in signaling (5, 6). As LA1-3 bind to and activate CD11b/CD18, it is conceivable that such binding alone may trigger integrin-mediated outside-in signaling, thus mimicking a ligand bound integrin state for the cell, which may have profound consequences on leukocyte lifetime and function. To test, we used confocal microscopy for imaging CD11b/CD18 clustering on cell surface (5). A-B. Cell suspensions were incubated with DMSO, LA1, LA2 or LA3 in the absence (A) or presence (B) of ligand Fg. Representative fluorescent and DIC images for cells stained for CD11b (Green) are shown. Also shown is a 3D representation of CD11b fluorescence intensity for selected cells, analyzed in ImageJ. Scale bar represents 20 mm. Cells displayed no detectable CD11b/CD18 macro clustering in the absence of ligand (A, DMSO), but showed high-degree of clustering upon addition of exogenous Fg (B, DMSO). Similarly, treatment with LA1-3 exhibited integrin macro-clustering only upon addition of external Fg, suggesting that LA1-3 are not integrin ligand mimics;

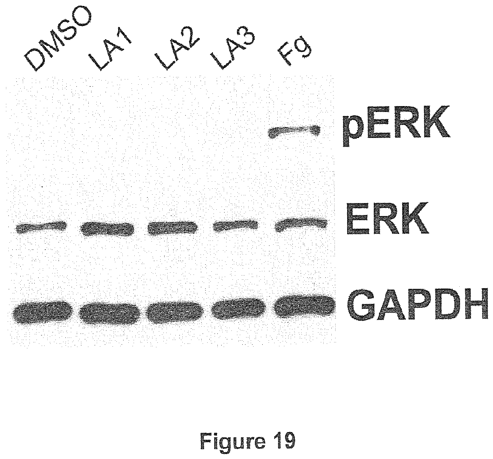

FIGURE 19 shows that leukadherins do not induce CD11b/CD18 mediated outside-in signaling. Integrin activation and ligand binding also initiates outside-in signaling, including the activation of p38 mitogen activated protein kinase/extracellular signal-regulated kinase (MAPK/ERK) pathways (5, 6), thereby mimicking the anchorage-dependent pro-survival signals in most cells (7). Additionally, it synergizes with inflammatory stimuli in potentiating pro-inflammatory NF-kB signaling (8-10). Furthermore, although known CD11b/CD18 agonists Mn²⁺ (11) and activating mAbs (12) and its ligands (5) induce ERK1/2 phosphorylation, cells from knock-in animals expressing mutant constitutively active integrins do not (13). To examine the effects of LA1-3, we analyzed ERK1/2 phosphorylation in LA1-3 treated cells. K562 CD11b/CD18 cells were incubated with DMSO (control), LA1, LA2, LA3 or ligand Fg and the cell lysates were subsequently analyzed by 1D SDS-PAGE followed by western blot for phosphorylated ERK1/2 (pERK), total ERK1/2 and GAPDH. It shows that LA1-3 treatment did not induce it (pERK) as opposed to incubation with ligand Fg or phorbol ester PMA (14). Thus, we conclude that leukadherins do not induce outside-in signaling leading to ERK phosphorylation in the absence or presence of ligand over and above the basal level in each case;

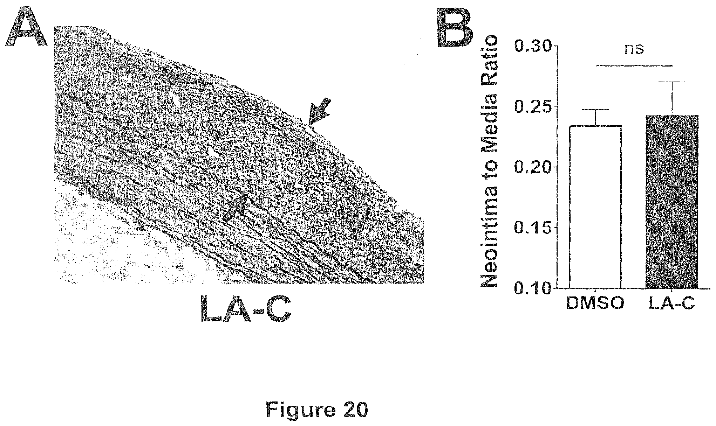

FIGURES 20A-20B show that control compound (LA-C) has no effect on neointimal thickening upon balloon injury in WT rats, 9A is a representative photomicrograph of rat arteries 21 days after balloon injury from animals treated with control compound LA-C. Arrows point to the neoinitmal thickening in the artery; 9B is a bar graph showing the neointima to media ratio determined by morphometric analysis of the injured rat arteries from DMSO and LA-C treated animals (n = 7-9 animals per group), data shown are mean+SEM. ns=not significant (one-way ANOVA);

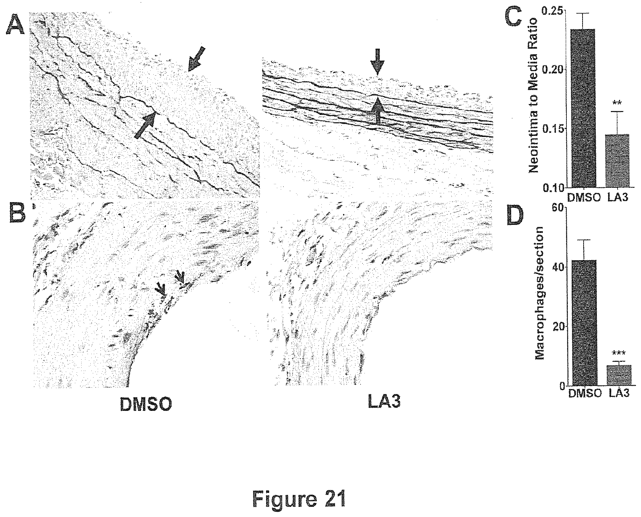

FIGURES 21A-21D shows that leaukadherin LA3 significantly reduces neointimal thickening after balloon injury in rats. A. Representative photomicrographs of rat arteries 21 days after balloon injury from animals treated with vehicle (DMSO) or LA3. Arrows point to the neoinitmal thickening. B. Photomicrographs of representative arteries 3 days after balloon injury from rats treated with DMSO or LA3. Arrows point to CD68⁺ macrophages. C. A bar graph showing the neointima to media ratio determined by morphometric analysis of the injured arteries from DMSO or LA3 treated rats (n = 7-9 per group). Data shown are mean±SEM. **p<0.001. D. Bar graphs showing quantitation of macrophage infiltrates in injured arteries (3 days post injury) from rats treated with DMSO or LA3 (n = 12 per group). Data shown are mean±SEM. ***p<0.0001;

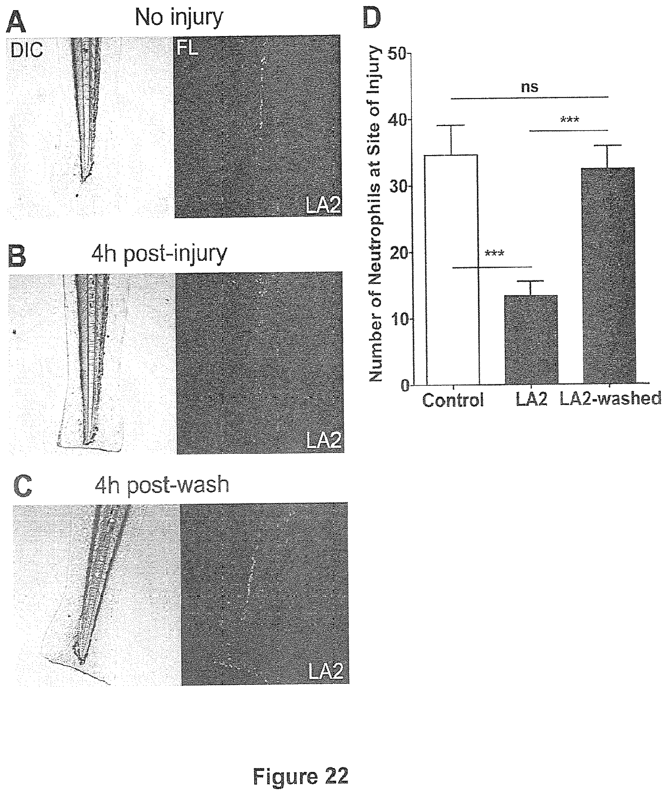

FIGURES 22A-22D show that leukadherin LA2 also prevents neutrophil recruitment to injured tissue and this blockade of inflammatory neutrophil recruitment can also be reversed by LA2 removal. A-B. Representative photomicrograph (left) and fluorescence images (right) of the 3dpf larvae tail without (A) and with injury (B) from zebrafish treated with LA2 show decreased neutrophil (green) accumulation in the tail, as compared to DMSO treated zebrafih (Figure 4A-B, main text). C. Representative photomicrograph (left) and fluorescence images (right) of the larvae tail showing neutrophil (green) accumulation in the tail 4h after removal of LA2. D. Bar graph showing quantitation of the number of neutrophils near the site of tailfin injury in zebrafish larvae treated with vehicle (Control), and LA2 and the number of neutrophils near the site of tailfin injury 4h after removal of LA2 (n = 12-16 zebrafish larvae per group). Data shown are mean±SEM. ***p<0.0001, ns=not significant;

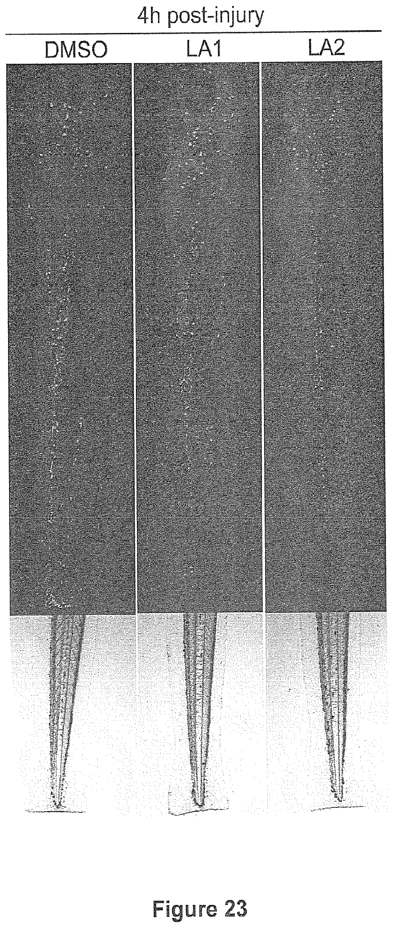

FIGURE 23 shows that Leukadherin treatment does not lead to loss of neutrophil cell number in zebrafish larvae, representative fluorescence images of the whole 3dpf zebrafish larvae (top) and photomicrographs of the tail (bottom) from injured fish show neutrophils (green) in each zebrafish larva. (n = 12-16 zebrafish larvae per group), LA1 and LA2 treated zebrafish show slightly higher green background in the fluorescence image due to compound autofluorescence;

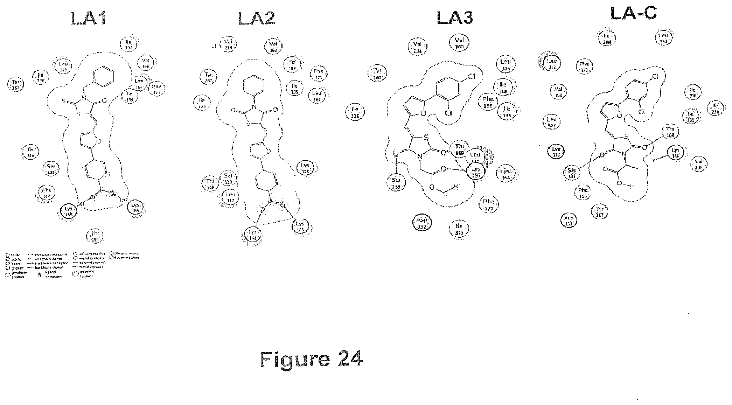

FIGURE 24 shows cartoon diagrams showing 2D projections from computational models for the binding of various leukadherins in an activation-sensitive region of the CD11b A-domain, the hydophilic carboxylic acid moiety of the leukadherin compounds is oriented away from the hydrophobic pocket potentially forming ionic interactions with Lys166 and/or Lys168;

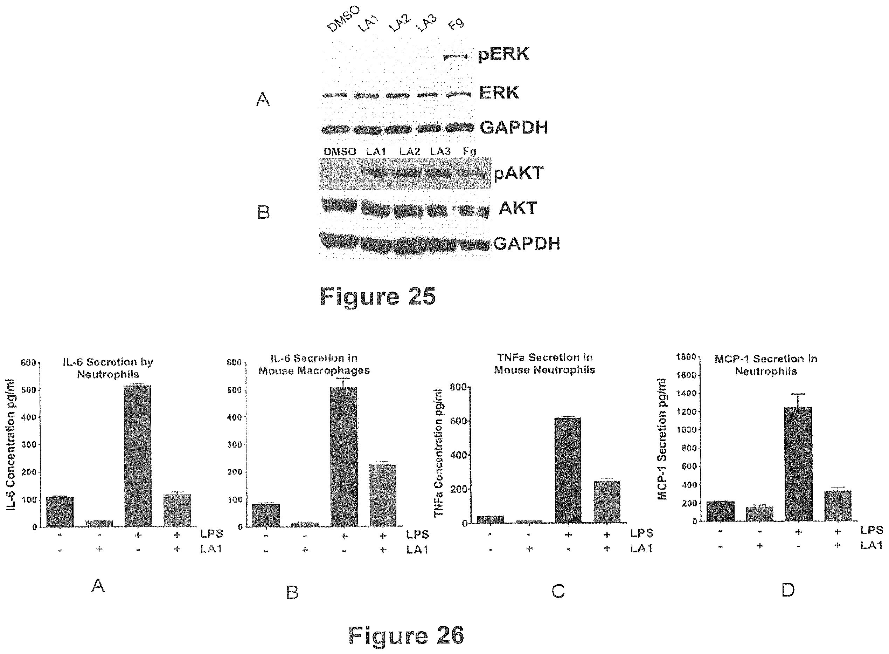

FIGURES 25A-25B show analysis of outside-in signaling, K562 CD1 1 b/CD18 cells were incubated with DMSO, LA1, LA2, LA3 or ligand Fg and the cell lysates were subsequently for phosphorylated ERK1/2 (pERK), total ERK1/2 (12A) and phosphorylated AKT (pAKT), total AKT and GAPDH (12B);
FIGURES 26A-26D are histograms showing secretion of pro-inflammatory factors by WT B6 macrophages and neutrophils upon stimulation with LPS (10ng/mL) in the absence and presence of LA1;

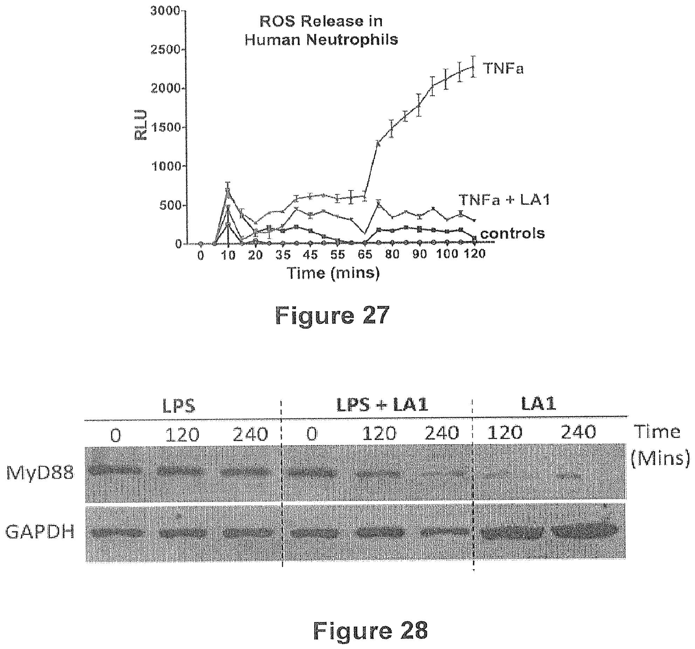

FIGURE 27 is a graph showing levels of ROS in human neutrophils in basal state (controls) and upon TNFa-activation in the absence (gray) or presence of LA1 (red line);
FIGURE 28 shows analysis of MyD88, human THP-1 cells were incubated with LPS, LPS and LA1 or LA1 alone for a set amount of time and the cell lysates were subsequently analyzed by 1D-SDS PAGE followed by western blotting for MyD88 and GAPDH;

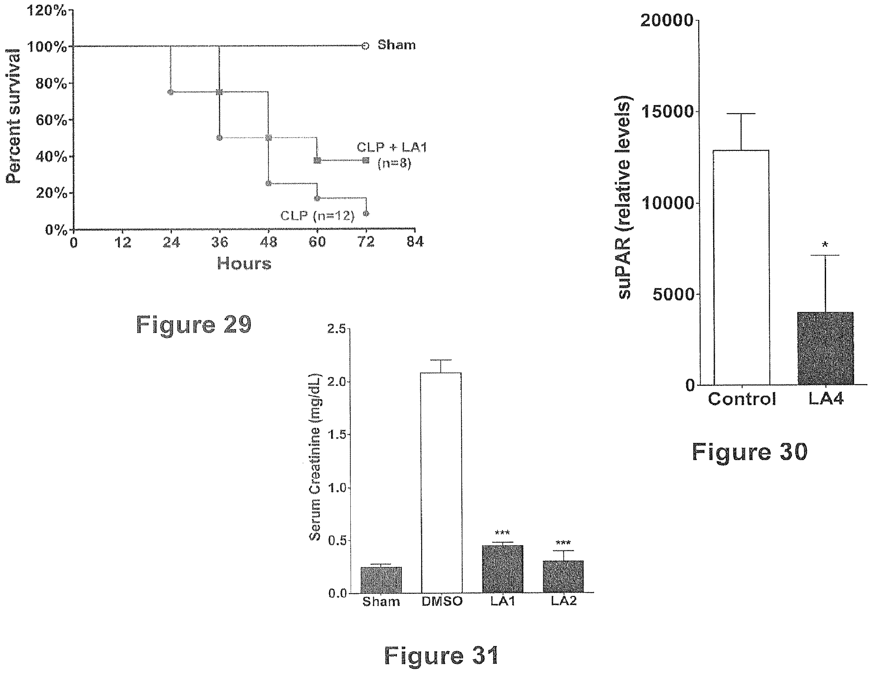

FIGURE 29 shows survival curves showing survival of WT mice upon CLP in the absence or presence of CD11b/CD18 agonist LA1;
FIGURE 30 is a histogram showing serum suPAR levels from vehicle treated (control) and leukadherin treated animals 8-days after initiation of progressive anti-GBM nephritis; and
FIGURE 31 is a graph showing kidney function in WT mice treated with DMSO, LA1 or LA2 30 min prior to induction of ischemia, sCr measurements were performed after 24 hours of reperfusion, ***p<0.0001 (n=5 mice/grp).

### DETAILED DESCRIPTION OF THE INVENTION

The present invention is generally directed to various agents, including chemical compounds termed leukadherins, and methods for activating β2 integrins, especially CD11b/CD18. In other words, the agents of the present invention act as agonists of β2 integrins, rather than antagonists. The agonists and methods are useful for treating inflammatory and autoimmune diseases, among other diseases.

### Definitions

The term "agent" refers to a chemical compound, peptide, protein, antibody, antibody fragment, lipid, nucleic acid or a polymer.

The term "alkoxy" refers to an alkyl group having an oxygen attached thereto. Representative alkoxy groups include methoxy, ethoxy, propoxy, tert-butoxy and the like.

The term "alkoxyalkyl" refers to an alkyl group substituted with an alkoxy group and can be represented by the general formula alkyl-O-alkyl.

The term "alkyl" refers to saturated aliphatic groups, including straight-chain alkyl groups, branched-chain alkyl groups, cycloalkyl (alicyclic) groups, alkyl-substituted cycloalkyl groups, and cycloalkyl-substituted alkyl groups. In preferred embodiments, a straight chain or branched chain alkyl has 30 or fewer carbon atoms in its backbone (e.g., C1-30 for straight chains, C3-30 for branched chains), and more preferably 20 or fewer.

Moreover, the term "alkyl" as used throughout the specification, examples, and claims is intended to include both unsubstituted and substituted alkyl groups, the latter of which refers to alkyl moieties having substituents replacing a hydrogen on one or more carbons of the hydrocarbon backbone, including haloalkyl groups such as trifluoromethyl and 2,2,2-trifluoroethyl, etc.

The term "Cx-y" when used in conjunction with a chemical moiety, such as, acyl, acyloxy, alkyl, alkenyl, alkynyl, or alkoxy is meant to include groups that contain from x to y carbons in the chain. C0alkyl indicates a hydrogen where the group is in a terminal position, a bond if internal. A C1-6alkyl group, for example, contains from one to six carbon atoms in the chain.

The terms "amine" and "amino" are art-recognized and refer to both unsubstituted and substituted amines and salts thereof, e.g., a moiety that can be represented by 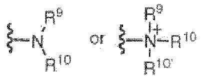 wherein R9, R10, and R10' each independently represent a hydrogen or a hydrocarbyl group, or R9 and R10 taken together with the N atom to which they are attached complete a heterocycle having from 4 to 8 atoms in the ring structure.

The term "aminoalkyl", as used herein, refers to an alkyl group substituted with an amino group.

The term "aralkyl", as used herein, refers to an alkyl group substituted with an aryl group.

The term "aryl" as used herein include substituted or unsubstituted single-ring aromatic groups in which each atom of the ring is carbon. Preferably the ring is a 5- to 7-membered ring, more preferably a 6-membered ring. The term "aryl" also includes polycyclic ring systems having two or more cyclic rings in which two or more carbons are common to two adjoining rings wherein at least one of the rings is aromatic, e.g., the other cyclic rings can be cycloalkyls, cycloalkenyls, cycloalkynyls, aryls, heteroaryls, and/or heterocyclyls. Aryl groups include benzene, naphthalene, phenanthrene, phenol, aniline, and the like.

The terms "carbocycle", "carbocyclyl", and "carbocyclic", as used herein, refers to a non-aromatic saturated or unsaturated ring in which each atom of the ring is carbon. Preferably a carbocycle ring contains from 3 to 10 atoms, more preferably from 5 to 7 atoms.

The term "carbocycloalkyl", as used herein, refers to an alkyl group substituted with a carbocycle group.

The term "ether", as used herein, refers to a hydrocarbyl group linked through an oxygen to another hydrocarbyl group. Accordingly, an ether substituent of a hydrocarbyl group can be hydrocarbyl-O-, Ethers can be either symmetrical or unsymmetrical. Examples of ethers include, but are not limited to, heterocycle-O-heterocycle and aryl-O-heterocycle. Ethers include "alkoxyalkyl" groups, which can be represented by the general formula alkyl-O-alkyl

The terms "halo" and "halogen" as used herein means halogen and includes chloro, fluoro, bromo, and iodo.

The term "heteroaralkyl", as used herein, refers to an alkyl group substituted with a hetaryl group.

The term "heteroaryl" include substituted or unsubstituted aromatic single ring structures, preferably 5- to 7-membered rings, more preferably 5-to 6-membered rings, whose ring structures include at least one heteroatom, preferably one to four heteroatoms, more preferably one or two heteroatoms. The term "heteroaryl" also includes polycyclic ring systems having two or more cyclic rings in which two or more carbons are common to two adjoining rings wherein at least one of the rings is heteroaromatic, e.g., the other cyclic rings can be cycloalkyls, cycloalkenyls, cycloalkynyls, aryls, heteroaryls, and/or heterocyclyls. Heteroaryl groups include, for example, pyrrole, furan, thiophene, imidazole, oxazole, thiazole, pyrazole, pyridine, pyrazine, pyridazine, and pyrimidine, and the like.

The term "heteroatom" as used herein means an atom of any element other than carbon or hydrogen. Preferred heteroatoms are nitrogen, oxygen, and sulfur.

The term "heterocycloalkyl", as used herein, refers to an alkyl group substituted with a heterocycle group.

The terms "heterocyclyl", "heterocycle", and "heterocyclic" refer to substituted or unsubstituted non-aromatic ring structures, preferably 3- to 10-membered rings, more preferably 3- to 7-membered rings, whose ring structures include at least one heteroatom, preferably one to four heteroatoms, more preferably one or two heteroatoms. The terms "heterocyclyl" and "heterocyclic" also include polycyclic ring systems having two or more cyclic rings in which two or more carbons are common to two adjoining rings wherein at least one of the rings is heterocyclic, e.g., the other cyclic rings can be cycloalkyls, cycloalkenyls, cycloalkynyls, aryls, heteroaryls, and/or heterocyclyls. Heterocyclyl groups include, for example, piperidine, piperazine, pyrrolidine, morpholine, lactones, lactams, and the like.

The term "hydroxyalkyl", as used herein, refers to an alkyl group substituted with a hydroxy group.

The term "leukadherin", as used herein, refers to agonist compounds of β2 integrin described herein that are characterized by a core furanyl thiazolidinone, a furanyl imidazolidinone, a furanyl oxazolidinone or a furanyl isooxazolidinone motif.

The term "lower" when used in conjunction with a chemical moiety, such as, acyl, acyloxy, alkyl; alkenyl, alkynyl, or alkoxy is meant to include groups where there are ten or fewer atoms in the substituent, preferably six or fewer. A "lower alkyl", for example, refers to an alkyl group that contains ten or fewer carbon atoms, preferably six or fewer. In certain embodiments, acyl, acyloxy, alkyl, alkenyl, alkynyl, or alkoxy substituents defined herein are respectively lower acyl, lower acyloxy, lower alkyl, lower alkenyl, lower alkynyl, or lower alkoxy, whether they appear alone or in combination with other substituents, such as in the recitations hydroxyalkyl and aralkyl (in which case, for example, the atoms within the aryl group are not counted when counting the carbon atoms in the alkyl substituent).

The terms "polycyclyl", "polycycle", and "polycyclic" refer to two or more rings (e.g., cycloalkyls, cycloalkenyls, cycloalkynyls, aryls, heteroaryls, and/or heterocyclyls) in which two or more atoms are common to two adjoining rings, e.g., the rings are "fused rings". Each of the rings of the polycycle can be substituted or unsubstituted. In certain embodiments, each ring of the polycycle contains from 3 to 10 atoms in the ring, preferably from 5 to 7.

The term "substituted" refers to moieties having substituents replacing a hydrogen on one or more carbons of the backbone. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds. For purposes of this invention, the heteroatoms such as nitrogen can have hydrogen substituents and/or any permissible substituents of organic compounds described herein which satisfy the valences of the heteroatoms. Substituents can include any substituents described herein, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain can themselves be substituted, if appropriate.

The term "thioalkyl", as used herein, refers to an alkyl group substituted with a thiol group.

The term "a cell" as used herein includes a plurality of cells. Administering a compound to a cell includes in vivo, ex vivo, and in vitro administration.

To "inhibit" or "suppress" or "reduce" a function or activity, such as cancer cell proliferation, is to reduce the function or activity when compared to otherwise same conditions except for a condition or parameter of interest, or alternatively, as compared to another conditions.

The term "modulate" as used herein includes the inhibition or suppression of a function or activity (such as cell proliferation) as well as the enhancement of a function or activity.

The phrase "pharmaceutically acceptable" is art-recognized. In certain embodiments, the term includes compositions, excipients, adjuvants, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filter, diluent, excipient, solvent or encapsulating material useful for formulating a drug for medicinal or therapeutic use. Each carrier must be "acceptable" in the sense of being compatible with other ingredients of the formulation and not injurious to the patient.

Some examples of materials which can serve as pharmaceutically acceptable carriers include (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "pharmaceutically acceptable salt" means an acid addition salt or a basic addition salt which is suitable for or compatible with the treatment of patients.

The term "pharmaceutically acceptable acid addition salt" as used herein means any non-toxic organic or inorganic salt of any base compounds represented by Formula I or II. Illustrative inorganic acids which form suitable salts include hydrochloric, hydrobromic, sulfuric and phosphoric acids, as well as metal salts such as sodium monohydrogen orthophosphate and potassium hydrogen sulfate. Illustrative organic acids that form suitable salts include mono-, di-, and tricarboxylic acids such as glycolic, lactic, pyruvic, malonic, succinic, glutaric, fumaric, malic, tartaric, citric, ascorbic, maleic, benzoic, phenylacetic, cinnamic and salicylic acids, as well as sulfonic acids such as p-toluene sulfonic and methanesulfonic acids. Either the mono or di-acid salts can be formed, and such salts can exist in either a hydrated, solvated or substantially anhydrous form. In general, the acid addition salts of compounds of Formula I or II are more soluble in water and various hydrophilic organic solvents, and generally demonstrate higher melting points in comparison to their free base forms. The selection of the appropriate salt will be known to one skilled in the art. Other non-pharmaceutically acceptable salts, e.g. oxalates, can be used, for example, in the isolation of compounds of Formula I or II for laboratory use, or for subsequent conversion to a pharmaceutically acceptable acid addition salt.

The term "pharmaceutically acceptable basic addition salt" as used herein means any non-toxic organic or inorganic base addition salt of any acid compounds represented by Formula I or II or any of their intermediates. Illustrative inorganic bases which form suitable salts include lithium, sodium, potassium, calcium, magnesium, or barium hydroxide. Illustrative organic bases which form suitable salts include aliphatic, alicyclic, or aromatic organic amines such as methylamine, trimethylamine and picoline or ammonia. The selection of the appropriate salt will be known to a person skilled in the art.

The term "preventing" is art-recognized, and when used in relation to a condition, such as a local recurrence (e.g., pain), a disease such as cancer, a syndrome complex such as heart failure or any other medical condition, is well understood in the art, and includes administration of a composition which reduces the frequency of, or delays the onset of, symptoms of a medical condition in a subject relative to a subject which does not receive the composition. Thus, prevention of cancer includes, for example, reducing the number of detectable cancerous growths in a population of patients receiving a prophylactic treatment relative to an untreated control population, and/or delaying the appearance of detectable cancerous growths in a treated population versus an untreated control population, e.g., by a statistically and/or clinically significant amount. Prevention of an infection includes, for example, reducing the number of diagnoses of the infection in a treated population versus an untreated control population, and/or delaying the onset of symptoms of the infection in a treated population versus an untreated control population. Prevention of pain includes, for example, reducing the magnitude of, or alternatively delaying, pain sensations experienced by subjects in a treated population versus an untreated control population. Prevention of neurological disorders includes, for example, reducing the magnitude of, or alternatively delaying, neurologic symptoms experienced by subjects in a treated population versus an untreated control population.

The term "solvate" as used herein means a compound of Formula I or II, or a pharmaceutically acceptable salt of a compound of Formula I or II, wherein molecules of a suitable solvent are incorporated in the crystal lattice. A suitable solvent is physiologically tolerable at the dosage administered. Examples of suitable solvents are ethanol, water and the like. When water is the solvent, the molecule is referred to as a "hydrate".

As used herein, and as well understood in the art, "treatment" is an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable. "Treatment" can also mean prolonging survival as compared to expected survival if not receiving treatment.

One aspect of the invention relates to a compound of Formula (I) 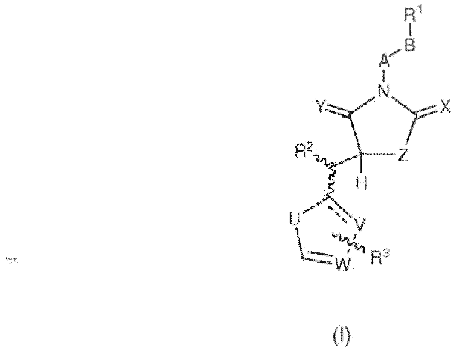 wherein
A is absent or is selected from alkyl and alkenyl;
B is absent or is selected from alkyl, alkenyl, O, S and NR4;
N is selected from nitrogen and CR4;
X and Y are independently selected from O and S;
Z is selected from CR4, O, S and NR4;
U, V and W are independently selected from CR4, O, S and NR4;
R1 and R3 are independently selected from acyl, alkyl, alkenyl, alkynyl, hydroxyalkyl, aminoalkyl, thioalkyl, aryl, aralkyl, carboxyaryl, alkoxyalkyl, alkoxyaryl, alcoxycarbonylaryl, aminoaryl, amidoaryl, haloaryl, heteroaryl, heteroaralkyl, carbocyclyl, heterocyclyl, heterocyclylalkyl, alkoxycarbonyl, alkylaminocarbonyl, alkylthiocarbonyl, sulfonate, alkylsulfonate, arylsulfonate, sulfone, alkylsulfone, arylsulfone, sulfoxide, alkylsulfoxide, arylsulfoxide, alkylsulfonamide, arylsulfonamide, and sulfonamide, piperidinyl, morpholinyl, pyrrolidinyl, phenyl, pyridyl, pyrimidinyl, furyl, thienyl, pyrrolyl, imidazolyl, triazolyl, pyrazolyl, thiazolyl, isoxazolyl, oxazolyl, oxadiazolyl, indolyl, naphthyl, quinolinyl, isoquinolinyl, quinoxalinyl, benzyl, benzofuryl, dibenzofuryl, benzthienyl, benzoxazolyl, benzothiazolyl, benzimidazolyl, pyridoimidazolyl, pyrimidoimidazolyl, pyridopyrazolyl, pyrazolopyrimidinyl, and any of which is optionally substituted with 1-6 independent substituents;
R2 selected from hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl; and
R4 is absent or is selected from hydrogen and alkyl.

In certain embodiments, Z is S.

In certain embodiments, X and Y are O. In certain other embodiments, X and Y are S. In certain other embodiments, X is S and Y is O. In certain other embodiments, X and Y are O and Z is S. In certain other embodiments, X, Y and Z are S. In certain other embodiments, X and Z are S and Y is O.

In certain embodiments, U is O and V and W are CR4. In certain such embodiments, R4 is hydrogen. In certain other embodiments, U is S and V and W are CR4. In certain such embodiments, R4 is hydrogen. In certain other embodiments, U is CR4, V is N and W is O. In certain such embodiments, R4 is hydrogen. In certain other embodiments, U is CR4, V is O and W is N. In certain such embodiments, R4 is hydrogen.

In certain embodiments, B is alkyl and A is absent. In certain such embodiments, R1 is selected from alkoxycarbonyl, aryl, heteroaryl, carbocyclyl, heterocyclyl and alkoxycarbonyl. In certain embodiments, B is methylene and A is absent. In certain such embodiments, R1 is selected from alkoxycarbonyl, aryl, heteroaryl, carbocyclyl, heterocyclyl and alkoxycarbonyl. In certain such embodiments, B is methylene and A is absent.

In certain embodiments, where A is alkyl and B is absent, R1 is alkoxycarbonyl.

In certain embodiments, A and B are both absent. In certain such embodiments, R1 is selected from alkoxycarbonyl, aryl, heteroaryl, carbocyclyl, heterocyclyl and alkoxycarbonyl.

In certain embodiments, R1 substituent is further substituted with 1-6 independent substituents.

In certain embodiments, R1 is selected from furan, phenyl, benzyl, tetrahydrofuran, tetrahydrothiophene, pyrrolidine, tetrahydropyran, tetrahydrothiopyran, piperidine, piperazine, and morpholine. In certain embodiments R1 is selected from tetrahydrofuran, tetrahydrothiophene, and pyrrolidine, preferably tetrahydrofuran.

In certain embodiments, R1 is phenyl, preferably substituted phenyl. In certain such embodiments, R1 is phenyl substituted one to five, preferably one to three, more preferably one or two times. In certain such embodiments, R1 is phenyl substituted with one or two, preferably one substituent independently selected from halogen, nitro, cyano, hydroxyl, thiol, amino, alkoxy, alkylamino, alkylthio, hydroxyalkyl, alkoxyalkyl, aminoalkyl, thioalkyl, and alkyl, more preferably from alkyl and halogen, e.g., from methyl, fluoro and chloro.

In certain embodiments, R2 is selected from hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl.

In certain embodiments, R3 is selected from alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl. In certain embodiments, R2 is hydrogen and R3 is selected from aryl, aralkyl, heteroaryl, heteroaralkyl, carbocyclyl, carbocyciylalkyl, heterocyclyl, and heferocyclylalkyl, preferably aryl, heteroaryl, carbocyclyl, and heterocyclyl.

In certain embodiments, R3 is heteroaryl selected from pyrrole, furan, pyrimidine, oxazole, isooxazole and thiophene, preferably furan. In certain embodiments, R3 is furan substituted one to three, preferably one to two times, more preferably once. In certain such embodiments, R3 is furan substituted once with a substituent selected from aryl, aralkyl, heteroaryl, heteroaralkyl, carbocyclyl, carbocyclylalkyl, heterocyclyl and heterocyclylalkyl, preferably aryl, heteroaryl, carbocyclyl, and heterocyclyl. In certain embodiments, R3 is furan substituted once with an aryl group, which itself is optionally substituted, preferably one to two times with alkyl, carboxyl, alkoxycarbonyl and halogen, e,g., chlorophenyl, dichlorophenyl, carboxyphenyl.

In certain embodiments, R3 is aryl, preferably phenyl. In certain such embodiments, R3 is phenyl substituted with one or two, preferably two substituents independently selected from halogen, nitro, cyano, hydroxyl, thiol, amino, alkoxy, alkylamino, alkylthio, hydroxyalkyl, alkoxyalkyl, aminoalkyl, thioalkyl, and alkyl. In certain such embodiments, R3 is phenyl substituted once with a halogen, preferably bromo.

One aspect of the invention relates to a compound of Formula (II) 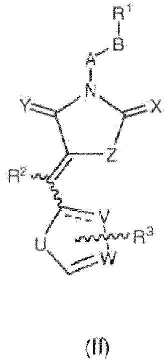 wherein
A is absent or is selected from alkyl and alkenyl;
B is absent or is selected from alkyl, alkenyl, O, S and NR4;
N is selected from nitrogen and CR4;
X and Y are independently selected from O and S;
Z is selected from CR4, O, S and NR4;
U, V and W are independently selected from CR4, O, S and NR4;
R1 and R3 are independently selected from acyl, alkyl, alkenyl, alkynyl, hydroxyalkyl, aminoalkyl, thioalkyl, aryl, aralkyl, carboxyaryl, alkoxyalkyl, alkoxyaryl, alcoxycarbonylaryl, aminoaryl, amidoaryl, haloaryl, heteroaryl, heteroaralkyl, carbocyclyl, heterocyclyl, heterocyclylalkyl, alkoxycarbonyl, alkylaminocarbonyl, alkylthiocarbonyl, sulfonate, alkylsulfonate, arylsulfonate, sulfone, alkylsulfone, arylsulfone, sulfoxide, alkylsulfoxide, arylsulfoxide, alkylsulfonamide, arylsulfonamide, and sulfonamide, piperidinyl, morpholinyl, pyrrolidinyl, phenyl, pyridyl, pyrimidinyl, furyl, thienyl, pyrrolyl, imidazolyl, triazolyl, pyrazolyl, thiazolyl, isoxazolyl, oxazolyl, oxadiazolyl, indolyl, naphthyl, quinolinyl, isoquinolinyl, quinoxalinyl, benzyl, benzofuryl, dibenzofuryl, benzthienyl, benzoxazolyl, benzothiazolyl, benzimidazolyl, pyridoimidazolyl, pyrimidoimidazolyl, pyridopyrazolyl, pyrazolopyrimidinyl, and any of which is optionally substituted with 1-6 independent substituents;
R2 selected from hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl; and
R4 is absent or is selected from hydrogen and alkyl.

In certain embodiments, Z is S.

In certain embodiments, X and Y are O. In certain other embodiments, X and Y are S. In certain other embodiments, X is S and Y is O. In certain other embodiments, X and Y are O and Z is S. In certain other embodiments, X, Y and Z are S. In certain other embodiments, X and Z are S and Y is O.

In certain embodiments, U is O and V and W are CR4. In certain such embodiments, R4 is hydrogen. In certain other embodiments, U is S and V and W are CR4. In certain such embodiments, R4 is hydrogen. In certain other embodiments, U is CR4, V is N and W is O. In certain such embodiments, R4 is hydrogen. In certain other embodiments, U is CR4, V is O and W is N. In certain such embodiments, R4 is hydrogen.

In certain embodiments, B is alkyl and A is absent. In certain such embodiments, R1 is selected from alkoxycarbonyl, aryl, heteroaryl, carbocyclyl, heterocyclyl and alkoxycarbonyl. In certain embodiments, B is methylene and A is absent. In certain such embodiments, R1 is selected from alkoxycarbonyl, aryl, heteroaryl, carbocyclyl, heterocyclyl and alkoxycarbonyl, In certain such embodiments, B is methylene and A is absent.

In certain embodiments, where A is alkyl and B is absent, R1 is alkoxycarbonyl.

In certain embodiments, A and B are both absent. In certain such embodiments, R1 is selected from alkoxycarbonyl, aryl, heteroaryl, carbocyclyl, heterocyclyl and alkoxycarbonyl.

In certain embodiments, R1 substituent is further substituted with 1-6 independent substituents.

In certain embodiments, R1 is selected from furan, phenyl, benzyl, tetrahydrofuran, tetrahydrothiophene, pyrrolidine, tetrahydropyran, tetrahydrothiopyran, piperidine, piperazine, and morpholine. In certain embodiments R1 is selected from tetrahydrofuran, tetrahydrothiophene, and pyrrolidine, preferably tetrahydrofuran.

In certain embodiments, R1 is phenyl, preferably substituted phenyl. In certain such embodiments, R1 is phenyl substituted one to five, preferably one to three, more preferably one or two times. In certain such embodiments, R1 is phenyl substituted with one or two, preferably one substituent independently selected from halogen, nitro, cyano, hydroxyl, thiol, amino, alkoxy, alkylamino, alkylthio, hydroxyalkyl, alkoxyalkyl, aminoalkyl, thioalkyl, and alkyl, more preferably from alkyl and halogen, e.g., from methyl, fluoro and chloro.

In certain embodiments, R2 is selected from hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl.

In certain embodiments, R3 is selected from alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl. In certain embodiments, R2 is hydrogen and R3 is selected from aryl, aralkyl, heteroaryl, heteroaralkyl, carbocyclyl, carbocyclylalkyl, heterocyclyl, and heterocyclylalkyl, preferably aryl, heteroaryl, carbocyclyl, and heterocyclyl.

In certain embodiments, R3 is heteroaryl selected from pyrrole, furan, pyrimidine, oxazole, isooxazole and thiophene, preferably furan. In certain embodiments, R3 is furan substituted one to three, preferably one to two times, more preferably once. In certain such embodiments, R3 is furan substituted once with a substituent selected from aryl, aralkyl, heteroaryl, heteroaralkyl, carbocyclyl, carbocyclylalkyl, heterocyclyl and heterocyclylalkyl, preferably aryl, heteroaryl, carbocyclyl, and heterocyclyl. In certain embodiments, R3 is furan substituted once with an aryl group, which itself is optionally substituted, preferably one to two times with alkyl, carboxyl, alkoxycarbonyl and halogen, e.g., chlorophenyl, dichlorophenyl, carboxyphenyl.

In certain embodiments, R3 is aryl, preferably phenyl. In certain such embodiments, R3 is phenyl substituted with one or two, preferably two substituents independently selected from halogen, nitro, cyano, hydroxyl, thiol, amino, alkoxy, alkylamino, alkylthio, hydroxyalkyl, alkoxyalkyl, aminoalkyl, thioalkyl, and alkyl. In certain such embodiments, R3 is phenyl substituted once with a halogen, preferably bromo.

In certain embodiments, a compound of Formula II is selected from

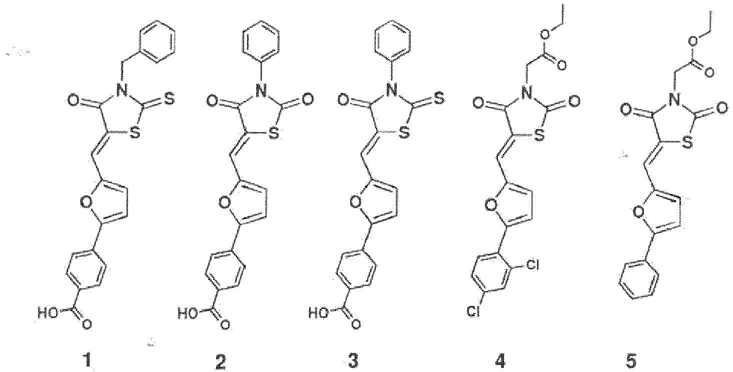

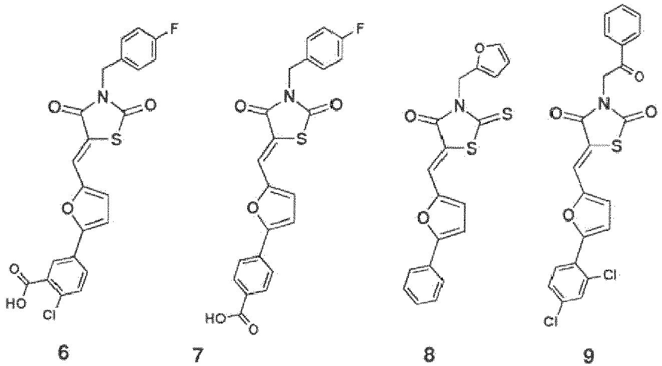

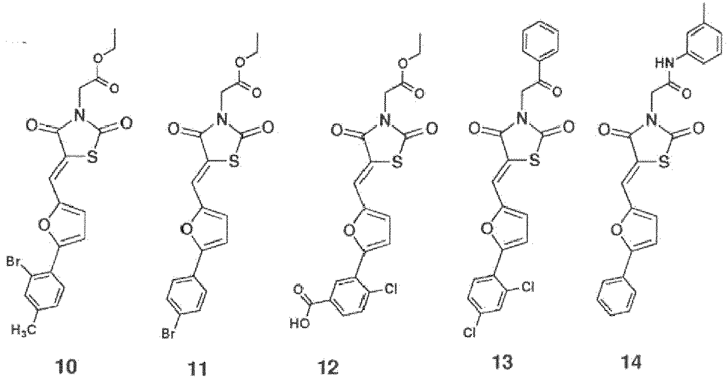

In certain embodiments, a compound of Formula II selected from the following compounds is less preferred

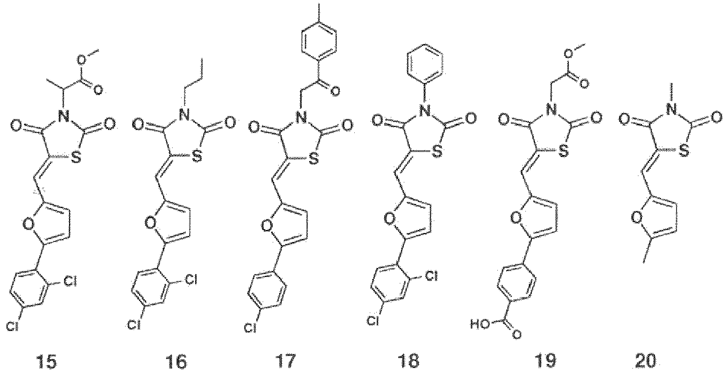

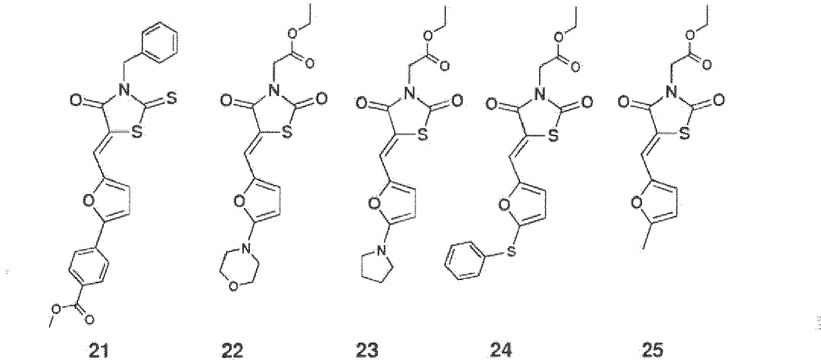

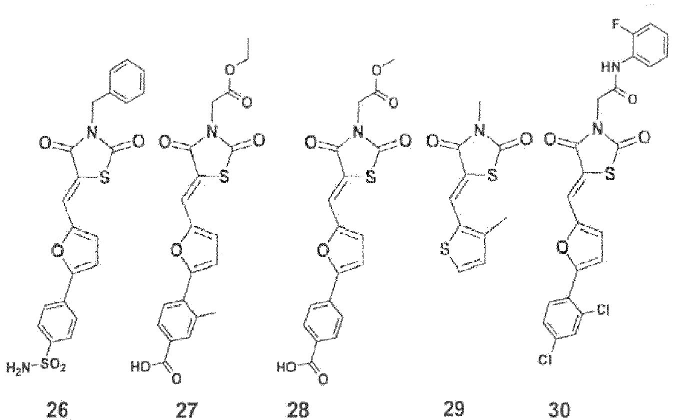

One aspect of the invention relates to a compound of Formula (III) 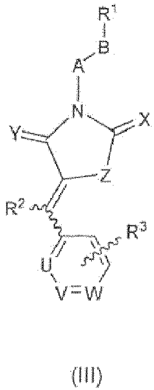 wherein
A is absent or is selected from alkyl and alkenyl;
8 is absent or is selected from alkyl, alkenyl, O, S and NR4;
N is selected from nitrogen and CR4;
X and Y are independently selected from O and S;
Z is selected from CR4, O. S and NR4;
U, V and W are independently selected from CR4, O, S and NR4;
R3 is 1-6 independent substituents present at position(s) 1-6 of the aryl ring;
R1 and R3 are independently selected from acyl, alkyl, alkenyl, alkynyl, hydroxyalkyl, aminoalkyl, thioalkyl, aryl, aralkyl, carboxyaryl, alkoxyalkyl, alkoxyaryl, alcoxycarbonylaryl, aminoaryl, amidoaryl, haloaryl, heteroaryl, heteroaralkyl, carbocyclyl, heterocyclyl, heterocyciylalkyl, alkoxycarbonyl, alkylaminocarbonyl, alkylthiocarbonyl, sulfonate, alkylsulfonate, arylsulfonate, sulfone, alkylsulfone, arylsulfone, sulfoxide, alkylsulfoxide, arylsulfoxide, alkylsulfonamide, arylsulfonamide, and sulfonamide, piperidinyl, morpholinyl, pyrrolidinyl, phenyl, pyridyl, pyrimidinyl, furyl, thienyl, pyrrolyl, imidazolyl, triazolyl, pyrazolyl, thiazolyl, isoxazolyl, oxazolyl, oxadiazolyl, indolyl, naphthyl, quinolinyl, isoquinolinyl, quinoxalinyl, benzyl, benzofuryl, dibenzofuryl, benzthienyl, benzoxazolyl, benzothiazolyl, benzimidazolyl, pyridoimidazolyl, pyrimidoimidazolyl, pyridopyrazolyl, pyrazolopyrimidinyl, and any of which is optionally substituted with 1-6 independent substituents;
R2 selected from hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl; and
R4 is absent or is selected from hydrogen and alkyl,

In certain embodiments, Z is S.

In certain embodiments, X and Y are O. In certain other embodiments, X and Y are S. In certain other embodiments, X is S and Y is O. In certain other embodiments, X and Y are O and Z is S. In certain other embodiments, X, Y and Z are S. In certain other embodiments, X and Z are S and Y is O.

In certain embodiments, U is N and V and W are CR4. In certain such embodiments, R4 is hydrogen. In certain other embodiments, V is N and U and W are CR4. In certain such embodiments, R4 is hydrogen. In certain other embodiments, W is N and V and V are CR4. In certain such embodiments, R4 is hydrogen.

In certain embodiments, B is alkyl and A is absent. In certain such embodiments, R1 is selected from alkoxycarbonyl, aryl, heteroaryl, carbocyclyl, heterocyclyl and alkoxycarbonyl. In certain embodiments, B is methylene and A is absent. In certain such embodiments, R1 is selected from alkoxycarbonyl, aryl, heteroaryl, carbocyclyl, heterocyclyl and alkoxycarbonyl. In certain such embodiments, B is methylene and A is absent.

In certain embodiments, where A is alkyl and B is absent, R1 is alkoxycarbonyl.

In certain embodiments, A and B are both absent. In certain such embodiments, R1 is selected from alkoxycarbonyl, aryl, heteroaryl, carbocyclyl, heterocyclyl and alkoxycarbonyl.

In certain embodiments, R1 substituent is further substituted with 1-6 independent substituents.

In certain embodiments, R1 is selected from furan, phenyl, benzyl, tetrahydrofuran, tetrahydrothiophene, pyrrolidine, tetrahydropyran, tetrahydrothiopyran, piperidine, piperazine, and morpholine. In certain embodiments R1 is selected from tetrahydrofuran, tetrahydrothiophene, and pyrrolidine, preferably tetrahydrofuran.

In certain embodiments, R1 is phenyl, preferably substituted phenyl. In certain such embodiments, R1 is phenyl substituted one to five, preferably one to three, more preferably one or two times. In certain such embodiments, R1 is phenyl substituted with one or two, preferably one substituent independently selected from halogen, nitro, cyano, hydroxyl, thiol, amino, alkoxy, alkylamino, alkylthio, hydroxyalkyl, alkoxyalkyl, aminoalkyl, thioalkyl, and alkyl, more preferably from alkyl and halogen, e.g., from methyl, fluoro and chloro.

In certain embodiments, R2 is selected from hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl.

In certain embodiments, R3 is selected from alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl. In certain embodiments, R2 is hydrogen and R3 is selected from aryl, aralkyl, heteroaryl, heteroaralkyl, carbocyclyl, carbocyclylalkyl, heterocyclyl, and heterocyclylalkyl, preferably aryl, heteroaryl, carbocyclyl, and heterocyclyl.

In certain embodiments, R3 is heteroaryl selected from pyrrole, furan, pyrimidine, oxazole, isooxazole and thiophene, preferably furan. In certain embodiments, R3 is furan substituted one to three, preferably one to two times, more preferably once. In certain such embodiments, R3 is furan substituted once with a substituent selected from aryl, aralkyl, heteroaryl, heteroaralkyl, carbocyclyl, carbocyclylalkyl, heterocyclyl and heterocyclylalkyl, preferably aryl, heteroaryl, carbocyclyl, and heterocyclyl. In certain embodiments, R3 is furan substituted once with an aryl group, which itself is optionally substituted, preferably one to two times with alkyl, carboxyl, alkoxycarbonyl and halogen, e.g., chlorophenyl, dichlorophenyl, carboxyphenyl.

In certain embodiments, R3 is aryl, preferably phenyl. In certain such embodiments, R3 is phenyl substituted with one or two, preferably two substituents independently selected from halogen, nitro, cyano, hydroxyl, thiol, amino, alkoxy, alkylamino, alkylthio, hydroxyalkyl, alkoxyalkyl, aminoalkyl, thioalkyl, and alkyl. In certain such embodiments, R3 is phenyl substituted once with a halogen, preferably bromo.

The compounds of the present invention can have an inherent end-to-end polarity such that compounds are more polar on one end of the molecule, for example on the top-end (N-substituted side of the thiazolidine ring) or the bottom-end (substituted furanyl side of the thiazolidine ring) as drawn, as compared to the other end of the molecule. Alternatively, compounds with two polar ends can be disfavored.

The compounds of the invention can be in a pure or substantially pure single configuration, such as a Z configuration.

The β2 integrin agonist compounds of the invention preferably occupy a binding pocket in the αA-domain of CD11b/CD18. The compounds of the invention occupy a binding pocket in the αA-domain in a manner similar to as described in FIGURES 12A-12-E and 24. More specifically, the compounds can interact with the hydrophobic pocket lined by residues L312, I308 L305 (α7 helix), L164, V160, F156 (α1 helix), and Y267, I269, I236, V238. I236, 1135 (central beta sheet) of integrin, as described in Example 1 below. The compounds of the invention can interact with polar residues or side-chains of the amino acids of αA-domain. For example, the more polar end of the compounds can interact with residues lysine 166 or lysine 168 of αA-domain. The more non-polar end of the compounds of the invention can occupy a hydrophobic pocket in the binding site in αA-domain, and the more polar end can occupy a pocket in the binding site in αA-domain, such that the polar end is more exposed to the solvent.

Certain compounds of the present invention can exist in particular geometric or stereoisomeric forms. The present invention contemplates all such compounds, including cis- and trans-isomers, R- and S-enantiomers, diastereomers, (d)-isomers, (I)-isomers, the racemic mixtures thereof, and other mixtures thereof, as falling within the scope of the invention. Additional asymmetric carbon atoms can be present in a substituent such as an alkyl group. All such isomers, as well as mixtures thereof, are intended to be included in this invention.

If, for instance, a particular enantiomer of a compound of the present invention is desired, it can be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts can be formed with an appropriate optically active acid or base, followed by resolution of the diastereomers thus formed by fractional crystallization or chromatographic means well known in the art, and subsequent recovery of the pure enantiomers.

The agonists of the present invention can also be labeled, for example, with a radiolabel by incorporation within the structure 3H or 14C or a radioactive halogen such as 125I, or with a label by linking the agonist structure with biotin or a fluorphore, with the help of a linker.

One aspect of the invention relates to non-blocking, activating anti-integrin antibodies or their fragments as agonists. Non-limiting examples of such antibodies include anti-CD11b antibody M18/2 (4, 5), anti-CD11b antibody ED7, anti-CD118 antibody ED8 (6), anti-GD11b antibody VIM12 (7), anti-CD11a antibody CBR LFA1/2 (8), anti-GD11a antibody NKI-L16 (9), anti-CD18 antibody KIM185 (10), anti-CD18 antibody KIM127 (11), anti-CD18 antibody 24 (12), anti-CD18 antibody NG2B12 (6), anti-CD18 antibody MEM48 (13). Additionally, such antibodies or their fragments can be modified for use in various animals, such as making humanized antibodies as is well-known in the art.

The agonists described above can be included in a pharmaceutical formulation. The compounds can include their derivatives, pharmaceutically-acceptable salts thereof, or hydrates thereof. Preferably, the pharmaceutical formulation includes an acceptable diluent, carrier, excipient, or adjuvant along with the active compounds 1-30.

The pharmaceutical formulation can further include other active compounds or agents to modulate or treat a condition. These other active compounds or agents can act synergistically with the agonists of the present invention. Therefore, both the other compounds/agents and agonists of the present invention can be given at lower doses than when used individually. This allows many known drugs that are potentially harmful when used at higher doses to be used effectively at lower doses while being safe for a patient.

For example, the agonists of the present invention can be used in combination with a TNF-α blocker, such as, but not limited to, ENBREL® (etanercept, Amgen), REMIGADE® (infliximab, Centocor Ortho Biotech, Inc.), or HUMIRA (adalimumab, Abbott Laboratories). Dosing for etanercept can be 25 to 50 mg weekly, dosing for infliximab can be 3 mg/kg every two to eight weeks, and dosing for adalimumab can be 40 mg every two weeks, and can be lowered in combination with the compounds of the present invention.

The agonists of the present invention can be used in combination with anti-inflammatory drugs, such as, but not limited to, non-steroidal anti-inflammatory drugs (NSAIDS) such as salicylates (aspirin), acetic acid derivatives (indomethacin), propionic acid derivatives (ibuprofen or naproxen), or Coxll inhibitors such as celecoxib (Celebrex®) or rofecoxib (Vioxx®). Dosing is generally 10 to 3200 mg for anti-inflammatory drugs per day, which can be lowered in combination with the agonists of the present invention.

The agonists of the present invention can be used in combination with anti-cancer compounds such as, but not limited to, cilengitide, a cyclo(RGDfV) peptide. Dosing can generally be 120 to 2400 mg/m², and can be lowered in combination with the compounds of the present invention.

The agonists of the present invention can be used in combination with anti-rejection drugs, such as, but not limited to, tacrolimus, cyclosporine, and various steroids. Dosing for tacrolimus can be 0.25 mg to 1 mg per day and can be lowered in combination with the agonists of the present invention.. Dosing for cyclosporine can be 1 to 12 mg/kg per day and can be lowered in combination with the agonists of the present invention.

The agonists of the present invention can be used in combination with anti-clotting drugs, such as, but not limited to, warfarin (COUMADIN®, Bristol-Myers Squibb), heparin, aspirin, ticlopidine (TICLID®, Roche Pharmaceuticals, Inc.), clopidogrel (PLAVIX®, Bristol-Myers Squibb/Sanofi Pharmaceuticals), dipyridamole (PERSANTINE®, Boehringer Ingelheim Pharmaceuticals, Inc.), and glycoprotein IIb/IIIa receptor agonists. Dosing for warfarin, for example, can be 1 to 10 mg daily, aspirin can be 50 to 6000 mg daily, ticlopidine can be 250 mg twice daily, clopidogrel can be 75 to 300 mg daily, dipyridamole can be 75 to 100 mg four times daily, and can be lowered in combination with the agonists of the present invention.

The agonists of the present invention can be used in combination with steroids, such as, but not limited to, tobramycin, dexamethasone, neomycin, hydrocortisone, prednisone, and erythromycin. Dosing for dexamethasone can be 0.75 to 9 mg daily, dosing for neomycin can be 3 to 12 g daily, dosing for prednisone can be 5 to 60 mg daily, dosing for erythromycin can be 30 mg to 4 g daily, and can be lowered in combination with the agonists of the present invention.

The agonists of the present invention can be used in combination with sphingosine-1-phosphate receptor modulators such as fingolimod (GILENYA™, Novartis Pharmaceuticals Corporation). Dosing can be 0.25 mg to 5 mg daily, and can be lowered in combination with the agonists of the present invention.

The agonists of the present invention can further be used in combination with a drug-eluting device media. Such devices include stents and catheters. The present invention also provides for a method of reducing injury in a patient due to insertion of a device, such as a stent or a catheter. More specifically, the agonists can be administered prior to insertion of a device in a patient, during an insertion as well as after insertion. As described in Example 1 below, the agonists act to reduce accumulation of leukocytes at the site of vascular injury with a device and decrease neointimal thickening. Prior art methods provided such results only with β2 integrin antagonists. Thus, it is unexpected that an agonist would also provide this function.

The agonists of the present invention can further be used as a coating for medical devices, such as stents and catheters. The present invention also provides for a method of reducing injury in a patient by administering a device to the patient that is coated with a β2 integrin agonist.

The agonists of the present invention can further be used for reducing stenosis in patients. The present invention also provides for a method of reducing stenosis in a patient by administering a β2 integrin agonist to the patient.

The agonists of the present invention can further be used for improving the patency of vascular access in a patient. The present invention also provides for a method of improving the patency of vascular access in a patient by administering a β2 integrin agonist to the patient.

The agonists of the present invention can further be- used for reducing stenosis in arteriovenous fistula (AVF) as well as in arteriovenous graft (AVG) in a patient. The present invention also provides for a method of reducing stenosis in arteriovenous fistula (AVF) as well as in arteriovenous graft (AVG) in a patient by administering a β2 integrin agonist to the patient.

Most generally, the present invention provides for a method of activating β2 integrins by interacting the β2 integrin with an agonist, preferably one of the compounds described herein. Prior art methods only provide β2 integrin activation via expression of mutant receptors in cells, where a hundred percent of integrin receptors contained activating mutant. Thus, it is unexpected that an agonist that binds to only a fraction of all wild type receptors would also provide this function.

Also, the present invention provides for a method of treating a patient by administering a β2 integrin agonist, preferably one of the agonists described herein, and activating β2 integrins. The activation can also be thought of as an over-activation of the β2 integrins.

The β2 integrin agonists can further be selective for CD11b/CD18 over other β2 integrins as described below. Alternatively, the β2 integrin agonists can activate any desired β2 integrin or integrins.

There are many different functions that the agonists of the present invention provide with respect to activating β2 integrins, and especially integrin CD11b/CD18. The agonists of the invention can regulate the function of β2 integrins. This regulation can be of the conformation of β2 integrins or the organization of β2 integrins in a cell or on a cell membrane, such as dimerization or multimurization with itself or other proteins and substances. The agonists of the invention can regulate the function of a cell by regulating β2 integrins. The agonists can increase cell adhesion and reduce cell recruitment into an inflamed tissue. Increased cell adhesivity can reduce the lateral motility of cells (including cellular chemotaxis). Increased cell adhesivity due to agonist treatment can reduce the transendothelial migration (TEM) of cells. The compositions and methods described herein affect leukocyte recruitment. They can achieve this, for example, by increasing leukocyte slow rolling and adhesivity to the inflammed endothelium, which can be reversed with a blocking antibody. In other words, increasing cell adhesion makes the cells more sticky so that they can not move outside of blood vessels to enter tissue, such as injured or inflamed tissue. This is a functional blockade of β2 integrin-mediated function through activation of the β2 integrins instead of through actually blocking. Prior art methods provided such results only with β2 integrin antagonists. Thus, it is unexpected that an agonist would also provide this function.

The agonists of the invention can regulate other functions of a cell by regulating β2 integrins *in vitro* and *in vivo.* For example, the agonists reduce the levels of chemical factors secreted by cells. Such factors include, without limitation, inflammatory factors, for example TNF-α, IL-1β, IL-6, IFN-γ. soluble uPAR and microparticles among others. The agonists and methods described herein reduce the levels of secreted factors by β2 integrin-expressing cells. The agonists and methods described herein reduce the levels of secreted factors by cells, such as vascular endothelial cells, that themselves do not express β2 integrins but that interact with β2 integrin expressing cells, such as leukocytes or microglia. The compositions and methods described herein increase the level of secreted factors. Such factors include anti-inflammatory factors, for example IL-10 among others.

The compositions and methods described herein can also modify the signaling pathways in cells. The agonists can modify intracellular signaling pathways in β2 integrin-expressing cells (including leukocytes, among others). Such pathways include, without limitation, the NF-kB pathway, AKT pathway, MAPK pathway, Toll-like receptor signaling pathway, cytokine receptor signaling pathways, among others. The compounds and methods of this invention activate β2 integrins, which induces intracellular signaling that synergizes or opposes other signaling pathways in the cells. The compositions and methods described herein modify the signaling pathways in cells that interact with the β2-integrin expressing cells. Such cells include other leukocyte subsets, lymphocytes, endothelial cells, astrocytes and hippocampal cells among others. The compositions and methods described herein modify the signaling pathways in cells that interact with factors secreted by the β2-integrin expressing cells (such as leukocytes, lymphocytes, endothelial cells, among others).

The agonists of the invention can also increase the binding of β2 integrins, especially integrin CD11b/CD18, to its ligands, either *In vitro* or *in vivo.* These ligands can be ICAM-1, ICAM-2, ICAM-3, iC3b, fibrinogen, Factor X, fibrin, uPAR, or GP Ibα. The binding of the agonist with the protein modulates at least one function normally associated with binding of a natural ligand of the protein. Such functions include rolling of cells, such as leukocytes on vascular endothelium, binding of cells with vascular endothelium, crawling of cells on vascular endothelium, translocation of cells through vascular endothelium, infiltration of cells into intimal tissue, release of one or more soluble factors from cells, release of a chemotactic factor from cells, release of a growth factor from cells, cell-binding-associated release of a chemotactic factor from a tissue, cell-binding-associated release of a growth factor from a tissue, cell-binding-associated release of one or more soluble factors from a tissue, change in the level of one or more soluble factors in circulation and change in the level of one or more insoluble factors. The soluble factors include cytokines, such as, but not limited to, pro-inflammatory cytokines, anti-inflammatory cytokines, IL-1β, IL-6 and IL-10, TNF-α, or IFN-γ. The agonist affected cells include cells such as, but not limited to, leukocytes, hepatocytes, microglia, certain T- and B-cells, stem cells, pluripotent cells and leukemia cells.

The agonists can also correct or reduce the functional deficit in cells that express mutant forms of β2 integrins. For example, mutations in CD11b have been linked to lupus and lupus nephritis. The agonists of the present invention can reduce or overcome the functional defects in cells, organisms, and animals that carry mutant forms of the β2 integrins.

The agonists of the present invention can also more generally modulate biological function *in vitro* or *in vivo,* such as, but not limited to, gene expression, epigenetic profile, protein expression, protein levels, protein modifications, post-translational modifications, and signaling. Preferably, the agonists of the invention modulate biological function in leukocytes, microglia and stem cells. Alternatively, the agonists of the invention can modulate biological function in other cells or tissues.

The agonists of the present invention can also modulate other biological functions *in vitro* or *in vivo,* such as, differentiation of stem cells, differentiation of pluripotent cells, maintenance of cells in culture or in long term storage, mobilization of cells, such as leukocytes from bone marrow into circulation or endothelial progenitor cells to sites of inflammation or injury and increasing retention of certain cells into their niches, such as leukemia cells in the marrow.

There are many diseases or conditions that can be treated or prevented that are associated with the activity of β2 integrins, such as, but not limited to inflammation (including, but not limited to, acute and chronic inflammation), inflammatory skin diseases, immune-related disorders, autoimmune diseases, burn, immune deficiency, acquired immune deficiency syndrome (AIDS), myeloperoxidase deficiency, Wiskott-Aldrich syndrome, chronic kidney disease, chronic granulomatous disease, hyper-IgM syndromes, leukocyte adhesion deficiency, iron deficiency, Chediak-Higashi syndrome, severe combined immunodeficiency, diabetes, obesity, hypertension, HIV, wound-healing, remodeling, scarring, fibrosis, stem cell therapies, cachexia, encephalomyelitis, multiple schlerosis, Alzheimers, psoriasis, lupus, rheumatoid arthritis, immune-related disorders, radiation injury, transplantation, cell transplantation, cell transfusion, organ transplantation, organ preservation, cell preservation, asthma, irritable bowel disease, irritable bowel syndrome, ulcerative colitis, colitis, bowel disease, cancer, leukemia, ischemia-reperfusion injury, stroke, neointimal thickening associated with vascular injury, bullous pemphigoid, neonatal obstructive nephropathy, familial hypercholesterolemia, atherosclerosis, dyslipidemia, aortic aneurisms, arteritis, vascular occlusion, including cerebral artery occlusion, complications of coronary by-pass surgery, myocarditis, including chronic autoimmune myocarditis and viral myocarditis, heart failure, including chronic heart failure (CHF), cachexia of heart failure, myocardial infarction, stenosis, restenosis after heart surgery, silent myocardial ischemia, post-implantation complications of left ventricular assist devices, thrombophlebitis, vasculitis, including Kawasaki's vasculitis, giant cell arteritis, Wegener's granulomatosis, traumatic head injury, post-ischemic-reperfusion injury, post-ischemic cerebral inflammation, ischemia-reperfusion injury following myocardial infarction, cerebral malaria, and cardiovascular disease. These diseases can be treated or prevented by administering the agonists of the present invention in a pharmaceutical formulation to a patient. The agonists can perform the various functions as described above to treat the diseases in the patient.

The treatment of the patient can be confirmed by detecting the activation of the β2 integrins. This can be accomplished by taking a sample from the patient and performing an assay, such as detection of levels of β2 integrin expression on the surface of leukocytes in the biological sample or the level of activated β2 integrin on such cells. Another approach for confirming the treatment of a patient is to evaluate levels of the other known markers in the patient that are typically associated with the said disease, such as levels of IL-6 in the blood samples, or disease symptoms in the patient.

In a specific method of treating inflammation, a β2 integrin agonist as described herein can be administered to a patient with inflammation to activate β2 integrins and reduce inflammation. In one mechanism of action, the agonist can suppress pro-inflammatory cytokine expression, such as IL-6, in leukocytes by phosphorylating Akt, as described in Example 1 below. Furthermore, the agonist can decrease secretion of soluble factors by neutrophils and macrophages that results in a decrease in inflammation. The agonist can also delay neutrophil recruitment by increasing neutrophil adhesivity near sites of inflammation and decreasing neutrophil motility. Also, the agonist activated CD11b/CD18 increases leukocyte adhesion, which decreases leukocyte crawling and transendothelial migration and, thus, reduced recruitment into the inflamed/injured tissue. CD11b activation induces intracellular signaling that provides a negative feedback loop on pro-inflammatory signals and pathways. Additionally, while some activating antibodies exist in literature that activate CD11b/CD18, such "antibodies" may not be ideal. The present invention provides that such activating antibodies need to be modified for use as agonists of this invention such that they do not induce integrin clustering or other harmful signaling pathways in cells, but instead induce pathways similar to what is induced with the leukadherins.

The present invention also provides for a method of treating and/or preventing renal ischemia-reperfusion (I/R) injury by administering a β2 integrin agonist to a patient and activating β2 integrins. Renal I/R injury is a major cause of acute renal failure after renal transplantation, major surgery, trauma, septic shock, and hemorrhagic shock. The β2 integrin agonist can be administered prior to surgery. Alternatively, the agonist can be administered after renal transplantation, major surgery, trauma, septic shock, and hemorrhagic shock. By administering the agonist, there is a significant reduction in sCr levels, leading to a reno-protective effect.

The present invention also provides for a method of performing an assay for the identification of small molecule modulators of β2 integrins, especially CD11b/CD18. More specifically, sites and domains can be identified in β2 integrins, especially in integrin CD11b/CD18, in integrin CD11c/CD18, in integrin CD11d/CD18, or in integrin CD11a/CD18, that modulate activity of the β2 integrin and the exact three-dimensional structure of the binding pocket can be determined, which can be used to derive more selective and/or potent binders. For example, a complex of CD11b/CD18 with a binding agonist can be prepared and analyzed, e.g., by x-ray crystallography, nuclear magnetic resonance, or other suitable means, to identify the binding site of CD11b/CD18 that interacts with the agonist. The assay can also be a cell-adhesion-based high-throughput screening assay to identify agonists that inhibit or enhance cell adhesion. For example, libraries of potential small molecule agonists can be screened against the particular β2 integrin. Non-adherent cells can be removed by gravity by inverting the assay plates. This step is performed instead of washing the assay plates. Cell nuclei can be stained and then imaged to quantitate the number of adherent cells, and then an agonist can be identified. The assay can be an *in vitro* or *in vivo* assay.

Computer-based modeling algorithms can be used to analyze the structures and conformations of agonists that bind β2 integrins, especially CD11b/CD18, to identify structural features that contribute to successful binding. Such information can be analyzed in conjunction with information about the structure or conformation of CD11b/CD18 or a binding pocket thereof, such as structural information obtained by analysis of CD11b/CD18 using analytical techniques such as x-ray crystallography or nuclear magnetic resonance, to analyze interactions between binding agonists and the binding pocket they interact with. Such analysis can be used to predict the portion of CD11b/CD18 that interacts with the agonist, to select agonists that possess structural features correlated with desired binding activity from a library of test agonists, or to design structures that are expected to exhibit binding with CD11b/CD18 for testing *in vivo* or *in vitro* using assays as described herein.

The computer-based modeling algorithms can also be used to identify novel agonists that bind β2 integrins, especially CD11b/CD18, using structural features of the chemical compound agonists of this invention. Scaffold hopping, atom replacement, residue replacement and/or molecule replacement methods can be used. The information can be analyzed in conjunction with information about the structure or conformation of CD11b/CD18 or a binding pocket thereof, such as structural information obtained by analysis of CD11b/CD18 using analytical techniques such as x-ray crystallography or nuclear magnetic resonance, to analyze interactions between binding agonists and the binding pocket they interact with. Such analysis can be used to predict the portion of CD11b/CD18 that interacts with the agonist, to select agonists that possess structural features correlated with desired binding activity from a library of test agonists, or to design structures that are expected to exhibit binding with CD11b/CD18 for testing *in vivo* or *in vitro* using assays as described herein.

A method of detecting or diagnosing a condition or disease in a patient is provided, by administering a β2 integrin agonist as described herein, detecting binding of the β2 integrin agonist to a β2 integrin, and confirming the presence of the disease. Preferably, the β2 integrin is CD11b/CD18. In other words, if binding is present, the patient has a disease as described above. For example, the disease can be an inflammatory disease or autoimmune disease, and by detecting the binding of the agonist to CD11b/CD18, it can be confirmed that a patient has those diseases. Also an agonist of the present invention can be administered to biological samples obtained from a patient in order to detect or diagnose a condition or a disease in a patient. The administered agonist can be derivatized, tagged, polymerized, encapsulated or embedded in such a way that it allows easy detection. The agonist can be tagged with a tracer, a radio-label or a fluorescent tag using a linker. The agonist can be detected using Magnetic Resonance Imaging (MRI) and other such diagnostic techniques as known in the art. Another method of detection can be as follows. A biological sample can be taken from a patient, such as blood or plasma, and an assay can be performed, such as to detect the binding of the β2 integrin agonist to the β2 integrin or measuring other markers (for example, IL-6 levels) in the sample.

The present invention also provides for a method of improving the general wellness of a patient by administering an effective amount of a β2 integrin agonist, and activating β2 integrins. In other words, by administering the agonists of the present invention, a patient's health and wellness improves because the agonists treat many different diseases as described above.

The compositions containing the agonists of the invention can be prepared by known methods for the preparation of pharmaceutically acceptable compositions which can be administered to subjects, such that an effective quantity of the active substance is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985). On this basis, the compositions include, albeit not exclusively, solutions of the substances in association with one or more pharmaceutically acceptable vehicles or diluents, and contained in buffered solutions with a suitable pH and iso-osmotic with the physiological fluids.

The agonists of this invention can be used in the form of the free base, in the form of salts, solvates and as hydrates. All forms are within the scope of the invention. Acid addition salts can be formed and provide a more convenient form for use; in practice, use of the salt form inherently amounts to use of the base form. The acids which can be used to prepare the acid addition salts include preferably those which produce, when combined with the free base, pharmaceutically acceptable salts, that is, salts whose anions are non toxic to the animal organism in pharmaceutical doses of the salts, so that the beneficial properties inherent in the free base are not vitiated by side effects ascribable to the anions. Although pharmaceutically acceptable salts of the basic agonists are preferred, all acid addition salts are useful as sources of the free base form even if the particular salt per se is desired only as an intermediate product as, for example, when the salt is formed only for the purposes of purification and identification, or when it is used as an intermediate in preparing a pharmaceutically acceptable salt by ion exchange procedures.

Pharmaceutically acceptable salts within the scope of the invention include those derived from the following acids; mineral acids such as hydrochloric acid, sulfuric acid, phosphoric acid and sulfamic acid; and organic acids such as acetic acid, citric acid, lactic acid, tartaric acid, malonic acid, methanesulfonic acid, ethanesulfonic acid, benzenesulfonic acid, p toluenesulfonic acid, cyclohexylsulfamic acid, quinic acid, and the like.

In accordance with the methods of the invention, the described agonists or salts or solvates thereof can be administered to a patient in a variety of forms depending on the selected route of administration, as will be understood by those skilled in the art. The compositions of the invention can be administered orally or parenterally. Parenteral administration includes intravenous, intraperitoneal, subcutaneous, intramuscular, transepithelial, nasal, intrapulmonary, intrathecal, rectal and topical modes of administration. Parenteral administration can be by continuous infusion over a selected period of time.

A agonist of the invention or a salt or solvate thereof can be orally administered, for example, with an inert diluent or with an assimilable edible carder, or it can be enclosed in hard or soft shell gelatin capsules, or it can be compressed into tablets, or it can be incorporated directly with the food of the diet. For oral therapeutic administration, the agonist of the invention can be incorporated with excipient and used in the form of ingestible tablets, buccal tablets, troches, capsules, elixirs, suspensions, syrups, wafers, and the like.

A agonist of the invention can also be administered parenterally or intraperitoneally. Solutions of a agonist of the invention as a free base or pharmacologically acceptable salt or solvate can be prepared in water suitably mixed with a surfactant such as hydroxypropylcellulose. Dispersions can also be prepared in glycerol, liquid polyethylene glycols, DMSO, and mixtures thereof with or without alcohol, and in oils. Under ordinary conditions of storage and use, these preparations contain a preservative to prevent the growth of microorganisms. A person skilled in the art would know how to prepare suitable formulations. Conventional procedures and ingredients for the selection and preparation of suitable formulations are described, for example, in Remington's Pharmaceutical Sciences (1990 - 18th edition) and in The United States Pharmacopeia: The National Formulary (USP 24 NF19) published in 1999.

The pharmaceutical forms suitable for injectable use include sterile aqueous solutions or dispersion and sterile powders for the extemporaneous preparation of sterile injectable solutions or dispersions. In all cases the form must be sterile and must be fluid to the extent that easy syringeability exists.

The agonists of the invention can be administered to an animal alone or in combination with pharmaceutically acceptable carriers, as noted above, the proportion of which is determined by the solubility and chemical nature of the agonist, chosen route of administration and standard pharmaceutical practice.

The dosage of the agonists and/or compositions of the invention can vary depending on many factors such as the pharmacodynamic properties of the agonist, the mode of administration, the age, health and weight of the recipient, the nature and extent of the symptoms, the frequency of the treatment and the type of concurrent treatment, if any, and the clearance rate of the agonist in the animal to be treated. One of skill in the art can determine the appropriate dosage based on the above factors. The agonists of the invention can be administered initially in a suitable dosage that can be adjusted as required, depending on the clinical response.

The invention is further described in detail by reference to the following experimental examples. These examples are provided for the purpose of illustration only, and are not intended to be limiting unless otherwise specified. Thus, the invention should in no way be construed as being limited to the following examples, but rather, should be construed to encompass any and all variations which become evident as a result of the teaching provided herein.

### EXAMPLE 1

### Materials and Methods of Synthesis

Agonists of the present invention can be readily synthesized using techniques known to those skilled in the art, such described, for example, in Advanced Organic Chemistry. March, 4th Ed., John Wiley and Sons, New York, NY, 1992; Advanced Organic Chemistry, Carey and Sundberg, Vol. A and B, 3rd Ed., Plenum Press, Inc., New York, NY, 1990; Protective groups in Organic Synthesis, Green and Wuts, 2"d Ed., John Wiley and Sons, New York, NY, 1991; Comprehensive Organic Transformations, Larock, VCH Publishers, Inc. , New York, NY, 1988 and references cited therein. The starting materials for the agonists described in this invention can be prepared using standard synthetic transformations of chemical precursors that are readily available from commercial sources, such as, Aldrich Chemical Co. (Milwaukee, WI) ; Sigma Chemical Co. (St. Louis, MO); Lancaster Synthesis (Windham, N. H.); Ryan Scientific (Columbia, S. C.); Canbridge (Cornwall, UK); Matrix Scientific (Columbia, S. C.); Arcos, (Pittsburgh, PA) and Trans World Chemicals (Rockville, MD).

Reagents and antibodies. The anti-CD11b monoclonal antibody (mAb) 44a (IgG2a) [37] and the heterodimer-specific anti-CD18 mAb IB4 (IgG2a) [38, 39] were from ATCC. The mAb 24 (IgG1) [40] was from Abcam and the isotype control antibodies MOPC-21 (IgG1) and MOPC-173 (IgG2a), FITC-conjugated mAbs A85-1 (rat anti-mouse IgG1), R19-15 (rat anti-mouse IgG2a) and FITC-conjugated goat anti-mouse immunoglobulin were from BD Pharmingen (San Diego, CA). Rat anti-mouse GR1-FITC and Mac-1-PE were from BD Pharmingen (San Diego, CA). Human Fibrinogen (Plasminogen, vonWillebrand Factor and Fibronectin depleted) was from EnzymeResearch Laboratories (SouthBend, IN), bovine serum albumin (BSA) was from Sigma (St. Louis, MI), recombinant human ICAM1-Fc was from R&D Systems (Minneapolis, MN) and iC3b was from Calbiochem (San Diego, CA). 384-well plates were from commercial sources (MaxiSorp from Nalgene (Rochester, NY) and Highbind from Corning (Corning, NY)). Non-fat milk was obtained from BioRad (Hercules, CA). Cell quantitation reagent MTS was from Promega (Madison, WI) and ATPLite was from PerkinElmer (Boston, MA). PCR reagents, and restriction and modification enzymes were obtained from New England Biolabs Inc. (Beverly, MA). Glutathione-beads were purchased from Sigma (St. Louis, MI). All cell culture reagents were from Invitrogen Corp. (San Diego, CA) and Mediatech (Manassas, VA). Fetal bovine serum was purchased from Atlanta Biologicals, Inc (Lawrenceville, GA). G418 antibiotic was purchased from Invivogen (San Diego, CA).

Mice. The C57BL/6J (B6) wild type and the B6 CD11b-/-(Jax 3991) [41] mice were purchased from The Jackson Laboratory (Bar Harbour, ME). The wild type Fischer 344 rats were purchased from Harlan Laboratories (Indianapolis, IN). Animal care and procedures were approved by the University of Miami Institutional Animal Care and Use Committee (IACUC) and were performed in accordance with the institutional guidelines.

Cell lines. K562 cells (ATCC) stably transfected with wild-type integrin CD11b/CD18 (K562 CD11b/CD18) have been described previously [42, 43]. Mutant CD11bE320A has been described previously [44]. K562 cells stably transfected with mutant integrin CD11bE320A/CD18 (K562 E320A) were generated according to literature protocols [42, 431. All cell lines were maintained in Iscove's Modified Dulbecco's Medium (IMDM) supplemented with 10% heat-inactivated fetal bovine serum, 50 IU/ml penicillin and streptomycin and 0,5 mg/ml G418.

K562 Cell Adhesion Assay. Cell adhesion assays with immobilized ligands were performed as previously described [42]. Assays with all different K562 cell lines (K562, K562 CD11b/CD18 and K562 E320A) were performed in an identical fashion. Briefly, 384-well Highbind microtiter plates were coated with a 30µL solution of ligand in Phosphate Buffered Saline, pH 7.4 containing 1mM each of Ca2+ and Mg2+ ions (PBS++) overnight at 4°C. Ligand Fg was coated at a concentration of 5-15mg/mL and iC3b at 1-5mg/mL. Heterodimer specific mAb IB4 (ascites) was coated at a 1:100 dilution. Subsequently, the non-specific sites in the wells were blocked by incubation with 1% non-fat milk in Tris Buffered Saline (TBS), pH 7.4, at room temperature for 1 hour, except for the neutrophil assay, where the wells were blocked with TBS containing 1% gelatin. Next, the wells were washed three times with TBS. K562 cells were suspended in the assay buffer (TBS containing 1mM each of Ca2+ and Mg2+ ions (TBS++)) and were transferred to the ligand-coated wells (30,000 cells/well). Stock solution of the leukadherin family of small molecule agonists was prepared by dissolving the agonists in DMSO at a concentration of 2-10mM, Final concentration of DMSO in the assay was approximately 1%. K562 cells were incubated in the presence of increasing concentration of leukadherins for 30 minutes at 37 °C. To dislodge non-adherent cells, the assay plates were gently inverted and kept in the inverted position for 30 minutes at room temperature. Cells remaining adherent were fixed using formaldehyde and were quantitated using imaging microscopy, as previously described [42]. For the blocking assays, cells were incubated with mAbs 44a and IB4 for 30 minutes at RT prior to adding them to the assay wells. Assays were performed in 3-6 replicate wells. Data reported is from one of at least three independent experiments. The high throughput screening (HTS) assay to identify novel agonists using a library of >100,000 small molecules was performed as previously described [42, 45].

Neutrophil adhesion assay. Neutrophils from 8- to 10-week old WT and CD11b-/- B6 mice were isolated from thioglycollate-stimulated peritonea according to literature protocols [46]. Cells were suspended in serum free medium (IMDM) and incubated with leukadherins in the ligand-coated wells for 10 minutes at 37 °C. Next, the assay plates were gently inverted and kept in the inverted position for 30 minutes at room temperature to dislodge the non-adherent cells. Cells remaining adherent were quantitated using imaging microscopy, as previously described [42, 47]. Assays were performed in triplicate wells. Data reported is from one of at least three independent experiments.

Chemotaxis assay and time-lapse video microscopy. Neutrophil chemotaxis on 2D surfaces was performed using Zigmond chamber (Neuro Probe) as described [48, 49] on acid cleaned glass or Fg coated glass coverslips. Cell migration was allowed in a gradient of 10 mM bacterial peptide formyl-methionyl-leucyl-phenylalanine (fMLP, Sigma), in the absence or presence of leukadherins (15 mM). Cell migration was recorded at every 30 second interval for a period of 25 minutes using a Nikon Eclipse 90i inverted microscope. Images were acquired with a Nikon DS camera using a PLAN APO 20X differential interference contrast (DIC) microscopy objective and captured into Nikon Imaging software. Analysis of neutrophil migration was performed with the motile population that had moved more than 10 µm [48] using ImageJ software (NIH, USA) with manual cell tracking using the Ibidi chemotaxis and migration tool plugin for ImageJ. Migration velocity and the total displacement (distance from origin) were also analyzed. Quantitation was performed using at least 50 independent cells per condition from at least three independent experiments.

Immunofluorescence. To examine localization of integrin CD11b/CD18 and F-actin in migrating neutrophils, cells (104) were stimulated with 10µM fMLP in serum free medium (RPMI 1640) on glass cover slips for 15 min at 37°C, in the absence or presence of leukadherins (15 mM). The cells were fixed, permeabilized with 0.1% Triton X-100, and stained with anti-mouse CD11b antibody (clone M/170, BD Biosciences) followed by goat anti-rat Alexa488 (Invitrogen) and rhodamine-labeled phalloidin (Invitrogen). A z-series of fluorescence images were recorded with a Leica TCS SP5 confocal microscope and an HCX PL APO 63x/1.4 NA objective and using Leica LAS-AF software. The z-series were analyzed with the Leica LAS-AF software suite. The images presented are from a z-stack projection of 15 confocal sections from the basal to the apical cell side (stack z-spacing, 0.29 µm). Images presented are representative of at least 20 cells analyzed per condition from at least two independent experiments.

To examine clustering of CD11b/CD18 on cell surface, K562 CD11b/CD18 cells (104) were suspended in serum free medium (IMDM) and incubated without or with the ligand Fg (100mg) for 3 hours at 37°C as described [50], in the absence or presence of leukadherins (15 mM). The cells were fixed in suspension and stained with anti-CD11b/CD18 mAb IB4 followed by goat anti-mouse Alexa488 (SIGMA). Fluorescence images were recorded with a Leica DMI16000 deconvolution microscope and HCX PL APO 63x/1.3 NA objective and using Leica LAS-AF software, with DCF360FX camera driven by LAS-AF software. The CD1 1 b/CD1 8 clusters were analyzed in ImageJ and a 3-dimensional representation of fluorescence intensity was also generated in ImageJ. The images presented are representative of at least 20 cells analyzed per condition from at least 3 independent experiments.

Purification of recombinant CD11b A-domain (αA-domain). Recombinant human αA-domains were constructed and purified according to published protocols [51], Briefly, the αA-domain in its inactive conformation was generated by cloning and expressing protein fragments spanning residues Gly111-Gly321 (321WT) using forward primer 5'-ggttccgcgtggatccgagaacctgtactttcaaggaggatccaacctacggcag-3' (SEQ ID NO: 1) and reverse primer 5'-gaattcccggggatccaccctcgatcgcaaagat-3' (SEQ ID NO: 2) and using the Infusion Cloning Kit (Clontech, Mountain View, CA) into the BamHI site in vector pGEX-2T according to manufacturer's protocol. The - αA-domain in its active conformation was generated by replacing Ile316 with Gly (I316G [52]) using forward primer 5'-ggttccgcgtggatccgagaacclgtactttcaaggaggtttlcaggaatgt-3' (SEQ ID NO: 3) and reverse primer 5'-atatccccgggattaaccctcgatcgcaaagcccttctc-3' (SEQ ID NO: 4), The insert was digested with BamHI and SmaI and ligated into pGEX-2T vector also digested with BamHI and SmaI. All constructs were confirmed by direct DNA sequencing. All recombinant proteins were expressed as glutathione S-transferase (GST) fusion proteins in Escherichia coli and purified by affinity chromatography (Glutathione-beads, Sigma) following manufacturer's instructions. Purified protein preparations were dialyzed against 20 mm Tris-HCl, pH 7.5, 150 mm NaCl (Tris-buffered saline) and subsequently concentrated using Amicon-10 columns (Millipore) and stored at -80ºC, Purity was confirmed by 1D SDS-polyacrylamide gel electrophoresis analysis.

αA-domain ligand-binding assay. Maxisorp 96-well plates were coaled with Fg (1 µg/well) in 10 mM PBS, pH 7.4 overnight and blocked with 1% bovine serum albumin in PBS. Binding of purified, GST-tagged αA-domain (50mL/well of 5mg/mL solution) to immobilized Fg was performed in the TBS assay buffer (TBS containing 0.1% BSA, 1 mM Mg2+, 1 mM Ca2+, and 0.05% Tween 20) for 1 hour at room temperature. Unbound αA-domain was removed by washing the assay wells twice with TBS++. Subsequently, the amound of bound protein was determined by incubating with anti-GST antibody conjugated to horseradish peroxidase (GE, Piscataway, NJ) (1:2000 dilution) for 1 hr. Unbound anti-GST-HRP was removed by washing the assay wells twice with TBS₊₊. Detection of bound protein was done using TMB substrate kit (Vector Labs, Burlingame, CA) according to manufacturer's protocol. Absorbance was read using Spectromax M5 spectrophotometer (Molecular Devices, Sunnyvale, CA). Assays were performed in triplicate wells and the data shown is from one of at least three independent experiments.

Flow Cytometry. Flow cytometric analysis of K562 cells for integrin CD11b/CD18 cell surface expression was performed using published protocols [53, 54]. Briefly, cells were suspended in the assay buffer (TBS containing 1mM each of Ca2+ and Mg2+ ions (TBS++) and 0.1% BSA). Cells (5 X 105) were incubated with primary mAb (1:100 dilution of IB4 or 44a ascites) in the absence or presence of 15 mM leukadherin in 100ml TBS++ at 37°C for 30 minutes. Subsequently, the cells were washed three times with the assay buffer and incubated with goat anti-mouse-APC (1mg/ml, Invitrogen) for 20 minutes at 4°C. Cells were washed twice with the assay buffer and analyzed using FACSCaliber flow cytometer (BD Biosciences, CA), counting at least 10,000 events. Data was analyzed using the CellQuest software (BD Biosciences). Assays were performed in triplicate and the data shown is from one of at least three independent experiments.

Cell viability assay. Cell viability assay was performed as described. Briefly, K562 CD11b/CD18 cells (10,000/well) were incubated in 96 well plates (Corning, Corning, NY) with increasing amounts of indicated agonist for 24 hours. The number of viable cells after 24 hours was determined by using MTS reagent according to manufacturers protocol (Promega, Madison, WI) and using Spectramax M5 spectrophotometer (Molecular Devices) for reading of the assay plates. Data presented is representative of at least two independent experiments.

Western Blot. K562 CD11b/GD18 cells were incubated with LA1, LA2, LA3 (15 mM) or FG (200 mg) in serum free media containing for 1 hour at 37°C, Cell lysates were run on a 10% SDS-PAGE gel and transferred to PVDF membrane (ThermoScientific, Waltham, MA) using established protocols. Membranes were probed with 1:1000 dilution of anti-phospho ERK1/2 antibody (Thr202/Tyr204, Cell Signaling, Danvers, MA), stripped with Reblot mild stripping solution (Millipore, Billerica, MA), and reprobed first with anti-ERK1/2 antibody (Cell Signal) and next with anti-GAPDH antibody (Cell Signaling) and developed according to manufacturer's protocols (ThermoScientific, Waltham, MA). Data presented is representative of at least three independent experiments.

Blood cell count. Complete peripheral blood leukocyte counts from the different mice were quantified at the mouse pathology core using standard assays.

*In vivo* peritonitis model. Thioglycollate-induced peritonitis was performed as previously described [46], using 8-10 week old WT B6 and CD11b-/- B6 mice. Leukadherin agonists were administered thirty min prior to intra peritoneal (i.p.) thioglycollate (3%) injection. LA1 and LA2 (200mL of 20mM solution in saline) were administered intra venously (i.v.), LA3 was administered i.p. (1 mL of 20 mM solution in saline). To evaluate peritoneal neutrophil recruitment, mice were euthanized at 4 hours following thioglycollate injection, the peritoneal lavage was collected and the number of emigrated neutrophils was quantified using double positive cells for GR-1 and Mac-1 staining as described [55].

Balloon induced arterial injury in rats. All surgeries were under isoflurane anaesthesia (Baxter, IL, USA). Balloon injury in the right iliac artery was inflicted with a 2F Fogarty catheter (Baxter Corp., Irvine, CA, USA) adapted to a custom angiographic kit (Boston Scientific, Scimed) [56], An aortotomy in the abdominal aorta was made to insert a 2F Fogarty embolectomy catheter to the level of the right iliac artery. The balloon was inflated to 1.5-1.6 atmospheres and retracted to the arteriotomy site three times. The aortic excision was repaired with 8.0 sutures. The abdominal cavity was closed by planes using interrupted suture pattern. Arterial specimens were collected 3-30 days after injury and fixed in 4% formalin-PBS (Sigma-Aldrich, St. Louis, MO) for 5 minutes and analyzed by histology and immunostaining.

Histology and immunostaining. Elastica van Gieson staining was used for histochemical analyses to evaluate neointima formation, Morphometric analyses was performed in a blinded fashion using NIH ImageJ. Immunostaining with anti-rat CD68 (1:50, AbD Serotec) for the detection of macrophages in the tissue.

Zebrafish tailfin injury assays. Transgenic Tg(mpx::eGFP) [57] were maintained according to standard protocols [58]. Tailfin injury in three days post fertilization (dpf) larvae was performed as described [57]. Larvae were anesthetized by immersion in E3 with 4.2% tricane, and tails were completely transected with a sterile microdissection scalpel in accordance with the approved protocols and were recovered for the indicated time points. Zebrafish larvae (3dpf) were treated with agonists as described (Ref). Briefly, small molecule agonists were administered by immersing the larvae in a solution of the agonists in E3. The final concentration of DMSO was kept at <1%. For the assessment of the inflammatory response, injured larvae were analyzed at 4 hours post-injury. For the post-wash assay, uninjured larvae incubated with the agonists in E3 for 4-8 hours, washed into E3 and injured. Larvae were analyzed using a Leica DMI6000B microscope and an Hamamatsu Orca-3CCD camera using Volocity. Excitation was performed using the laser at 488nm and the images were analyzed using Volocity. The number of fluorescent neutrophils at the site of inflammation were counted by eye in a blinded fashion.

Statistical analysis. Data were compared using one-way ANOVA with posthoc analysis, when comparing two or more groups, p values < 0.05 were considered significant.

Computational Modeling. To model binding of leukadherins to the active, open conformation of the αA-domain, a series of computational studies were performed as follows. First, a model was generated of the αA-domain in the open (active) conformation. The high-resolution three-dimensional structure of αA in both its closed (inactive) and open (active, ligand-competent) conformations is available from PDB [59, 60]. However, the α7 helix in αA (that creates part of a hydrophobic pocket known as Socket for Isoleucine (SILEN) in CD11b [60] or IDAS in CD11a [61] and that shows the highest conformational change upon αA-activation [62-64]) is shorter by three residues in the three-dimensional structure of the open form [60] as compared to that of the closed form of αA [59]. Because an αA agonist is predicted to bind in this region 11, the α7 helix was manually extended in the high resolution structure of αA-domain [60] by three additional residues from the domain of the closed (inactive) form of αA [59]. The model was refined by hydrogen bond optimization and constrained minimization.

To identify possible ligand binding modes, an induced fit docking (IFD) procedure implemented in the Schrodinger software suite was applied as previously described [36]. The αA poses were ranked using MM GB/SA; this approach was used to estimate the free energy of binding for agonists to αA using the Generalized Born solvation model augmented with the hydrophobic solvent accessible surface area term (GBSA). The optimized αA structure was then used for agonist re-docking using a standard potential [65]. Several IFD runs were performed and they resulted in high-scoring poses for the most agonists [36]. The optimized receptor structures after IFD were then used to dock the novel agonists using Schrodinger Glide and the SP scoring function. Next, the best scoring poses for agonists in their Z configuration were further optimized in molecular dynamics simulations. Molecular dynamics simulations are performed as multi-step protocols with several minimization and simulation steps preceding the production molecular dynamics run. Simulations were performed with the molecular dynamics package Desmond by DEShaw Research [66] at 300K and 325K (NPT ensemble) using the SPC water model (cubic box of 10 Å around the receptor) on IBM E-server 1350 cluster (36 nodes of 8 Xeon 2.3 GHZ cores and 12 GB of memory). The final simulations times were 12 ns in which the reported poses remained stable. FIGURES 12A-12E show poses of LA1, LA2 and LA3.

### Results

Integrin agonists have several advantages over antagonists. Research with antagonists over last several years has shown them to be suboptimal. First, it has been showed that suppressing leukocyte recruitment with antagonists requires occupancy of >90% of active integrin receptors [32], usually requiring high levels of blocking antibodies *in vivo.* Second, complete blockade of cell surface-expressed CD11b/CD18 even with antibodies is difficult due to availability of a large mobilizable intracellular pool of CD11b/CD18 [30, 31]. Third, several other antagonists, such as ligand- ' mimetic neutrophil inhibitory factor (NIF) [67] and recombinant αA-domain [68], were effective in animal models but their large size and immunogenicity preclude their use as a therapeutic agent. Recombinant NIF (UK-279276) failed in clinical trials. Likewise, peptides derived from either anti-CD11b/CD18 antibodies or CD11b/CD18 ligands are not very efficacious in blocking ligand binding *in vitro* [69], perhaps owing to their improper conformation in solution or to their small size relative to the ligand-binding region on CD11b/CD18. Finally, many antagonistic antibodies (such as rhuMAb CD18, anti-CD 18 LeukArrest (Hu23F2G) and anti-ICAM1 mAb Enlimomab (R6.5)) failed in treating inflammatory/autoimmune diseases in several clinical trials [28, 29] and β2 integrin blockers have also shown unexpected side effects and have had to be withdrawn from the market [33].

Assays for identification of CD11b/CD18 agonists: Currently; small molecules agonists of CD11b/CD18 are not available primarily because: a) current screening assays, which rely on purified CD11b/CD18 adsorbed to microtiter plates, are not tenable for a high throughput screening (HTS) campaign because it is difficult to obtain the requisite amounts of CD11b/CD18 from mammalian cells and because a large fraction of the adsorbed protein does not retain its natural conformation upon adsorption to the plastic surfaces, and b) optimized cell-based assays for CD11b/CD18 are currently lacking in literature, because such assays are difficult to automate due to the low binding affinity of cell-surface expressed CD11b/CD18 to its physiologic ligands. Applicant recently described a novel cell-adhesion based HTS assay for screening libraries of small molecules against CD11b/CD18 [42]. The assay uses immobilized fibrinogen (Fg) as ligand for CD11b/CD18 stably expressed on mammalian K562 cells. The main issue that was encountered in adapting this concept for use in HTS was the fact that no matter how gently the wells of the plates were washed to remove non-adherent cells, the automated washing step caused substantial and uneven detachment of adherent cells from wells. This resulted in huge assay variability. Surprisingly, it was found that simple inversion of the assay plates to gently remove non-adherent cells by gravity, rather than with an automated plate-washer, eliminated the variability and made a robust and reproducible screening assay. Subsequently, automated imaging of DAPI-stained cell nuclei was used to quantitate the adherent cells. The newly developed 384-well plate based assay is fast, inexpensive, consistently produces acceptable Z'-values (>0.5) for HTS and is easy to implement in an HTS environment.

Discovery of novel CD11b/CD18 agonists - leukadherins. An in-house developed cell-based high throughput screening (HTS) assay was used [42] to screen a chemical library of >100,000 molecules for agonists against K562 CD11b/CD18 cells. As a unique strategy, agonists that increase cell adhesion (agonists) were focused on, rather than those that inhibit it. A series of agonists were identified containing a core furanyl thiazolidinone motif that increased adhesion (agonists) of K562 CD11b/CD18 to its physiologic ligand fibrinogen (Fg) [42, 45]. K562 CD11b/CD18 cells showed virtually no binding to immobilized Fibrinogen (Fg) when incubated in the assay buffer (1mM each of physiologic ions Ca2+ and Mg2+ in Tris buffered saline (TBS++)) alone. Surprisingly, it was found that a large subset of the hits contained a central five-membered 2,4-di-oxo-thiazolidine [42] and 2,4-di-oxo-thiazolidine motif containing agonists as hits [45]. Targeting of the αA-domain by the 2,4-di-oxo-thiazolidine motif containing agonists was confirmed using binding assays with purified recombinant αA-domain, where these agonists increased binding of αA-domain to immobilized Fg [70]. Additionally, binding was selective as cells not expressing CD11b/CD18 did not show any appreciable binding and the binding of CD11b/CD18 expressing cells could be blocked with known blocking monoclonal antibodies (mAbs) 44a [37] (anti-CD11b) and IB4 [38, 39] (anti-CD18).

The structure-activity relationship (SAR) of various substitutions on the central core was explored [36] and several agonists were identified, that have been termed leukadherins. This includes leukadherin-1 (LA1), leukadherin-2 (LA2) and leukadherin-3 (LA3) that increased CD11b/CD18 dependent cell adhesion to Fg with EC50 (effective concentration for 50% increase in adhesion) values of 4µM, 12µM and 14µM respectively (FIGURES 1A-1D). Several other agonists provided similar level of activity. A structurally related compound was also identified, leukadherin-control (LA-C), which showed no affect on CD11b/CD18 dependent cell adhesion (FIGURES 1A and 1E). Cells not expressing CD11b/CD18 did not show any significant binding (Figures 1B-1E). Unlike a recently described inverse agonist of LFA-1 that increased LFA-1-mediated adhesion under basal conditions but inhibited it under activating conditions (Yang, W., C.V. Carman, M. Kim, A. Salas, M. Shimaoka, and T.A. Springer. 2006. A small molecule agonist of an integrin, alpha L beta 2. J Biol Chem), LA1-3 did not inhibit cell adhesion in the presence of agonist Mn²⁺ (FIGURES 7A-7C), showing that they are true agonists. Increased adhesion of CD11b/CD18 expressing cells, induced by known agonist Mn²⁺ and LA1-3, was blocked by anti-CD11b/CD18 monoclonal antibodies (mAbs) IB4 and 44a (FIGURE 1F), further confirming that these compounds mediate CD11b/CD18-dependent cell adhesion. Neutrophils contain a large, mobilizable intracellular pool of CD11b/CD18 which, in addition to a conformational switch of CD11b/CD18 from an inactive to an active form, helps increase neutrophils adhere to the extracellular matrix. To rule out upregulation of CD11b/CD18 surface expression as a reason for increased cell adhesion by LA1-3, its surface expression on K562 CD11b/CD18 cells was measured (FIGURE 5) and neutrophils (FIGURE 6) and no increase by LA1-3 was found. The increase in CD11b/CD18-dependent cell adhesion by LA1-3 was independent of the type of ligand, as they also increased cell adhesion to CD11b/CD18 ligands iC3b (FIGURES 8A-C) and ICAM-1 (FIGURE 9). Human monocytic THP-1 cells also showed a similar leukadherin-induced increase in cell adhesion, showing that the effects of leukadherins are independent of cell-type (FIGURES 10A-10C). LA1-3 also increased binding of wild-type (WT) neutrophils to immobilized Fg, but not the CD11b^{-/-} neutrophils (3) (FIGURE 1G), further demonstrating that these compounds target CD11b/CD18. To determine whether leukadherins also affect CD11b/CD18-mediated phagocytosis, K562 CD11b/CD18 cells were incubated with iC3b-opsonized RBCs (EiC3bs). It was found that LA1-3 significantly increased the capture and rosetting of EiC3bs, showing that these agonists can also upregulate CD11b/CD18-mediated phagocytosis function (FIGURE 11). This allows, for the first time, the testing of whether CD11b/CD18 activation is anti-inflammatory and whether small molecule agonists of integrins can activate integrins *in vivo* and lead to outcomes as anticipated by the animals knocked-in for activating mutants of other integrins.

Also, *in silico* examination of a number of analogs was performed. *In silico* docking studies using high-resolution three-dimensional structures of the αA-domain in its low-affinity and its high-affinity conformations [59, 60, 62] showed that the LA1-3 preferentially bind to the open, high-affinity conformation of αA-domain, near the activation-sensitive α7-helix region, allosterically stabilizing the αA-domain in its high affinity conformation [36] (FIGURES 12A-12E).

For certain agonists of Formula (II), it was found that substitutions at the C-5 position of the furan ring (R3 substituents) had the largest effect on agonist potency (such as, compounds 1-30) [36]. Non-aromatic or nonconjugated substituents that disrupted the pi-conjugation with the planar furanyl ring were strongly disfavored. In certain orientations, planar aromatic rings were preferred and a non-substituted phenyl ring was also preferred over aliphatic groups at ortho- or the para- positions of the phenyl ring. For the substituents at the N-3 position of the thaizolidine ring, shortening the length of the substituted ester (from ethyl to methyl), and shortening the aliphatic chain length was highly disfavored. Similarly, substitution of the aliphatic chain with a phenyl ring was disfavored. Long-chain, bulky residues were also disfavored at the N-3 position. However, a compound containing methylene substituted small aromatic ring bound to a level similar to LA3. Conversely, a co-substitution of benzyl at the N-3 position with a highly electron-withdrawing and bulky para-substituted aromatic at R3 was highly disfavored. Certain compounds also showed selective binding to the purified recombinant αA-domain by increasing binding to immobilized Fg [70] as well as a high selectivity for integrin CD11b/CD18 over CD11a/CD18.

To further evaluate the compounds, various physicochemical descriptors were calculated using Schrodinger QikProp program. The most favored compounds in that series (compounds 1-14) have good predicted Caco-2 cell permeability and human oral absorption. Among them LA1-3 have a slightly better clogP and better predicted solubility and among the highest ligand efficiency (BEI = 14) [71],

Next, in order to gain insights into potential binding pockets for this subset of small molecules in the αA-domain, *in*-*silico* docking experiments were conducted. The high-resolution three-dimensional structure of CD11b A-domain in both its closed (inactive) and open (active, ligand-competent) conformations is available from PDB [59, 60, 62]. However, the α7 helix in αA (that creates part of a hydrophobic pocket known as Socket for Isoleucine (SILEN) in CD11b [60] or IDAS in CD11a [61] and that shows the highest conformational change upon αA-activation [62-64]) is shorter by three residues in the three-dimensional structures of the open form [59, 60] as compared to that of the closed form of αA [59, 62]. As the newly discovered agonists are predicted to bind in this region and stabilize this conformation of αA, a model of the open (active, ligand-competent) conformation of the CD11b A-domain was constructed by manually extending the α7 helix in the high resolution structure of CD11b A-domain [59, 60] by three additional residues from the structure of the closed form followed by hydrogen bond optimization and constrained (Impref) minimization as implemented in the Maestro protein preparation facility (Schrodinger Inc, Portland).

Conformational repositioning of the α7 helix upon activation, which appears to be stabilized upon agonist binding shows that the agonists bind in the region between helix α7 and α1 and the central β sheet. [11, 62] This has also been showed by a previous report. [11] Therefore, the above optimized structure of the αA-domain was utilized in the open conformation to initiate compound docking. In the apo structure, this activation sensitive α7 helix region is spatially crowded by many hydrophobic residues lining the pocket. An induced fit docking procedure implemented in the Schrodinger software suite was applied in which initial docking with a softened potential to generate an ensemble of possible poses is followed by receptor optimization and ligand re-docking [65]. This protocol resulted in a high scoring pose of LA1-3 (Z configuration) in which the carbonyl oxygens of the 2,4-di-oxo-thiazolidine core and its analogs are fixed by Ser133 and Thr169 and, for example, the hydrophobic 2,4-dichlorophenyl moiety of LA3 is interacting in the hydrophobic pocket. In a stable 6 ns all-atom explicit solvent molecular dynamics simulation at increased temperature (using Desmond by DEShaw Research)[66] the α7 helix adjusts only very slightly. The induced-fit docking receptor was used to dock additional structures using Schrodinger Glide Program. [72] The obtained poses were then rescored using MM-GB/SA methodology [73] allowing receptor flexibility to obtain more accurate estimates of relative binding free energies. The resulting binding hypothesis of the best compounds are shown in FIGURES 12A-12E and 24. As was expected, the hydrophobic phenyl furanyl moiety (the C5-substituent on the thiazolidine ring) is puried in a hydrophobic pocket lined by residues L312, I308, L305 (α7 helix), L164, V160, F156 (α1 helix), and Y267, I269, I236, V238, I236, I135 (central beta sheet). This structural model also explains why compound LA-C (structurally related to LA1-3) is inactive, as in this binding mode the αC carbon of the ethylcarboxylate moiety at N-3 position of the central thiazolidine ring of LA-C is in close proximity to Ser133, Thr169, and Asp132 (less than 2.5 Å), which creates a tight fit and does not tolerate the larger methyl group at aC that is present in some compounds but is absent in LA1-3. In general, it was found that, for the most comparable compounds, the lower activity of the sterically more demanding compound can (at least partially) be attributed to increased receptor and/or ligand strain,

The SAR and the binding hypothesis show that one hydrophobic interaction is critical. Compounds with two polar ends are generally found to be inactive. The interaction in the hydrophobic pocket appears quite sensitive to sterical demand and the overall size of the molecule. For example, in case of the smaller ethyl acetate N-3 substituent, larger as well as smaller phenyl furanyl substituents are tolerated (although the smallest is the most active) while for structures with larger N-3 substituents only the unsubstituted phenyl furanyl is active. Thus, the *in silico* docking studies show a reasonable hypothesis for the binding of these novel allosteric agonists of integrin CD11b/CD18. Additionally, during the various induced fit docking studies other poses were obtained. For example, one model showed the compounds "flipped" along its long, vertical axis. However, in all cases the hydrophobic moiety interacts in the same region described and illustrated in FIGURES 12A-12E.

Next, the selectivity was determined of compounds for integrin CD11b/CD18 over highly homologous integrin CD11a/CD18 (also known as LFA-1). K562 cells stably transfected with wild type integrin CD11a/CD18 (K562 CD11a/CD18) were generated. Next, the ability of LA3 to increase cell adhesion to immobilized ICAM-1 was measured, a physiologic ligand of integrin CD11a/CD18. It showed two-fold higher selectivity for integrin CD11b/CD18 over CD11a/CD18, with EC50 values of 13.6±5µM with K562 CD11b/CD18 cells. This is in contrast with the previously described compounds,[35] which showed equal binding to both integrins in the assays.

To identify binding site of LA1-3, which is predicted to bind the ligand-binding αA (or αl) domain in CD11b/CD18 [35, 36, 42], K562 cells stably expressing mutant integrin CD11bE320A/CD18 (K562 E320A) were generated. The highly conserved residue E320 in the linker following the activation-sensitive α7-helix in the CD11b A-domain (αA-domain) acts as an endogenous ligand of the CD18 vWFA-domain (βA- or I-domain) [44]. The E320A mutation abolishes agonist Mn2+-ion mediated increase in ligand-binding by CD11b/CD18. However, stabilization of αA in a high-affinity conformation by additional activating mutations overcomes this deficit and induces ligand binding in the E320A mutant [63]. LA1, LA2 and LA3 (but not Mn2+) selectively increased binding of K562 E320A to Fg (FIGURE 1H), showing that these compounds also bind to and stabilize the aA-domain in a high-affinity conformation. To confirm, purified recombinant αA [70] was used, as expected [35, 42], it was found that LA1 and LA2 increased binding of the WT αA to the immobilized ligand (FIGURE 1I) to a level of binding observed with a mutant αA-domain containing a constitutionally activating mutation (I316G) [52]. This shows that leukadherin binding stabilizes αA in its open, high-affinity conformation.

Flow cytometry analysis showed an increase in the binding of activation sensitive mAb24 to K562 CD11b/CD18 cells in the presence of LA1, confirming that LA1 activates full-length integrin expressed on live cells (FIGURE 13). Björklund *et al.* described a CD11b/CD18 agonist (IMB-10) that also targets the CD11b/CD18 aA. We compared the relative affinities of LA1 and IMB-10 for CD11b/CD18 using our cell-based adhesion assay and found that LA1 showed higher affinity (FIGURE 14), perhaps owing to its more rotationally constrained furanyl-thiazolidinone central scaffold.

Leukocyte chemotaxis on 2D surfaces involves integrin-mediated sequential adhesion and de-adhesion steps [74]. Cells expressing constitutively active integrin mutants show increased adhesion and dramatically reduced cell migration in chemotactic gradients, by freezing integrins in a ligand-bound state [75, 76]. To test if increased cell-adhesion by leukaderins affects cell migration, murine neutrophils chemotaxing in response to a gradient of chemokine peptide formyl-Met-Leu-Phe (fMLP) [49] were used. Live cell imaging showed smooth migration of neutrophils in physiologic buffer (FIGURE 2A). However, treatment with LA1, LA2 or LA3 lead to a significant decrease in the lateral migration and the migration velocity of these cells (FIGURES 2A-2C). Although the LA1-3 treated cells showed some movement towards the chemokine, they displayed reduced directional persistence (FIGURE 2D) and reduced mean squared displacement (MSD, FIGURE 2E), showing constrained motility, as compared to a more directed motility for control (DMSO) cells. Unlike the neutrophils chemotaxing in the absence of leukadherins, where they displayed a typical flattened leading edge and short narrow tail, cells migrating in the presence of LA1-3 showed elongated uropods, showing defects in cell de-adhesion as the key mechanism behind decreased cell migration, as has been seen with activating integrin mutations [55, 77]. To investigate, confocal microscopy was used that showed clustered CD11b/CD18 in the extended uropods of LA1-3 treated cells (FIGURE 2F), showing that the failure to release integrin-substrate interactions in the uropod was responsible for the defective migration. Leukadherin treatment showed no change in neutrophil migration in 3D collagen gels (FIGURES 15A-15E), supporting recent findings that leukocyte migration in 3D is integrin independent. Yet, leukadherins reduced the efficiency of trans-endothelial migration (TEM) by THP-1 cells across a TNFa-activated HUVEC layer *in vitro* by increasing cell adhesion to the HUVEC layer (FIGURES 2G-I). In all, these data show that leukadherins increase cell adhesivity and reduce their lateral motility, thereby affecting TEM.

Integrin activation and ligand binding leads to clustering of integrins on the cell surface and initiates outside-in signaling, including the activation of p38 mitogen activated protein kinase/extracellular signal-regulated kinase (MAPK/ERK1/2) pathways [17, 22], thereby mimicking the anchorage-dependent pro-survival signals in most cells [50]. As LA1-3 bind to and activate CD11b/CD18, it is conceivable that such binding alone can trigger integrin-mediated outside-in signaling, thus mimicking a ligand bound integrin state for the cell, which can have profound consequences on leukocyte lifetime and function. To test, confocal microscopy was used for imaging CD11b/CD18 clustering on cell surface [17]. Cells displayed no detectable CD11b/CD18 macro clustering in the absence of ligand (FIGURE 18A, DMSO), but showed high-degree of clustering upon addition of exogenous Fg (FIGURE 18B, DMSO). Similarly, treatment with LA1-3 exhibited integrin macro-clustering only upon addition of external Fg (FIGURES 18A-18B), showing that LA1-3 are not integrin ligand mimics. Additionally, since known CD11b/CD18 agonists Mn2₊ [78] and activating mAbs [79] and its ligands [17] induce ERK1/2 phosphorylation, ERK1/2 phosphorylation was examined in cells and it was found that LA1-3 treatment did not induce it (pERK, FIGURE 19) as opposed to incubation with ligand Fg (FIGURE 19) or phorbol ester PMA (not shown). Thus, it can be concluded that leukadherins do not mimic ligands and do not induce outside-in signaling in cells.

Surprisingly, LA1-3 treatment resulted in robust Akt phosphorylation, like with ligand Fg, as compared to treatment with DMSO alone (not shown). As pAkt is known to suppress inflammatory signal (e.g. LPS) dependent expression of pro-inflammatory cytokines, this shows that leukadherins can also suppress pro-inflammatory cytokine expression in leukocytes.

Leukadherins decrease secretion of soluble factors by neutrophils and macrophages. In an experiment to test the effect of leukadherin treatment on secretion of pro-inflammatory cytokines by leukocytes, WT mouse macrophages and neutrophils were stimulated with LPS in the absence or presence of two different concentrations of agonist LA1 and measured levels of pro-inflammatory cytokine in the cell supernatant. LPS treatment significantly increased cytokine secretion in both cell types (not shown), as compared to unstimulated cells, and that addition of LA1 significantly decreased it in the supernatant, showing that CD11b/CD18 activation with leukadherins can have anti-inflammatory effects,

To determine the effects of LA1-3 on inflammatory responses *in vivo*, their effects were monitored on neutrophil recruitment upon acute thioglycollate-induced peritonitis in mice [41]. LA1-3 showed no *in vitro* cytotoxicity to K562 cells (FIGURES 16A-16D) or to murine neutrophils at concentrations as high as 50µM (FIGURES 17A-17C). Intraperitoneal injection of thioglycollate resulted in significant peritoneal accumulation of neutrophils, as compared with saline alone (p<0.001) (FIGURE 3A). Administration of LA1 30 minutes prior to thioglycollate injection significantly reduced neutrophil accumulation (by 40%, p<0.05), LA2 reduced it by 65% (p<0.0001) and LA3 reduced it by 55% (p<0.05) as compared to administration of the vehicle alone. Determination of the leukocytes in circulation from mice treated with leukadherins showed no reduction in their cell number as compared to the vehicle treated animals (Table 1).

**TABLE 1**

| | Saline | LA1 | LA2 | LA3 |
|---|---|---|---|---|
| WBC count | 3.6 ± 1.1 x 10³/µL | 3.4 ± 0.7 x 10³/µL | 3.8 ± 1.3 x 10³/µL | 3.8 ± 0.7 x 10³/µL |
| p-value | | ns | ns | ns |

This shows that leukadherins do not cause leukocyte cytotoxicity *in vivo*, thus ruling it out as a reason for the observed reduction in marginated neutrophils in leukadherin treated animals. It was also found that LA1-3 administration did not significantly reduce the number of recruited neutrophils in the peritoneum of CD11b-/- mice (FIGURE 3B), which showed increased neutrophil accumulation compared to WT, as published before [41]. This further shows that LA1-3 selectively target integrin CD11b/CD18 *in vivo.*

In TGC-induced peritonitis, it was found that the number of peritoneal neutrophil increased after 4 hours in vehicle-treated animals, peaked after 12 hours and declined thereafter (FIGURE 3C). In LA1 treated animals, neutrophil accumulation was significantly reduced at 4 hours and stayed reduced after 12 hours. Comparable numbers of peritoneal neutrophils were observed after 24 hours among both groups of animals, showing that leukadherins significantly delayed neutrophil recruitment.

Tissue histology was used from TGC-induced peritonitis animals to determine if leukadherin-treatment led to sequestration of neutrophils in any particular organ. No sequestration of neutrophils was found in leukadherin-treated animals (FIGURE 3D) confirming that leukadherins do not lead to sequestration of neutrophils in any particular organ in mice and that the effects of leukadherins are indeed, in part, mediated through its increase in neutrophil adhesivity near sites of inflammation and decreased neutrophil motility.

Leukocyte recruitment precedes neointimal thickening and restenosis following percutaneous transluminal coronary angioplasty (PTCA) [3]. Denudation of the endothelial cell lining at the site of mechanical vascular injury leads to the deposition of fibrin and platelets, where selective binding between the platelet cell surface receptor GP Ibα and the integrin CD11b/CD18 expressed on the surface of leukocytes mediates the recruitment of leukocytes [80]. Indeed, in the experimental models of mechanical vascular injury, antibody-mediated CD11b/CD18 blockade or its absence (CD11b-/-) decreases intimal thickening after angioplasty or stent implantation [25]. To investigate the effects of pharmacologically activating CD11b/CD18 *in vivo* on this injury, the agonists were tested in an arterial balloon injury model in rats [56], Leukadherins LA1, LA3 or vehicle (DMSO) were administered to Fisher male rats 30 minutes prior to injury and continued injections every other day for three weeks. LA2 showed no effect, perhaps due to differences in the binding pocket between the rat and human αA (only ∼70% homology [68]). Injured arteries of the LA1 and LA3 treated animals developed significantly reduced neointimal thickening (neointima to media ratio of 0.16 ± 0.02 and 0.14 ± 0.01 respectively, p<0.05) as compared to vehicle treated animals (ratio of 0.23 ± 0.01) (FIGURES 3E-3J and 21A-21D). Control compound LA-C showed no effect (FIGURES 20A-20B). To determine if leukadherin treatment leads to reduced leukocyte accumulation, which precedes vascular remodeling, immunohistochemical analyses were performed of arteries three days post-injury using macrophage specific anti-CD68 antibody. A significant reduction was observed in the number of medial macrophages in the arteries of LA1 and LA3 treated animals (17.7 ± 3.1 and 6.9 ± 1.3, respectively, p<0.0001) over the vehicle controls (42.2 ± 6.7) (FIGURES 3E-3J and 21A-21D). Together, these results show that leukadherin treatment leads to reduced accumulation of leukocytes at the site of vascular injury and subsequent decrease in neointimal thickening.

Surprisingly, using the following experiment, it was found that integrin agonists (leukadherins) have therapeutic advantages over integrin antagonists in treating inflammatory injury. A head-to-head comparison was performed between a well-characterized CD11b/CD18 antagonist (anti-GD11b antibody M1/70) and LA1 using an established mouse model of kidney disease, the anti-glomerular basement membrane (anti-GBM) nephritis. This model is characterized by neutrophil infiltration that mediates urinary protein loss, including albumin. In line with an important role for CD11b in this disease, CD11b^{-/-} mice and anti-CD11b mAb treated rats show decreased leukocyte infiltration and a protection from proteinuria. Here, induction of disease in mice led to peak influx of neutrophils and maximal proteinuria at day 3 (FIGURES 3K-3L). M1/70 significantly decreased neutrophil influx and reduced proteinuria. However, LA1 produced significant and maximal decrease in both the number of infiltrated neutrophils and the proteinuria in treated mice, demonstrating a clear therapeutic advantage of agonists over antagonists,

To visualize effects of leukadherin treatment on leukocyte accumulation in live animals, transgenic Tg(mpx::eGFP) zebrafish were used that express GFP under a myeloid-specific peroxidase gene (mpx) promoter to specifically fluorescently tag neutrophils for live imaging of leukocyte recruitment [57]. Tailfin transection in zebrafish larvae at 3 days postfertilization (dpf) lead to a rapid and robust recruitment of neutrophils to the site of tissue injury (FIGURES 4A-4C and 22A-22D) [57]. Administration of LA1 and LA2 to uninjured larvae showed no observable effects (FIGURE 4B and 22B). However, both LA1 and LA2 significantly reduced the neutrophil accumulation in the zebrafish tailfin 4 hours post injury (15.6 ± 1.7 and 13.3 ± 2.1, respectively, p<0.0001) as compared to the treatment with vehicle alone (34.6 ± 4.5). Fluorescence imaging of the injured whole zebrafish larvae showed no difference in the total number of neutrophils in the leakadherin treated and untreated animals (FIGURE 23), showing that the leukadherin-mediated reduction in neutrophil accumulation at the site of injury was not due to an overall diminished neutrophil cell number, further confirming the results from the experiments in mice which showed that leukadherins do not cause cytotoxicity *in vivo* (Table 1). Finally, to determine if the *in vivo* effects of leukadherin treatment were reversible, LA1 and LA2 were administered to uninjured zebrafish for 4-8 hours, the zebrafish were rinsed, tailfin injury was induced and neutrophil accumulation was quantitated 4 hours post-wash. It was found that removal of compounds lead to a similar level of neutrophil accumulation at the injured tailfins as in untreated zebrafish larvae. Collectively, these data demonstrate that leukadherins down-regulate neutrophil accumulation at the site of tissue injury and that their *in vivo* effects can be reversed by the removal of these compounds.

Additionally, leukadherin treatment led to reduced T-cell proliferation in a mixed lymphocyte reaction (MLR), showing additional use in treating various inflammatory and autoimmune diseases.

Leukadherins increase neutrophil adhesion and decrease 2D chemotaxis *in vitro.* To determine if leukadherins have a similar effect on neutrophils *in vivo*, intravital microscopy was used on mouse cremaster muscle and it was found that leukadherins increased neutrophil adhesion in the postcapillary venules and decreased their rolling velocity (not shown), showing that these agonists behave similarly *in vivo.* More importantly, it was found that blocking anti-CD11b mAb M1/70 reversed the effects of leukadherins, confirming that the effects of leukadherins are via its agonism of CD11b/CD18.

Integrin activation was first proposed as a potential therapeutic strategy for modulating tissue invasion by inflammatory leukocytes more than 15 years ago [81], Here, it is shown that CD11b/CD18 agonists can modulate leukocyte recruitment and inflammatory injury. The leukadherin activated CD11b/CD18 increases leukocyte adhesion, which decreases leukocyte crawling and transendothelial migration and, thus, reduced recruitment into the inflamed/injured tissue [82]. The data presented here shows that integrin-specific small molecule mediated increase in leukocyte adhesion in the vasculature reduces leukocyte infiltration and inflammation and can be an effective pharmacologically targetable methodology to treat a variety of inflammatory and autoimmune diseases.

### EXAMPLE 2

One, the initial nonimmune injury during renal injury initiates an innate immune response causing inflammation and tissue injury (43). Endogenous ligands released from damaged tissues utilize Toll-like receptors (TLRs), such as Toll-like receptor 4 (TLR4), such that TLR4 activation on kidney cells and on leukocytes further exacerbates renal injury. CD11b/CD18, in addition to increasing cell adhesion and modulating migration, modulates the TLR4-mediated pro-inflammatory signaling in leukocytes (44-46), showing CD11b/CD18 has many roles in regulating leukocyte activation and inflammation. Two, CD11b/CD18 activation also mediates a number of intracellular signaling events, including production of reactive oxygen species (ROS) and modulation of a number of pro- and anti-inflammatory genes in myeloid cells (47-52). CD11/CD18 activation and ligand binding initiates outside-in signaling, including the activation of PI3-K/Akt and MAPK/ERK1/2 pathways (48, 53), thereby mimicking the anchorage-dependent pro-survival signals. Ligation and clustering of CD11b/CD18 also synergistically potentiates NF-kB dependent expression of pro-inflammatory cytokines (e.g.; IL1b, IL6, TNF-α) and other factors (e.g.; matrix metalloproteinases (MMPs)). However, GD11b/CD18 deficiency enhances TLR4-triggered production of pro-inflammatory cytokines, showing that CD11b/CD18 activation can be protective and can negatively regulate pro-inflammatory pathways in leukocytes (54-56). Leukadherins are novel compounds that activate CD11b/CD18 and reduce leukocyte recruitment *in vivo.* Here, it is hypothesized that leukadherin-mediated activation of CD11b/CD18 will reduce TLR4- and TNFR-mediated leukocyte activation and generation of pro-inflammatory molecules, proving that CD11b/CD18 activation can negatively regulate pro-inflammatory pathways in leukocytes. It is also hypothesized that activation of CD11b/CD18 limits the pro-inflammatory responses in leukocytes by increasing the degradation of intracellular adaptor MyD88, suppressing the NF-kB pathway and thereby reducing levels of leukocyte generated pro-inflammatory cytokines, chemokines, ROS and MMPs. Three, as leukocytes are a source for circulating suPAR, a factor associated with FSGS, it is hypothesized that leukocytes play a role in non-inflammatory glomerulopathies and such leukocytic activation can also be reduced with leukadherins.

### Data

1.1 Leukadherins increase pAKT but do not mimic integrin ligands. It is not clear if integrin activation versus integrin ligation and clustering differentially synergizes with TLR and cytokine receptor signaling. It has been shown that activated (activated by Mn2+-ions or with activating mAbs) or ligand-bound (48) CD11b/CD1 8 is present as clusters on neutrophil surface (48). It remains unclear if activation with leukadherin would lead to increased avidity. Furthermore, CD11b/CD18 agonists Mn2+ ions and activating mAbs are known to induce pERK1/2 and pAkt (57, 58), as does CD11b/CD18 ligand Fg (48), thereby inducing pro-survival and pro-inflammatory signaling. Similarly, binding of activating mAbs to CD11b/CD18 is sufficient to induce outside-in signaling and mimics ligand-bound state (57, 58), showing that stabilization of active integrin conformation by exogenous agents can have harmful immunologic consequences (58). It was not known how the newly discovered CD1 1b/CD18 agonists modulate intracellular events.

Therefore, the effects of integrin activation by leukadherins on such intracellular signaling events was investigated. It was found that LA1-3 treated CD11b/CD18₊ cells showed no pERK1/2 (FIGURE 25A), similar to the DMSO treated cells. However, incubation with ligand Fg showed a clear, expected increase in pERK in these cells, as did treatment with phorbol ester PMA (not shown). Surprisingly, LA1-3 treatment resulted in robust Akt phosphorylation as compared to treatment with DMSO alone (FIGURE 25B). As pAkt is known to suppress inflammatory signal (e.g. LPS) dependent expression of pro-inflammatory cytokines, this shows that leukadherins may also suppress pro-inflammatory cytokine expression in leukocytes. To test whether agonist mediated integrin activation also changes avidity, immunofluorescence microscopy was used for imaging and integrin clustering was analyzed on cell surface (48). In the absence of ligands, cells showed no detectable macro clustering of CD11b/CD18 in the absence (DMSO) or presence of LA1, LA2 and LA3 (32), showing that agonist binding alone does not induce integrins clustering. Expectedly, addition of external ligand Fg produced marked clustering in both conditions.

1.2 Leukadherins decrease secretion of pro-inflammatory factors. In a proof-of-concept experiment to test the effect of leukadherin treatment on secretion of pro-inflammatory factors by leukocytes, WT mouse neutrophils or macrophages were stimulated with LPS in the absence or presence of agonist LA1 and levels of various factors were measured in the cell culture supernatant. FIGURES 26A-26D show that LPS treatment significantly increased the levels of IL-6, TNF-α and MCP-1, as compared to unstimulated cells, and that addition of LA1 significantly decreased the levels of all three factors in the supernatant. Similarly, LA1 diminished the levels of reactive-oxygen species (ROS) in TNF-α-activated human neutrophils (FIGURE 27). These data show that CD11b/CD18 activation with leukadherins can suppress pro-inflammatory signaling in leukocytes and have anti-inflammatory effects.

1.3 Leukadherins accelerate MyD88 degradation. TLR4-mediated signaling requires participation of the adaptor protein MyD88 (43) and MyD88-/- mice are protected from kidney damage following IRI. TLR4 activation leads to the binding and stabilization of adaptor protein MyD88, which recruits downstream kinases to initiate Nf-kB-mediated pro-inflammatory signaling. Subsequently, TLR4 signaling induces a negative feedback loop by endogenous activation of CD11b/CD18, which activates Syk to phosphorylate MyD88, tagging it for ubiquitin-mediated destruction. This shows that CD11b/CD18 agonists would lead to accelerated degradation of MyD88, thereby inducing a faster dampening of TLR4-mediated pro-inflammatory signaling pathways. A pilot experiment was performed to validate this hypothesis by determining the levels of MyD88 in human monocytic THP-1 cells. It was found that TLR4 agonist LPS produced a robust MyD88 signal that was stable for at least 4 hours (FIGURE 28). However, co-treatment of cells with LA1 lead to a much faster degradation⁻ of MyD88. Indeed, incubation of cells with LA1 alone (in the absence of LPS) resulted in a complete degradation of MyD88 in less than 2 hours, showing that activation of CD1 1b/CD18 can down-modulate MyD88-dependent intracellular signaling in leukocytes.

1.4 Leukadherins protect mice from sepsis. Sepsis is characterized by a severe inflammatory response to infection, and its complications lead to multiorgan failure, including acute kidney injury, and can be fatal (59). Given that CD11b/CD18 activation via leukadherins dramatically reduced TLR4-mediated intracellular signaling, it was wondered if these compounds would also reduce the TLR4-induced sepsis in WT mice, as is observed with MyD88-/- animals. After induced of sepsis upon cecal ligation and puncture (CLP) (59), 20% of untreated mice died within 24 hours (FIGURE 29) and all of them died within 72 hours, whereas 80% of LA1 treated mice were alive for 36 hours and 40% of them ultimately survived, strongly showing that CD11b/CD18 agonists can reduce TLR4-mediated inflammation *in vivo.* A more complete analysis of the animals is currently underway.

1.5 Decreasing circulating suPAR levels in sera with novel small molecules targeting leukocytes. Leukocytes retain large amounts of uPAR in intracellular pools and, upon activation, increase surface expression of uPAR as well as release suPAR into circulation (22). Given their central role in the production of suPAR, leukocytes are viable target cells for reducing suPAR levels in the serum. It was found that the leukadherin treatment significantly reduced circulating suPAR levels and preserved kidney function in a mouse model of anti-GBM nephritis (FIGURE 30), showing that these compounds can be therapeutically relevant as agents for mitigating leukocyte-dependent circulating disease factors.

1.6 Leukadherins reduced renal IRI at 24 hours. To evaluate use of leukadherins in the IRI induced changes in the kidney function of WT mice, a proof-of-concept experiment was performed. B6 male mice (8 - 12 weeks of age) were anesthetized and kept on a heating pad to keep them at a constant 37°C temperature. Next, an abdominal incision was made and the renal pedicles were occluded bilaterally with a nontraumatic vascular clamp for 30 minutes, the clamp then removed and the surgical incision was closed. Sham surgery was performed with an identical procedure but without application of the clamps. Renal IR injury was assessed at 24 hours post-ischemia by measuring the serum creatinine levels (FIGURE 31). Mouse sera from the tail incision was used for the analyses of sCr (and BUN levels (not shown)) using manual kits (Stanbio Laboratory). Administration of leukadherins LA1 and LA2 30 minutes prior to IR injury showed a significant reduction in sCr levels as compared to vehicle DMSO treated animals, showing that these compounds have a reno-protective effect.

Throughout this application, various publications, including United States patents, are referenced by author and year and patents by number. Full citations for the publications are listed below. The disclosures of these publications and patents in their entireties are hereby incorporated by reference into this application in order to more fully describe the state of the art to which this invention pertains.

The invention has been described in an illustrative manner, and it is to be understood that the terminology, which has been used is intended to be in the nature of words of description rather than of limitation.

Obviously, many modifications and variations of the present invention are possible in light of the above teachings. It is, therefore, to be understood that within the scope of the appended claims, the invention can be practiced otherwise than as specifically described.

### REFERENCES

1. Canadas, T.N. and X. Cullere, Neutrophil beta2 integrins: moderators of life or death decisions. Trends Immunol, 2005. 26(7): p. 388-95.
2. Ley, K., et al., Getting to the site of inflammation: the leukocyte adhesion cascade updated. Nat Rev Immunol, 2007. 7(9): p. 678-89.
3. Simon, D.I., et al., Decreased neointimal formation in Mac-1(-/-) mice reveals a role for inflammation in vascular repair after angioplasty. J Clin Invest, 2000. 105(3): p. 293-300.
4. Cao, C., et al., A specific role of integrin Mac-1 in accelerated macrophage efflux to the lymphatics. Blood, 2005. 106(9): p. 3234-41.
5. Tang, T., et al., A role for Mac-1 (CDIIb/CD18) in immune complex-stimulated neutrophil function in vivo: Mac-1 deficiency abrogates sustained Fcgamma receptor-dependent neutrophil adhesion and complement-dependent proteinuria in acute glomerulonephritis. J Exp Med, 1997. 186(11): p. 1853-63.
6. Plow, E.F., et al., Ligand binding to integrins. J Biol Chem, 2000. 275(29): p. 21785-8.
7. Soriano, S.G., et al., Mice deficient in Mac-1 (CD11b/CD18) are less susceptible to cerebral ischemia/reperfusion injury. Stroke, 1999. 30(1): p. 134-9.
8. Kubota, Y., et al., M-CSF inhibition selectively targets pathological angiogenesis and lymphangiogenesis. J Exp Med, 2009. 206(5): p. 1089-102.
9. Hynes, R.O., integrins: bidirectional, allosteric signaling machines. Cell, 2002. 110(6): p. 673-87.
10. Arnaout, M.A., Leukocyte adhesion molecules deficiency: its structural basis, pathophysiology and implications for modulating the inflammatory response. Immunol Rev, 1990. 114: p. 145-80.
11. Phillipson, M., et al., Intraluminal crawling of neutrophils to emigration sites: a molecularly distinct process from adhesion in the recruitment cascade. J Exp Med, 2006. 203(12): p. 2569-75.
12. Ahn, G.O., et al., Inhibition of Mac-1 (CD11b/CD18) enhances tumor response to radiation by reducing myeloid cell recruitment. Proc Natl Acad Sci U S A. 107(18): p. 8363-8.
13. Ophascharoensuk, V., et al., Role of intrinsic renal cells versus infiltrating cells in glomerular crescent formation. Kidney Int, 1998. 54(2): p. 416-25.
14. Le Hir, M., et al., Podocyte bridges between the tuft and Bowman's capsule: an early event in experimental crescentic glomerulonephritis. J Am Soc Nephrol, 2001. 12(10): p. 2060-71.
15. Tang, T., et al., A role for Mac-1 (CDIIb/CD18) in immune complex-stimulated neutrophil function in vivo: Mac-1 deficiency abrogates sustained Fcgamma receptor- dependent neutrophil adhesion and complement-dependent proteinuria in acute glomerulonephritis. J Exp Med, 1997. 186(11): p. 1853-63.
16. Fan, S.T. and T.S. Edgington, Coupling of the adhesive receptor CD11b/CD18 to functional enhancement of effector macrophage tissue factor response. J Clin Invest, 1991. 87(1): p. 50-7.
17. Whitlock, B.B., et al., Differential roles for alpha(M)beta(2) integrin clustering or activation in the control of apoptosis via regulation of akt and ERK survival mechanisms. J Cell Biol, 2000. 151(6): p. 1305-20.
18. Rezzonico, R., et al., Ligation of CD11b and CD11c beta(2) integrins by antibodies or soluble CD23 induces macrophage inflammatory protein 1alpha (MIP-1alpha) and MIP-1beta production in primary human monocytes through a pathway dependent on nuclear factor-kappaB. Blood, 2001. 97(10): p. 2932-40.
19. Guha, M. and N. Mackman, The phosphatidylinositol 3-kinase-Akt pathway limits lipopotysaccharide activation of signaling pathways and expression of inflammatory mediators in human monocytic cells. J Biol Chem, 2002. 277(35): p. 32124-32.
20. Rubel, C., et al., Fibrinogen-CD11b/GD18 interaction activates the NF-kappa B pathway and delays apoptosis in human neutrophils. Eur J Immunol, 2003. 33(5): p. 1429-38.
21. Kettritz, R., et al., Integrins and cytokines activate nuclear transcription factor-kappaB in human neutrophils. J Biol Chem, 2004. 279(4): p. 2657-65.
22. Giancotti, F.G. and E, Ruoslahti, Integrin signaling. Science, 1999. 285(5430): p. 1028-32.
23. Sheikh, S. and G.B, Nash, Continuous activation and deactivation of integrin CD11b/CD18 during de novo expression enables rolling neutrophils to immobilize on platelets. Blood, 1996. 87(12): p. 5040-50.
24. Jaeschke, H., et al., Functional inactivation of neutrophils with a Mac-1 (CD11b/CD18) monoclonal antibody protects against ischemia-reperfusion injury in rat liver. Hepatology, 1993. 17(5): p. 915-23.
25. Rogers, C., E.R. Edelman, and D.I. Simon, A mAb to the beta2-leukocyte integrin Mac-1 (CD11b/CD18) reduces intimal thickening after angioplasty or stent implantation in rabbits. Proc Natl Acad Sci U S A, 1998. 95(17): p. 10134-9.
26. Wilson, I., et al., Inhibition of neutrophil adherence improves postischemic ventricular performance of the neonatal heart. Circulation, 1993. 88(5 Pt 2): p. II372-9.
27. Plow, E.F. and L. Zhang, A MAC-1 attack: integrin functions directly challenged in knockout mice. J Clin Invest, 1997. 99(6): p. 1145-6.
28. Yonekawa, K. and J.M. Harlan, Targeting leukocyte integrins in human diseases. J Leukoc Biol, 2005. 77(2): p. 129-40.
29. Dove, A., CD18 trials disappoint again. Nat Biotechnol, 2000. 18(8): p. 817-8.
30. Shimizu, K., et al., Leukocyte integrin Mac-1 promotes acute cardiac allograft rejection. Circulation, 2008. 117(15): p. 1997-2008.
31. Ramamoorthy, C., et al., CD18 adhesion blockade decreases bacterial clearance and neutrophil recruitment after intrapulmonary E. coli, but not after S. aureus. J Leukoc Biol, 1997. 61(2): p. 167-72.
32. Lum, A.F., et al., Dynamic regulation of LFA-1 activation and neutrophil arrest on intercellular adhesion molecule 1 (ICAM-1) in shear flow. J Biol Chem, 2002. 277(23): p. 20660-70.
33. Allison, M., PML problems loom for Rituxan. Nat Biotechnol, 2010. 28(2): p. 105-6.
34. Bansal, V.S., et al., Small molecule antagonists of complement receptor type 3 block adhesion and adhesion-dependent oxidative burst in human polymorphonuclear leukocytes. J Pharmacol Exp Ther, 2003. 304(3): p. 1016-24.
35. Bjorklund, M., et al., Stabilization of the activated alphaMbeta2 integrin by a small molecule inhibits leukocyte migration and recruitment. Biochemistry, 2006. 45(9): p. 2862-71.
36. Faridi, M.H., et al., Identification of novel agonists of the integrin CD11b/CD18, Bioorg Med Chem Lett, 2009.
37. Arnaout, M.A., et al., Inhibition of phagocytosis of complement C3- or immunoglobulin G-coated particles and of C3bi binding by monoclonal antibodies to a monocyte-granulocyte membrane glycoprotein (Mol). J Clin Invest, 1983. 72(1): p. 171-9.
38. Wright, S.D., et al., Identification of the C3bi receptor of human monocytes and macrophages by using monoclonal antibodies. Proc Natl Acad Sci U S A, 1983. 80(18): p. 5699-703.
39. Hogg, N., et al., A novel leukocyte adhesion deficiency caused by expressed but nonfunctional beta2 integrins Mac-1 and LFA-1. J Clin Invest, 1999. 103(1): p. 97-106.
40. Dransfield, I. and N. Hogg, Regulated expression of Mg2+ binding epitope on leukocyte integrin alpha subunits. EMBO J, 1989. 8(12): p. 3759-65.
41. Coxon, A., et al., A novel role for the beta 2 integrin CD11b/CD18 in neutrophil apoptosis: a homeostatic mechanism in inflammation. Immunity, 1996. 5(6): p. 653-66.
42. Park, J.Y., M.A. Arnaout, and V. Gupta, A simple, no-wash cell adhesion-based high-throughput assay for the discovery of small-molecule regulators of the integrin CD11b/CD18. J Biomol Screen, 2007. 12(3): p. 406-17.
43. Gupta, V., et al., The beta-tail domain (betaTD) regulates physiologic ligand binding to integrin CD11b/CD18. Blood, 2007. 109(8): p. 3513-20.
44. Alonso, J.L., et al., Does the integrin alphaA domain act as a ligand for its betaA domain? Curr Biol, 2002. 12(10): p. R340-2.
45. Gupta, V., HTS identification of compounds that enhance the binding of CD11b/CD18 to fibrinogen via a luminescence assay. Pubchem Assay ID 1499, 2009.
46. Chen, L.Y,. et al., Impaired glucose homeostasis, neutrophil trafficking and function in mice lacking the glucose-6-phosphate transporter. Hum Mol Genet, 2003. 12(19): p. 2547-58.
47. Bergmeier, W., et al., Mice lacking the signaling molecule CalDAG-GEFI represent a model for leukocyte adhesion deficiency type III. J Clin Invest, 2007. 117(6): p. 1699-707,
48. Szczur, K., et al., Rho GTPase CDC42 regulates directionality and random movement via distinct MAPK pathways in neutrophils. Blood, 2006. 108(13): p. 4205-13.
49. Zigmond, S.H., Orientation chamber in chemotaxis. Methods Enzymol, 1988. 162: p. 65-72.
50. Pluskota, E., et al., Neutrophil apoptosis: selective regulation by different ligands of integrin alphaMbeta2. J Immunol, 2008. 181(5): p. 3609-19.
51. Li, R. and M.A. Arnaout, Functional analysis of the beta 2 integrins. Methods Mol Biol, 1999. 129: p. 105-24.
52. Xiong, J.P., et al., An isoleucine-based allosteric switch controls affinity and shape shifting in integrin CD11b A-domain. J Biol Chem, 2000. 275(49): p. 38762-7.
53. Lu, C., et al., Epitope mapping of antibodies to the C-terminal region of the integrin beta 2 subunit reveals regions that become exposed upon receptor activation. J Immunol, 2001. 166(9): p. 5629-37.
54. Xiong, J.P., et al., New insights into the structural basis of integrin activation. Blood, 2003. 102(4): p. 1155-9.
55. Semmrich, M., et al., Importance of integrin LFA-1 deactivation for the generation of immune responses. J Exp Med, 2005. 201(12): p. 1987-98.
56. Gabeler, E.E., et al., A comparison of balloon injury models of endovascular lesions in rat arteries, BMC Cardiovasc Disord, 2002. 2: p. 16.
57. Renshaw, S.A., et al., A transgenic zebrafish model of neutrophilic inflammation. Blood, 2006. 108(13): p. 3976-8.
58. Nüsslein-Volhard, C., Zebrafish - A Practical Approach. Practical Approach Series. 2002: Oxford University Press.
59. Lee, J.O., et al., Crystal structure of the A domain from the alpha subunit of integrin CR3 (CD11lb/CD18). Cell, 1995. 80(4): p. 631-8.
60. Xiong, J.P., et al., An isoleucine-based allosteric switch controls affinity and shape shifting in integrin CD11b A-domain. J Biol Chem, 2000. 275(49): p. 38762-7,
61. Weitz-Schmidt, G., et al., Statins selectively inhibit leukocyte function antigen-1 by binding to a novel regulatory integrin site. Nat Med, 2001. 7(6): p. 687-92.
62. Lee, J.O., et al., Two conformations of the integrin A-domain (I-domain): a pathway for activation? Structure, 1995. 3(12): p. 1333-40.
63. Shimaoka, M., et al., Stabilizing the integrin alpha M inserted domain in alternative conformations with a range of engineered disulfide bonds. Proc Natl Acad Sci U S A, 2002. 99(26): p. 16737-41.
64. McCleverty, C.J. and R.C. Liddington, Engineered allosteric mutants of the integrin alphaMbeta2 / domain: structural and functional studies. Biochem J, 2003. 372(Pt 1): p. 121-7.
65. Sherman, W., et al., Novel procedure for modeling ligand/receptor induced fit effects. J Med Chem, 2006. 49(2): p. 534-53.
66. Kevin, J.B., et al., Scalable Algorithms for Molecular Dynamics Simulations on Commodity Clusters. Proceedings of the ACM/IEEE Conference on Supercomputing (SC06), Tampa, Florida, November 11-17, 2006.
67. Barnard, J.W., et al., Neutrophil inhibitory factor prevents neutrophil-dependent lung injury. J Immunol, 1995. 155(10): p. 4876-81.
68. Zerria, K., et al., Recombinant integrin CD11b A-domain blocks polymorphonuclear cells recruitment and protects against skeletal muscle inflammatory injury in the rat. Immunology, 2006. 119(4): p. 431-40.
69. Feng, Y., et al., Peptides derived from the complementarity-determining regions of anti-Mac-1 antibodies block intercellular adhesion molecule-1 interaction with Mac-1. J Biol Chem, 1998. 273(10): p. 5625-30.
70. Li, R., et al., Two functional states of the CD11b A-domain: correlations with key features of two Mn2+-complexed crystal structures. J Cell Biol, 1998. 143(6): p. 1523-34.
71. Abad-Zapatero, C. and J.T. Metz, Ligand efficiency indices as guideposts for drug discovery. Drug Discov Today, 2005. 10(7): p. 464-9.
72. Friesner, R.A., et al., Glide: a new approach for rapid, accurate docking and scoring. 1. Method and assessment of docking accuracy. J Med Chem, 2004, 47(7): p. 1739-49.
73. Guimaraes, C.R. and M. Cardozo, MM-GB/SA rescoring of docking poses in structure-based lead optimization. J Chem Inf Model, 2008. 48(5): p. 958-70.
74. Liu, L., et al., Requirement for RhoA kinase activation in leukocyte de-adhesion. J Immunol, 2002. 169(5): p. 2330-6.
75. Huttenlocher, A., M.H. Ginsberg, and A.F. Horwitz, Modulation of cell migration by integrin-mediated cytoskeletal linkages and ligand-binding affinity. J Cell Biol, 1996. 134(6): p. 1551-62.
76. Palecek, S.P., et al., Integrin-ligand binding properties govern cell migration speed through cell-substratum adhesiveness. Nature, 1997. 385(6616):p. 537-40.
77. Park, E.J., et al., Aberrant activation of integrin alpha4beta7 suppresses lymphocyte migration to the gut. J Clin Invest, 2007. 117(9): p, 2526-38,
78. de Bruyn, K.M., et al., The small GTPase Rap1 is required for Mn(2+)- and antibody-induced LFA-1- and VLA-4-mediated cell adhesion. J Biol Chem, 2002. 277(33): p. 29468-76.
79. Lefort, C.T., et al., Outside-in signal transmission by conformational changes in integrin Mac-1. J Immunol, 2009. 183(10): p. 6460-8.
80. Wang, Y., et al., Leukocyte engagement of platelet glycoprotein Ibalpha via the integrin Mac-1 is critical for the biological response to vascular injury. Circulation, 2005. 112(19): p. 2993-3000.
81. Kuijpers, T.W., et al., Freezing adhesion molecules in a state of high- avidity binding blocks eosinophil migration. J Exp Med, 1993. 178(1): p. 279-84.
82. Park, E.J., et al., Distinct roles for LFA-1 affinity regulation during T-cell adhesion, diapedesis, and interstitial migration in lymph nodes. Blood, 2010. 115(8): p. 1572-81,
83. Han C, Jin J, Xu S, Liu H, Li N, Cao X. Integrin CD11b negatively regulates TLR-triggered inflammatory responses by activating Syk and promoting degradation of MyD88 and TRIF via Cbl-b. Nat immunol.11(8):734-42.
84. Cao C, Gao Y, Li Y, Antalis TM, Castellino FJ, Zhang L. The efficacy of activated protein C in murine endotoxemia is dependent on integrin CD11b. J Clin Invest.120(6):1971-80.
85. Means TK, Luster AD. Integrins limit the Toll. Nat Immunol.11(8):691-3.
86. Driessens MH, van Hulten P, Zuurbier A, La Riviere G, Roos E. Inhibition and stimulation of LFA-1 and Mac-1 functions by antibodies against murine CD18. Evidence that the LFA-1 binding sites for ICAM-1, -2, and - 3 are distinct. J Leukoc Biol. 1996;60(6):758-65.
87. Reichert F, Slobodov U, Makranz C, Rotshenker S. Modulation (inhibition and augmentation) of complement receptor-3-mediated myelin phagocytosis. Neurobiol Dis. 2001;8(3):504-12.
88. Pavlovic MD, Colic M, Pejnovic N, Tamatani T, Miyasaka M, Dujic A. A novel anti-rat CD18 monoclonal antibody triggers lymphocyte homotypic aggregation and granulocyte adhesion to plastic: different intracellular signaling pathways in resting versus activated thymocytes. Eur J Immunol. 1994;24(7):1640-8.
89. Stockl J, Majdic O, Pickl WF, Rosenkranz A, Prager E, Gschwantler E, et al. Granulocyte activation via a binding site near the C-terminal region of complement receptor type 3 alpha-chain (CD11b) potentially involved in intramembrane complex formation with glycosylphosphatidylinositol-anchored Fc gamma RIIIB (CD16) molecules. J Immunol. 1995;154(10):5452-63.
90. Petruzzelli L, Maduzia L, Springer TA. Activation of lymphocyte function-associated molecule-1 (CD11a/CD18) and Mac-1 (CD11b/CD18) mimicked by an antibody directed against CD18. J Immunol. 1995;155(2):854-66.
91. Keizer GD, Visser W, Vliem M, Figdor CG. A monoclonal antibody (NKI-L16) directed against a unique epitope on the alpha-chain of human leukocyte function-associated antigen 1 induces homotypic cell-cell interactions. J Immunol. 1988;140(5):1393-400.
92. Andrew D, Shock A, Ball E, Ortlepp S, Bell J, Robinson M. KIM185, a monoclonal antibody to CD18 which induces a change in the conformation of CD18 and promotes both LFA-1- and CR3-dependent adhesion. Eur J Immunol. 1993;23(9):2217-22.
93. Robinson MK, Andrew D, Rosen H, Brown D, Ortlepp S, Stephens P, et al. Antibody against the Leu-CAM beta-chain (CD18) promotes both LFA-1- and CR3-dependent adhesion events. J Immunol. 1992;148(4):1080-5.
94. Dransfield I, Hogg N. Regulated expression of Mg2+ binding epitope on leukocyte integrin alpha subunits. EMBO J. 1989;8(12):3759-65.
95. Bazil V, Stefanova I, Hilgert I, Kristofova H, Vanek S, Horejsi V, Monoclonal antibodies against human leucocyte antigens, IV. Antibodies against subunits of the LFA-1 (CD11a/CD18) leucocyte-adhesion glycoprotein. Folia Biol (Praha). 1990;36(1):41-50,

### Clauses

1. A pharmaceutical formulation of a β2 integrin agonist, comprising an effective amount of a β2 integrin agonist and a pharmaceutically acceptable carrier.
2. The pharmaceutical formulation of clause 1, wherein said β2 integrin agonist is a compound of Formula (I) 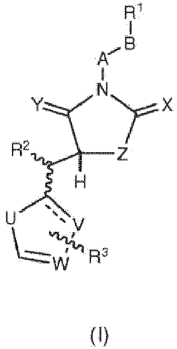 wherein
   A is absent or is alkyl;
   B is absent or is chosen from the group consisting of alkyl, alkenyl, O, S and NR4;
   N is chosen from the group consisting of nitrogen and CR4;
   X and Y are independently chosen from the group consisting of O and S;
   Z is chosen from the group consisting of CR4, O, S and NR4;
   U, V, and W are independently chosen from the group consisting of CR4, O, S and NR4;
   R1 and R3 are independently chosen from the group consisting of acyl, alkyl, alkenyl, alkynyl, hydroxyalkyl, aminoalkyl, thioalkyl, aryl, aralkyl, carboxyaryl, alkoxyalkyl, alkoxyaryl, alcoxycarbonylaryl, aminoaryl, amidoaryl, haloaryl, heteroaryl, heteroaralkyl, carbocyclyl, heterocyclyl, heterocyclylalkyl, alkoxycarbonyl, alkylaminocarbonyl, alkylthiocarbonyl, sulfonate, alkylsulfonate, arylsulfonate, sulfone, alkylsulfone, arylsulfone, sulfoxide, alkylsulfoxide, arylsulfoxide, alkylsulfonamide, arylsulfonamide, and sulfonamide, piperidinyl, morpholinyl, pyrrolidinyl, phenyl, pyridyl, pyrimidinyl, furyl, thienyl, pyrrolyl, imidazolyl, triazolyl, pyrazolyl, thiazolyl, isoxazolyl, oxazolyl, oxadiazolyl, indolyl, naphthyl, quinolinyl, isoquinolinyl, quinoxalinyl, benzyl, benzofuryl, dibenzofuryl, benzthienyl, benzoxazolyl, benzothiazolyl, benzimidazolyl, pyridoimidazolyl, pyrimidoimidazolyl, pyridopyrazolyl, pyrazolopyrimidinyl, and any of which is substituted with 1-6 independent substituents;
   R2 is chosen from the group consisting- of hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl;
   R4 is absent or is chosen from the group consisting of hydrogen and alkyl; and said β2 integrin agonist is a substantially pure single conformer or a pharmaceutically acceptable salt or composition thereof.
3. The pharmaceutical formulation of clause 1, wherein said β2 integrin agonist is a compound of Formula (II) 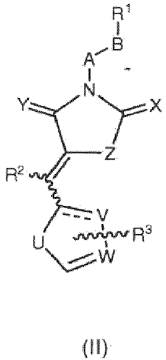 wherein
   A is absent or is alkyl;
   B is absent or is chosen from the group consisting of alkyl, alkenyl, O, S and NR4;
   N is chosen from the group consisting of nitrogen and CR4;
   X and Y are independently chosen from the group consisting of O and S;
   Z is chosen from the group consisting of CR4, O, S or NR4;
   U, V, and W are independently chosen from the group consisting of CR4, O, S and NR4;
   R1 and R3 are independently chosen from the group consisting of acyl, alkyl, alkenyl, alkynyl, hydroxyalkyl, aminoalkyl, thioalkyl, aryl, aralkyl, carboxyaryl, alkoxyalkyl, alkoxyaryl, alcoxycarbonylaryl, aminoaryl, amidoaryl, haloaryl, heteroaryl, heteroaralkyl, carbocyclyl, heterocyclyl, heterocyclylalkyl, alkoxycarbonyl, alkylaminocarbonyl, alkylthiocarbonyl, sulfonate, alkylsulfonate, arylsulfonate, sulfone, alkylsulfone, arylsulfone, sulfoxide, alkylsulfoxide, arylsulfoxide, alkylsulfonamide, arylsulfonamide, and sulfonamide, piperidinyl, morpholinyl, pyrrolidinyl, phenyl, pyridyl, pyrimidinyl, furyl, thienyl, pyrrolyl, imidazolyl, triazolyl, pyrazolyl, thiazolyl, isoxazolyl, oxazolyl, oxadiazolyl, indolyl, naphthyl, quinolinyl, isoquinolinyl, quinoxalinyl, benzyl, benzofuryl, dibenzofuryl, benzthienyl, benzoxazolyl, benzothiazolyl, benzimidazolyl, pyridoimidazolyl, pyrimidoimidazolyl, pyridopyrazolyl, pyrazolopyrimidinyl, and any of which is substituted with 1-6 independent substituents;
   R2 chosen from the group consisting of hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl;
   R4 is absent or is chosen from the group consisting of hydrogen and alkyl; and said β2 integrin agonist is a substantially pure single conformer or a pharmaceutically acceptable salt or composition thereof.
4. The pharmaceutical formulation of clause 3, wherein said β2 integrin agonist is chosen from the group consisting of 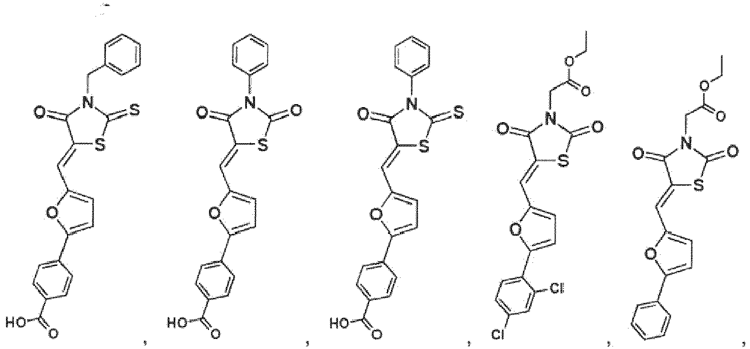 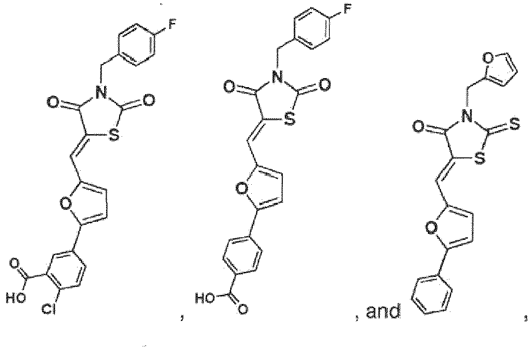 wherein said β2 integrin agonist is a substantially pure single conformer, or a pharmaceutically acceptable salt or composition thereof.
5. The pharmaceutical formulation of clause 1, wherein said β2 integrin agonist is a compound of Formula (III) 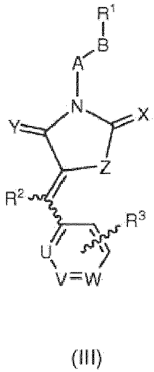 wherein
   A is absent or is alkyl;
   B is absent or is chosen from the group consisting of alkyl, alkenyl, O, S and NR4;
   N is chosen from the group consisting of nitrogen and CR4;
   X and Y are independently chosen from the group consisting of O and S;
   Z is chosen from the group consisting of CR4, O, S or NR4;
   U, V, and W are independently chosen from the group consisting of CR4, O, S and NR4;
   R3 is 1-6 independent substituents present at position(s) 1-6 of the aryl ring;
   R1 and R3 are independently chosen from the group consisting of acyl, alkyl, alkenyl, alkynyl, hydroxyalkyl, aminoalkyl, thioalkyl, aryl, aralkyl, carboxyaryl, alkoxyalkyl, alkoxyaryl, alcoxycarbonylaryl, aminoaryl, amidoaryl, haloaryl, heteroaryl, heteroaralkyl, carbocyclyl, heterocyclyl, heterocyclylalkyl, alkoxycarbonyl, alkylaminocarbonyl, alkylthiocarbonyl, sulfonate, alkylsulfonate, arylsulfonate, sulfone, alkylsulfone, arylsulfone, sulfoxide, alkylsulfoxide, arylsulfoxide, alkylsulfonamide, arylsulfonamide, and sulfonamide, piperidinyl, morpholinyl, pyrrolidinyl, phenyl, pyridyl, pyrimidinyl, furyl, thienyl, pyrrolyl, imidazolyl, triazolyl, pyrazolyl, thiazolyl, isoxazolyl, oxazolyl, oxadiazolyl, indolyl, naphthyl, quinolinyl, isoquinolinyl, quinoxalinyl, benzyl, benzofuryl, dibenzofuryl, benzthienyl, benzoxazolyl, benzothiazolyl, benzimidazolyl, pyridoimidazoiyl, pyrimidoimidazolyl, pyridopyrazolyl, pyrazolopyrimidinyl, and any of which is substituted with 1-6 independent substituents;
   R2 chosen from the group consisting of hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl;
   R4 is absent or is chosen from the group consisting of hydrogen and alkyl; and said β2 integrin agonist is a substantially pure single conformer, or a pharmaceutically acceptable salt or composition thereof.
6. The pharmaceutical formulation of clause 1, further including an active agent in a synergistic amount.
7. The pharmaceutical formulation of clause 1, further including a TNF-α blocker chosen from the group consisting of etanercept, infliximab, and adalimumab in a synergistic amount.
8. The pharmaceutical formulation of clause 1, further including an anti-inflammatory drug chosen from the group consisting of steroidal anti-inflammatory drugs (NSAIDS), salicylates, acetic acid derivatives, indomethacin, propionic acid derivatives, ibuprofen, naproxen, Coxll inhibitors, celecoxib, and rofecoxib in a synergistic amount.
9. The pharmaceutical formulation of clause 1, further including an anti-cancer compound of cilengitide in a synergistic amount.
10. The pharmaceutical formulation of clause 1, further including an anti-rejection drug chosen from the group consisting of tacrolimus, cyclosporine, and steroids in a synergistic amount.
11. The pharmaceutical formulation of clause 1, further including an anti-clotting drug chosen from the group consisting of warfarin, heparin, aspirin, ticlopidine, clopidogrel, dipyridamole, and glycoprotein IIb/IIIa receptor agonists in a synergistic amount.
12. The pharmaceutical formulation of clause 1, further including a steroid chosen from the group consisting of tobramycin, dexamethasone, neomycin, hydrocortisone, prednisone, and erythromycin in a synergistic amount.
13. The pharmaceutical formulation of clause 1, further including a sphingosine-1-phosphate receptor modulator of fingolimod in a synergistic amount.
14. The pharmaceutical formulation of clause1, further including a drug-eluting stent media.
15. A method of activating β2 integrins, including the step of interacting the β2 integrins with a β2 integrin agonist.
16. The method of clause15, wherein said interacting step is further defined as occupying a binding pocket in an αA-domain of the β2 integrins with the β2 integrin agonist.
17. The method of clause 15, wherein said interacting step is further defined as interacting a polar end of the β2 integrin agonist with polar residues of the β2 integrins, and interacting a non-polar end of the β2 integrin agonist with a hydrophobic pocket in the β2 integrins.
18. The method of clause 17, wherein said interacting step is further defined as interacting the β2 integrin agonist with a hydrophobic pocket lined by residues L312, I308, L305 (α7 helix), L164, V160, F156 (α1 helix), and Y267, I269, I236, V238, I236, I135 (central beta sheet) of the β2 integrins.
19. The method of clause15, wherein said interacting step is further defined as selectively interacting CD11b/CD18 with the agonist.
20. A method of treating a patient, including the steps of:
   administering an effective amount of a β2 integrin agonist; and
   activating β2 integrins.
21. The method of clause 20, wherein the β2 integrin agonist is a compound of Formula (I) 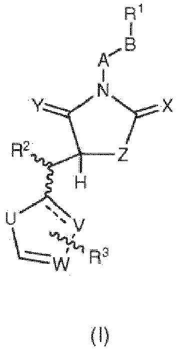 wherein
   A is absent or is alkyl;
   B is absent or is chosen from the group consisting of alkyl, alkenyl, O, S and NR4;
   N is chosen from the group consisting of nitrogen and CR4;
   X and Y are independently chosen from the group consisting of O and S;
   Z is chosen from the group consisting of CR4, O, S and NR4;
   U, V, and W are independently chosen from the group consisting of CR4, O, S and NR4;
   R1 and R3 are independently chosen from the group consisting of acyl, alkyl, alkenyl, alkynyl, hydroxyalkyl, aminoalkyl, thioalkyl, aryl, aralkyl, carboxyaryl, alkoxyalkyl, alkoxyaryl, alcoxycarbonylaryl, aminoaryl, amidoaryl, haloaryl, heteroaryl, heteroaralkyl, carbocyclyl, heterocyclyl, heterocyclylalkyl, alkoxycarbonyl, alkylaminocarbonyl, alkylthiocarbonyl, sulfonate, alkylsulfonate, arylsulfonate, sulfone, alkylsulfone, arylsulfone, sulfoxide, alkylsulfoxide, arylsulfoxide, alkylsulfonamide, arylsulfonamide, and sulfonamide, piperidinyl, morpholinyl, pyrrolidinyl, phenyl, pyridyl, pyrimidinyl, furyl, thienyl, pyrrolyl, imidazolyl, triazolyl, pyrazolyl, thiazolyl, isoxazolyl, oxazolyl, oxadiazolyl, indolyl, naphthyl, quinolinyl, isoquinolinyl, quinoxalinyl, benzyl, benzofuryl, dibenzofuryl, benzthienyl, benzoxazolyl, benzothiazolyl, benzimidazolyl, pyridoimidazolyl, pyrimidoimidazolyl, pyridopyrazolyl, pyrazolopyrimidinyl, and any of which is substituted with 1-6 independent substituents;
   R2 is chosen from the group consisting of hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl;
   R4 is absent or is chosen from the group consisting of hydrogen and alkyl; and the β2 integrin agonist is a substantially pure single conformer or a pharmaceutically acceptable salt or composition thereof.
22. The method of clause20, wherein the β2 integrin agonist is a compound of Formula (II) 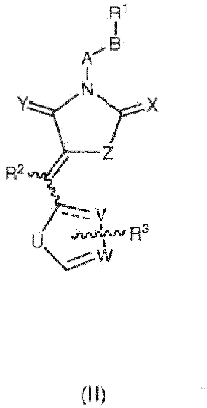 wherein
   A is absent or is alkyl;
   B is absent or is chosen from the group consisting of alkyl, alkenyl, O, S and NR4;
   N is chosen from the group consisting of nitrogen and CR4;
   X and Y are independently chosen from the group consisting of O and S;
   Z is chosen from the group consisting of CR4, O, S or NR4;
   U, V, and W are independently chosen from the group consisting of CR4, O, S and NR4;
   R1 and R3 are independently chosen from the group consisting of acyl, alkyl, alkenyl, alkynyl, hydroxyalkyl, aminoalkyl, thioalkyl, aryl, aralkyl, carboxyaryl, alkoxyalkyl, alkoxyaryl, alcoxycarbonylaryl, aminoaryl, amidoaryl, haloaryl, heteroaryl, heteroaralkyl, carbocyclyl, heterocyclyl, heterocyclylalkyl, alkoxycarbonyl, alkylaminocarbonyl, alkylthiocarbonyl, sulfonate, alkylsulfonate, arylsulfonate, sulfone, alkylsulfone, arylsulfone, sulfoxide, alkylsulfoxide, arylsulfoxide, alkylsulfonamide, arylsulfonamide, and sulfonamide, piperidinyl, morpholinyl, pyrrolidinyl, phenyl, pyridyl, pyrimidinyl, furyl, thienyl, pyrrolyl, imidazolyl, triazolyl, pyrazolyl, thiazolyl, isoxazolyl, oxazolyl, oxadiazolyl, indolyl, naphthyl, quinolinyl, isoquinolinyl, quinoxalinyl, benzyl, benzofuryl, dibenzofuryl, benzthienyl, benzoxazolyl, benzothiazolyl, benzimidazolyl, pyridoimidazolyl, pyrimidoimidazolyl, pyridopyrazolyl, pyrazolopyrimidinyl, and any of which is substituted with 1-6 independent substituents;
   R2 chosen from the group consisting of hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl;
   R4 is absent or is chosen from the group consisting of hydrogen and alkyl; and the β2 integrin agonist is a substantially pure single conformer or a pharmaceutically acceptable salt or composition thereof.
23. The method of clause20, wherein the β2 integrin agonist is a compound of Formula (III) 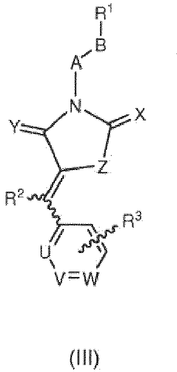 wherein
   A is absent or is alkyl;
   B is absent or is chosen from the group consisting of alkyl, alkenyl, O, S and NR4;
   N is chosen from the group consisting of nitrogen and CR4;
   X and Y are independently chosen from the group consisting of O and S; -
   Z is chosen from the group consisting of CR4, O, S or NR4;
   U, V, and W are independently chosen from the group consisting of CR4, O, S and NR4;
   R3 is 1-6 independent substituents present at position(s) 1-6 of the aryl ring;
   R1 and R3 are independently chosen from the group consisting of acyl, alkyl, alkenyl, alkynyl, hydroxyalkyl, aminoalkyl, thioalkyl, aryl, aralkyl, carboxyaryl, alkoxyalkyl, alkoxyaryl, alcoxycarbonylaryl, aminoaryl, amidoaryl, haloaryl, heteroaryl, heteroaralkyl, carbocyclyl, heterocyclyl, heterocyclylalkyl, alkoxycarbonyl, alkylaminocarbonyl, alkylthiocarbonyl, sulfonate, alkylsulfonate, arylsulfonate, sulfone, alkylsulfone, arylsulfone, sulfoxide, alkylsulfoxide, arylsulfoxide, alkylsulfonamide, arylsulfonamide, and sulfonamide, piperidinyl, morpholinyl, pyrrolidinyl, phenyl, pyridyl, pyrimidinyl, furyl, thienyl, pyrrolyl, imidazolyl, triazolyl, pyrazolyl, thiazolyl, isoxazolyl, oxazolyl, oxadiazolyl, indolyl, naphthyl, quinolinyl, isoquinolinyl, quinoxalinyl, benzyl, benzofuryl, dibenzofuryl, benzthienyl, benzoxazolyl, benzothiazolyl, benzimidazolyl, pyridoimidazolyl, pyrimidoimidazolyl, pyridopyrazolyl, pyrazolopyrimidinyl, and any of which is substituted with 1-6 independent substituents;
   R2 chosen from the group consisting of hydrogen, alkyl, hydroxyalkyl, aminoalkyl, thioalkyl, alkoxyalkyl, aryl, aralkyl, heteroaryl, heteroaralkyl, carbocyclyl, carbocyclylalkyl, heterocyclyl, and heterocyclylalkyl;
   R4 is absent or is chosen from the group consisting of hydrogen and alkyl; and the β2 integrin agonist is a substantially pure single conformer, or a pharmaceutically acceptable salt or composition thereof.
24. The method of clause 20, wherein the β2 integrins are CD11b/CD18.
25. The method of cfause 20, wherein said administering step further includes the step of administering a synergistic amount of an active agent chosen from the group consisting of a TNF-α blocker, an anti-inflammatory drug, an anti-cancer compound, anti-rejection drug, an anti-clotting drug, a steroid, a sphingosine-1-phosphate receptor modulator, and a drug-eluting stent media.
26. The method of clause20, wherein said activating step is further defined as interacting the β2 integrin agonist with β2 integrins.
27. The method of clause 26, wherein said interacting step is further defined as occupying a binding pocket in an αA-domain of the β2 integrins with the β2 integrin agonist.
28. The method of clause 26, wherein said interacting step is further defined as interacting a polar end of the β2 integrin agonist with polar residues of the β2 integrins, and interacting a non-polar end of the β2 integrin agonist with a hydrophobic pocket in the β2 integrins.
29. The method of clause 28, wherein said interacting step is further defined as interacting the β2 integrin agonist with a hydrophobic pocket lined by residues L312, I308, L305 α7 helix), L164, V160, F156 (α1 helix), and Y267, I269, I236, V238, I236, 1135 (central beta sheet) of the β2 integrins.
30. The method of clause 26, wherein said interacting step is further defined as selectively interacting CD11b/CD18 with the agonist.
31. The method of clause 20, further including the step of regulating a function of the β2 integrins chosen from the group consisting of conformation, organization in a cell, and organization on a cell membrane.
32. The method of clause 20, further including the step of clustering β2 integrins on a cell surface and producing a blockade of β2 integrins.
33. The method of clause 20, further including the step of increasing the binding of the β2 integrins to a ligand chosen from the group consisting of ICAM-1, ICAM-2, ICAM-3, iC3b, fibrinogen, Factor X, fibrin, uPAR, and GP Ibalpha.
34. The method of clause20, further including the step of modulating at least one function chosen from the group consisting of rolling of leukocytes with vascular endothelium, binding of leukocytes with vascular endothelium, crawling of leukocytes with vascular endothelium, translocation of leukocytes through vascular endothelium, infiltration of leukocytes into intimal tissue, release of one or more soluble factors from leukocytes, release of a chemotactic factor from leukocytes, release of a growth factor from leukocytes, leukocyte-binding-associated release of a chemotactic factor from a tissue, leukocyte-binding-associated release of a growth factor from a tissue, leukocyte-binding-associated release of one or more soluble factors from a tissue, change in the level of one or more soluble factors in circulation, and change in the level of one or more insoluble factors.
35. The method of clause 20, further including the step of modulating a biological function chosen from the group consisting of gene expression, epigenetic profile, protein expression, protein levels, protein modifications, post-translational modifications, and signaling.
36. The method of clause 20, wherein said activating step treats a disease chosen from the group consisting of inflammation, chronic inflammation, acute inflammation, inflammatory skin diseases, immune-related disorders, autoimmune diseases, burn, immune deficiency, acquired immune deficiency syndrome (AIDS), myeloperoxidase deficiency, Wiskott-Aldrich syndrome, chronic kidney disease, chronic granulomatous disease, hyper-IgM syndromes, leukocyte adhesion deficiency, iron deficiency, Chediak-Higashi syndrome, severe combined immunodeficiency, diabetes, obesity, hypertension, HIV, wound-healing, remodeling, scarring, fibrosis, stem cell therapies, cachexia, encephalomyelitis, multiple schlerosis, Alzheimers, psoriasis, lupus, rheumatoid arthritis, immune-related disorders, radiation injury, transplantation, cell transplantation, cell transfusion, organ transplantation, organ preservation, cell preservation, asthma, irritable bowel disease, irritable bowel syndrome, ulcerative colitis, colitis, bowel disease, cancer, leukemia, ischemia-reperfusion injury, stroke, neointimal thickening associated with vascular injury, bullous pemphigoid, neonatal obstructive nephropathy, familial hypercholesterolemia, atherosclerosis, dyslipidemia, aortic aneurisms, arteritis, vascular occlusion, including cerebral artery occlusion, complications of coronary by-pass surgery, myocarditis, including chronic autoimmune myocarditis and viral myocarditis, heart failure, including chronic heart failure (CHF), cachexia of heart failure, myocardial infarction, stenosis, restenosis after heart surgery, silent myocardial ischemia, post-implantation complications of left ventricular assist devices, thrombophlebitis, vasculitis, Kawasaki's vasculitis, giant cell arteritis, Wegener's granulomatosis, traumatic head injury, post-ischemic-reperfusion injury, post-ischemic cerebral inflammation, ischemia-reperfusion injury following myocardial infarction, cerebral malaria, and cardiovascular disease.
37. The method of clause 20, further including the step of detecting the activation of the β2 integrins and confirming that the patient has been treated.
38. A method of treating inflammation, including the steps of:
   administering a β2 integrin agonist to a patient with inflammation;
   activating β2 integrins; and
   reducing inflammation.
39. The method of clause 38, wherein the β2 integrins are CD11b/CD18.
40. The method of clause 38, further including the step of suppressing pro-inflammatory cytokine expression in leukocytes by phosphorylating Akt.
41. The method of clause 40, wherein said suppressing step is further defined as decreasing levels of IL-6, TNF-α, and MCP-1.
42. The method of claus40, further including the step of decreasing levels of reactive-oxygen species in TNF-α-activated neutrophils.
43. The method of clause 38, further including the steps of decreasing secretion of soluble factors by neutrophils and macrophages and decreasing inflammation.
44. The method of clause 38, further including the step of delaying neutrophil recruitment by increasing neutrophil adhesivity near sites of inflammation and decreasing neutrophil motility.
45. The method of clause 38, further including the steps of increasing leukocyte adhesion, decreasing leukocyte crawling and transendothelial migration, and reducing recruitment into inflamed/injured tissue.
46. The method of clause38, further including the step of inducing intracellular signaling and providing a negative feedback loop on pro-inflammatory signals and pathways.
47. The method of clause 46, wherein said inducing step is further defined as inducing TLR4, binding and stabilizing adaptor protein MyD88, recruiting downstream kinases, and initiating Nf-kB-mediated pro-inflammatory signaling.
48. The method of clause47, wherein said providing step is further defined as activating Syk, phosphorylating MyD88, and tagging MyD88 for ubiquitin-mediated destruction.
49. The method of clause 38, wherein the inflammation is caused by sepsis.
50. A method of treating and/or preventing renal ischemia-reperfusion (I/R) injury, including the steps of:
   administering a β2 integrin agonist to a patient; and
   activating β2 integrins.
51. The method of clause 50, wherein said administering step is performed after an event chosen from the group consisting of acute renal failure after renal transplantation, major surgery, trauma, septic shock, and hemorrhagic shock.
52. The method of clause 50, wherein said administering step is performed prior to surgery.
53. The method of clause 50, further including the step of reducing sCr levels, and producing a reno-protective effect.
54. A method of reducing restenosis in a patient due to insertion of a device, including the steps of:
   administering a β2 integrin agonist prior to the insertion of the device; and
   activating β2 integrins.
55. The method of clause 54, wherein the device is a stent and further including the steps of reducing accumulation of leukocytes at a site of vascular injury with the stent and decreasing neointimal thickening.
56. A method of reducing restenosis in a patient, including the steps of:
   administering a device that is coated with a β2 integrin agonist; and
   activating β2 integrins.
57. The method of clause 56, wherein the device is a stent.
58. A method of performing an assay for the identification of small molecule modulators of β2 integrins, including the steps of:
   identifying sites in the β2 integrins that modulate activity of the β2 integrin;
   determining an exact three-dimensional structure of a binding pocket; and
   identifying small molecules that can interact with the binding pocket.
59. The method of clause 58, wherein the β2 integrin is chosen from the group consisting of CD11b/CD18, CD11c/CD18, CD11d/CD18, and CD11a/CD18.
60. The method of clause 58, wherein said determining and identifying small molecules steps are performed by a method chosen from the group consisting of x-ray crystallography and nuclear magnetic resonance.
61. The method of clause 60, further including the step of performing a method chosen from the group consisting of scaffold hopping, atom replacement, residue replacement, molecule replacement, and combinations thereof.
62. The method of clause 58, wherein said identifying small molecules step is further defined as performing a cell-adhesion-based high-throughput screening assay and identifying small molecules that inhibit or enhance cell adhesion.
63. The method of clause 62, wherein said performing step is further defined as screening libraries of small molecule agonists against the β2 integrin, removing non-adherent cells by gravity by inverting assay plates, staining cell nuclei and imaging to quantitate the number of adherent cells, and identifying an agonist of the β2 integrin.
64. A method of detecting a disease in a patient, including the steps of:
   administering a β2 integrin agonist;
   detecting binding of the β2 integrin agonist to a β2 integrin; and
   confirming the presence of the disease.
65. The method of clause 64, wherein said detecting step is further defined by taking a sample from the patient and performing an assay to detect the binding of the β2 integrin agonist to the β2 integrin.
66. A method of improving the general wellness of a patient, including the steps of:
   administering an effective amount of a β2 integrin agonist; and
   activating β2 integrins.

## Claims

1. A pharmaceutical formulation comprising an effective amount of a compound having β2 integrin agonist activity, the compound having an inherent end-to-end polarity such that the compound is more polar at one end of the molecule compared to the other end of the molecule, the compound being a furanyl substituted thiazolidine selected from 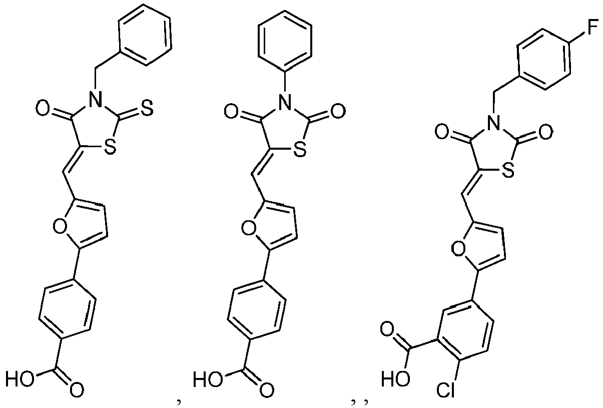 and pharmaceutically acceptable salts thereof, wherein
the compound being a substantially pure single conformer and being together with a pharmaceutically acceptable carrier;
the pharmaceutical formulation being for use in treating a condition associated with the activity of β2 integrins, wherein the condition is selected from acute inflammation, chronic inflammation, conditions associated with tumor infiltration modulated by leukocytic β2 integrins, and an autoimmune disease.

2. The pharmaceutical formulation for use of claim 1, wherein the condition is selected from acute inflammation and chronic inflammation.

3. The pharmaceutical formulation for use of claim 1, wherein the autoimmune disease is lupus.

4. The pharmaceutical formulation for the use according to any one of claims 1-3, wherein said compound having β2 integrin agonist activity is

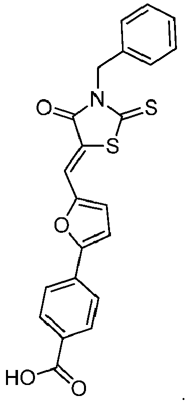

5. The pharmaceutical formulation for the use according to any one of claims 1-3, wherein said compound having β2 integrin agonist activity is

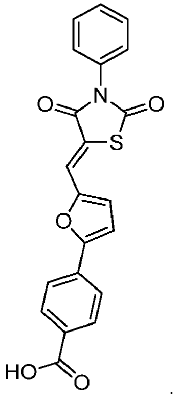

6. The pharmaceutical formulation for the use according to any one of claims 1-3, wherein said compound having β2 integrin agonist activity is

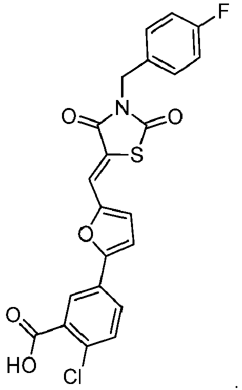

7. The pharmaceutical formulation for the use according to any one of claims 1-6, further including a synergistic amount of an active agent selected from a TNF-α blocker, a non-steroidal anti-inflammatory drug, an anti-cancer compound, an anti-rejection drug, an anti-clotting drug, a steroid, or a sphingosine-1-phosphate receptor modulator.

8. The pharmaceutical formulation for the use according to claim 7, wherein the TNF-α blocker chosen from etanercept, infliximab, and adalimumab.

9. The pharmaceutical formulation for the use according to claim 7, wherein the non-steroidal anti-inflammatory drug (NSAID) is chosen from salicylate, acetic acid derivatives, indomethacin, propionic acid derivatives, ibuprofen, naproxen, CoxII inhibitors, celecoxib, and rofecoxib.

10. The pharmaceutical formulation for the use according to claim 7, wherein the anti-cancer compound is cilengitide.

11. The pharmaceutical formulation for the use according to claim 7, wherein the sphingosine-1-phosphate receptor modulator is fingolimod.

12. The pharmaceutical formulation for the use according to claim 7, wherein the anti-rejection drug is chosen from tacrolimus, cyclosporine, and steroids.

13. The pharmaceutical formulation for the use according to claim 7, wherein the anti-clotting drug is chosen from warfarin, heparin, aspirin, ticlopidine, clopidogrel, dipyridamole, and glycoprotein IIIb/IIIa receptor agonists.

14. The pharmaceutical formulation for the use according to claim 7, wherein the steroid is chosen from tobramycin, dexamethasone, neomycin, hydrocortisone, prednisone, and erythromycin.

15. The pharmaceutical formulation for the use according to any one of claims 1-14, further including a drug-eluting stent medium.
